(19) Europäisches Patentamt
European Patent Office
Office européen des brevets

(11) EP 2 128 275 A2

(12) # EUROPEAN PATENT APPLICATION

published in accordance with Art. 153(4) EPC

(43) Date of publication:
**02.12.2009 Bulletin 2009/49**

(21) Application number: **08736698.5**

(22) Date of filing: **21.02.2008**

(51) Int Cl.:
***C12Q 1/68*** *(2006.01)*

(86) International application number:
**PCT/ES2008/000094**

(87) International publication number:
**WO 2008/102038 (28.08.2008 Gazette 2008/35)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.02.2007 ES 200700493**

(71) Applicants:
- **Progenika Biopharma, S.A.**
  **48160 Derio-Vizcaya (ES)**
- **Sabiobbi, S.L.**
  **28016 Madrid (ES)**

(72) Inventors:
- **TEJEDOR HERNÁNDEZ, Diego**
  **E-48160 Derio - Vizcaya (ES)**
- **SIMÓN BUELA, Laureano**
  **E-48160 Derio - Vizcaya (ES)**
- **MARTÍNEZ MARTÍNEZ, Antonio**
  **E-48160 Derio - Vizcaya (ES)**
- **VILLADONIGA LAO, José Ignacio**
  **E-48160 Derio - Vizcaya (ES)**

(74) Representative: **ABG Patentes, S.L.**
**Avenida de Burgos 16D**
**Edificio Euromor**
**28036 Madrid (ES)**

(54) **METHOD AND PRODUCT FOR "IN VITRO" GENOTYPING WITH APPLICATIONS IN ANTI-AGEING MEDICINE**

(57) The invention relates to an "in vitro" method for determining the global genetic risk a subject has of developing a pathology associated with aging. Said method is based on the combination of particular genetic risks of developing common pathologies associated with aging. Said particular genetic risks are determined from the results obtained from the simultaneous genotyping of certain genetic variations associated with said pathologies associated with aging and the main objective of which is the use thereof in anti-aging medicine.

EP 2 128 275 A2

SEQUENCE LISTING

<110> Sabiobbi, S.L.
Progenika Biopharma, S.A,

<120> METHOD AND PRODUCT FOR "IN VITRO" GENOTYPING WITH APPLICATION IN ANTI-AGING MEDICINE

<130> P2700EPPC

<140> EP 08736698.5
<141> 2009-09-22

<150> ES P200700493
<151> 2007-02-23

<160> 417

<170> PatentIn version 3.5

<210> 1
<211> 23
<212> DNA
<213> Artificial

<220>
<223> probe 1 for detecting the Intron 16 ins/del polymorphism in the ACE gene

<400> 1
gattacaggc gtgatacagt cac 23

<210> 2
<211> 23
<212> DNA
<213> Artificial

<220>
<223> probe 2 for detecting the Intron 16 ins/del polymorphism in the ACE gene

<400> 2
gtgactgtat cacgcctgta atc 23

<210> 3
<211> 23
<212> DNA
<213> Artificial

<220>
<223> probe 3 for detecting the Intron 16 ins/del polymorphism in the ACE gene

<400> 3
agacctgctg cctatacagt cac 23

<210> 4

<211> 23
<212> DNA
<213> Artificial

<220>
<223> probe 4 for detecting the Intron 16 ins/del polymorphism in the ACE gene

<400> 4
gtgactgtat aggcagcagg tct 23

<210> 5
<211> 23
<212> DNA
<213> Artificial

<220>
<223> probe 1 for detecting the ADRB1 Gly389Arg polymorphism in the ADRB1 gene

<400> 5
aggccttcca gcgactgctc tgc 23

<210> 6
<211> 23
<212> DNA
<213> Artificial

<220>
<223> probe 2 for detecting the ADRB1 Gly389Arg polymorphism in the ADRB1 gene

<400> 6
gcagagcagt cgctggaagg cct 23

<210> 7
<211> 23
<212> DNA
<213> Artificial

<220>
<223> probe 3 for detecting the ADRB1 Gly389Arg polymorphism in the ADRB1 gene

<400> 7
aggccttcca gggactgctc tgc 23

<210> 8
<211> 23
<212> DNA
<213> Artificial

<220>
<223> probe 8 for detecting the ADRB1 Gly389Arg polymorphism in the ADRB1 gene

<400> 8
gcagagcagt ccctggaagg cct 23

<210> 9
<211> 23
<212> DNA
<213> Artificial

<220>
<223> probe 1 for detecting the Gln27Glu polymorphism in the ADRB2 gene

<400> 9
acgtcacgca ggaaagggac gag 23

<210> 10
<211> 21
<212> DNA
<213> Artificial

<220>
<223> probe 2 for detecting the Gln27Glu polymorphism in the ADRB2 gene

<400> 10
cgtcacgcag gaaagggacg a 21

<210> 11
<211> 23
<212> DNA
<213> Artificial

<220>
<223> probe 3 for detecting the Gln27Glu polymorphism in the ADRB2 gene

<400> 11
acgtcacgca gcaaagggac gag 23

<210> 12
<211> 21
<212> DNA
<213> Artificial

<220>
<223> probe 4 for detecting the Gln27Glu polymorphism in the ADRB2 gene

<400> 12
cgtcacgcag caaagggacg a 21

<210> 13
<211> 23
<212> DNA
<213> Artificial

<220>
<223> probe 1 for detecting the Gly16Arg polymorphism in the ADRB2 gene

<400>  13
tggcacccaa tagaagccat gcg                                              23

<210>  14
<211>  25
<212>  DNA
<213>  Artificial

<220>
<223>  probe 2 for detecting the Gly16Arg polymorphism in the ADRB2 gene

<400>  14
ctggcaccca atagaagcca tgcgc                                            25

<210>  15
<211>  23
<212>  DNA
<213>  Artificial

<220>
<223>  probe 3 for detecting the Gly16Arg polymorphism in the ADRB2 gene

<400>  15
tggcacccaa tggaagccat gcg                                              23

<210>  16
<211>  25
<212>  DNA
<213>  Artificial

<220>
<223>  probe 4 for detecting the Gly16Arg polymorphism in the ADRB2 gene

<400>  16
ctggcaccca atggaagcca tgcgc                                            25

<210>  17
<211>  23
<212>  DNA
<213>  Artificial

<220>
<223>  probe 1 for detecting the Trp64Arg polymorphism in the ADRB3 gene

<400>  17
tggccatcgc ctggactccg aga                                              23

<210>  18
<211>  23
<212>  DNA
<213>  Artificial

<220>
<223>  probe 2 for detecting the Trp64Arg polymorphism in the ADRB3 gene

<400>  18
tctcggagtc caggcgatgg cca                                                23

<210>  19
<211>  23
<212>  DNA
<213>  Artificial

<220>
<223>  probe 3 for detecting the Trp64Arg polymorphism in the ADRB3 gene

<400>  19
tggccatcgc ccggactccg aga                                                23

<210>  20
<211>  23
<212>  DNA
<213>  Artificial

<220>
<223>  probe 4 for detecting the Trp64Arg polymorphism in the ADRB3 gene

<400>  20
tctcggagtc cgggcgatgg cca                                                23

<210>  21
<211>  25
<212>  DNA
<213>  Artificial

<220>
<223>  probe 1 for detecting the Met235Thr polymorphism in the AGT gene

<400>  21
gctgctccct gacgggagcc agtgt                                              25

<210>  22
<211>  27
<212>  DNA
<213>  Artificial

<220>
<223>  probe 2 for detecting the Met235Thr polymorphism in the AGT gene

<400>  22
cacactggct cccgtcaggg agcagcc                                            27

<210>  23
<211>  25
<212>  DNA
<213>  Artificial

<220>
<223>  probe 3 for detecting the Met235Thr polymorphism in the AGT gene

<400>  23
gctgctccct gatgggagcc agtgt                                          25

<210>  24
<211>  27
<212>  DNA
<213>  Artificial

<220>
<223>  probe 4 for detecting the Met235Thr polymorphism in the AGT gene

<400>  24
cacactggct cccatcaggg agcagcc                                        27

<210>  25
<211>  23
<212>  DNA
<213>  Artificial

<220>
<223>  probe 1 for detecting the 1166 A>C polymorphism in the AGTR1 gene

<400>  25
accaaatgag cattagctac ttt                                            23

<210>  26
<211>  23
<212>  DNA
<213>  Artificial

<220>
<223>  probe 2 for detecting the 1166 A>C polymorphism in the AGTR1 gene

<400>  26
aaagtagcta atgctcattt ggt                                            23

<210>  27
<211>  23
<212>  DNA
<213>  Artificial

<220>
<223>  probe 3 for detecting the 1166 A>C polymorphism in the AGTR1 gene

<400>  27
accaaatgag ccttagctac ttt                                            23

<210>  28
<211>  23
<212>  DNA
<213>  Artificial

<220>
<223>  probe 4 for detecting the 1166 A>C polymorphism in the AGTR1 gene

<400> 28
aaagtagcta aggctcattt ggt 23

<210> 29
<211> 21
<212> DNA
<213> Artificial

<220>
<223> probe 1 for detecting the -75 G>A polymorphism in the APOA1 gene

<400> 29
agcccagccc cggccctgtt g 21

<210> 30
<211> 19
<212> DNA
<213> Artificial

<220>
<223> probe 2 for detecting the -75 G>A polymorphism in the APOA1 gene

<400> 30
gcccagcccc ggccctgtt 19

<210> 31
<211> 21
<212> DNA
<213> Artificial

<220>
<223> probe 3 for detecting the -75 G>A polymorphism in the APOA1 gene

<400> 31
agcccagccc tggccctgtt g 21

<210> 32
<211> 19
<212> DNA
<213> Artificial

<220>
<223> probe 4 for detecting the -75 G>A polymorphism in the APOA1 gene

<400> 32
gcccagccct ggccctgtt 19

<210> 33
<211> 23
<212> DNA
<213> Artificial

<220>
<223> probe 1 for detecting the Arg3480Trp polymorphism in the APOB gene

<400>  33
cggttctttc tcgggaatat tca                                               23

<210>  34
<211>  23
<212>  DNA
<213>  Artificial

<220>
<223>  probe 2 for detecting the Arg3480Trp polymorphism in the APOB gene

<400>  34
tgaatattcc cgagaaagaa ccg                                               23

<210>  35
<211>  23
<212>  DNA
<213>  Artificial

<220>
<223>  probe 3 for detecting the Arg3480Trp polymorphism in the APOB gene

<400>  35
cggttctttc ttgggaatat tca                                               23

<210>  36
<211>  23
<212>  DNA
<213>  Artificial

<220>
<223>  probe 4 for detecting the Arg3480Trp polymorphism in the APOB gene

<400>  36
tgaatattcc caagaaagaa ccg                                               23

<210>  37
<211>  23
<212>  DNA
<213>  Artificial

<220>
<223>  probe 1 for detecting the Arg3500Gln polymorphism in the APOB gene

<400>  37
caagagcaca cggtcttcag tga                                               23

<210>  38
<211>  23
<212>  DNA

<213> Artificial

<220>
<223> probe 2 for detecting the Arg3500Gln polymorphism in the APOB gene

<400> 38
tcactgaaga ccgtgtgctc ttg 23

<210> 39
<211> 23
<212> DNA
<213> Artificial

<220>
<223> probe 3 for detecting the Arg3500Gln polymorphism in the APOB gene

<400> 39
caagagcaca cagtcttcag tga 23

<210> 40
<211> 23
<212> DNA
<213> Artificial

<220>
<223> probe 4 for detecting the Arg3500Gln polymorphism in the APOB gene

<400> 40
tcactgaaga ctgtgtgctc ttg 23

<210> 41
<211> 23
<212> DNA
<213> Artificial

<220>
<223> probe 1 for detecting the Arg3531Cys polymorphism in the APOB gene

<400> 41
ccacactcca acgcatatat tcc 23

<210> 42
<211> 23
<212> DNA
<213> Artificial

<220>
<223> probe 2 for detecting the Arg3531Cys polymorphism in the APOB gene

<400> 42
ggaatatatg cgttggagtg tgg 23

<210> 43
<211> 23
<212> DNA
<213> Artificial

<220>
<223> probe 3 for detecting the Arg3531Cys polymorphism in the APOB gene

<400> 43
ccacactcca atgcatatat tcc 23

<210> 44
<211> 23
<212> DNA
<213> Artificial

<220>
<223> probe 4 for detecting the Arg3531Cys polymorphism in the APOB gene

<400> 44
ggaatatatg cattggagtg tgg 23

<210> 45
<211> 25
<212> DNA
<213> Artificial

<220>
<223> probe 1 for detecting the Cys112Arg polymorphism in the APOE gene

<400> 45
atggaggacg tgtgcggccg cctgg 25

<210> 46
<211> 25
<212> DNA
<213> Artificial

<220>
<223> probe 2 for detecting the Cys112Arg polymorphism in the APOE gene

<400> 46
ccaggcggcc gcacacgtcc tccat 25

<210> 47
<211> 25
<212> DNA
<213> Artificial

<220>
<223> probe 3 for detecting the Cys112Arg polymorphism in the APOE gene

<400> 47
atggaggacg tgcgcggccg cctgg 25

<210> 48
<211> 25
<212> DNA
<213> Artificial

<220>
<223> probe 4 for detecting the Cys112Arg polymorphism in the APOE gene

<400> 48
ccaggcggcc gcgcacgtcc tccat 25

<210> 49
<211> 25
<212> DNA
<213> Artificial

<220>
<223> probe 1 for detecting the Arg158Cys polymorphism in the APOE gene

<400> 49
gacctgcaga agcgcctggc agtgt 25

<210> 50
<211> 25
<212> DNA
<213> Artificial

<220>
<223> probe 2 for detecting the Arg158Cys polymorphism in the APOE gene

<400> 50
acactgccag gcgcttctgc aggtc 25

<210> 51
<211> 25
<212> DNA
<213> Artificial

<220>
<223> probe 3 for detecting the Arg158Cys polymorphism in the APOE gene

<400> 51
gacctgcaga agtgcctggc agtgt 25

<210> 52
<211> 25
<212> DNA
<213> Artificial

<220>
<223> probe 4 for detecting the Arg158Cys polymorphism in the APOE gene

<400> 52
acactgccag gcacttctgc aggtc 25

<210> 53
<211> 23
<212> DNA
<213> Artificial

<220>
<223> probe 1 for detecting the 833 T>C polymorphism in the CBS gene

<400> 53
gatccacccc agtgatctgc aga 23

<210> 54
<211> 21
<212> DNA
<213> Artificial

<220>
<223> probe 2 for detecting the 833 T>C polymorphism in the CBS gene

<400> 54
atccacccca gtgatctgca g 21

<210> 55
<211> 23
<212> DNA
<213> Artificial

<220>
<223> probe 3 for detecting the 833 T>C polymorphism in the CBS gene

<400> 55
gatccacccc aatgatctgc aga 23

<210> 56
<211> 21
<212> DNA
<213> Artificial

<220>
<223> probe 4 for detecting the 833 T>C polymorphism in the CBS gene

<400> 56
atccacccca atgatctgca g 21

<210> 57
<211> 23
<212> DNA
<213> Artificial

<220>
<223> probe 1 for detecting the 844ins68 polymorphism in the CBS gene

<400> 57
tggggtggat catccaggtg ggg 23

<210> 58
<211> 23
<212> DNA
<213> Artificial

<220>
<223> probe 2 for detecting the 844ins68 polymorphism in the CBS gene

<400> 58
ccccacctgg atgatccacc cca 23

<210> 59
<211> 23
<212> DNA
<213> Artificial

<220>
<223> probe 3 for detecting the 844ins68 polymorphism in the CBS gene

<400> 59
tggggtggat cccgaagggt cca 23

<210> 60
<211> 23
<212> DNA
<213> Artificial

<220>
<223> probe 4 for detecting the 844ins68 polymorphism in the CBS gene

<400> 60
tggacccttc gggatccacc cca 23

<210> 61
<211> 23
<212> DNA
<213> Artificial

<220>
<223> probe 1 for detecting the TaqIB polymorphism in the CETP gene

<400> 61
cactggggtt cgagttaggg ttc 23

<210> 62
<211> 23
<212> DNA
<213> Artificial

<220>
<223> probe 2 for detecting the TaqIB polymorphism in the CETP gene

<400>  62
gaaccctaac tcgaacccca gtg    23

<210>  63
<211>  23
<212>  DNA
<213>  Artificial

<220>
<223>  probe 3 for detecting the TaqIB polymorphism in the CETP gene

<400>  63
cactggggtt caagttaggg ttc    23

<210>  64
<211>  23
<212>  DNA
<213>  Artificial

<220>
<223>  probe 4 for detecting the TaqIB polymorphism in the CETP gene

<400>  64
gaaccctaac ttgaacccca gtg    23

<210>  65
<211>  23
<212>  DNA
<213>  Artificial

<220>
<223>  probe 1 for detecting the Arg451Gln polymorphism in the CETP gene

<400>  65
gattatcact cgagatgtga gta    23

<210>  66
<211>  21
<212>  DNA
<213>  Artificial

<220>
<223>  probe 2 for detecting the Arg451Gln polymorphism in the CETP gene

<400>  66
attatcactc gagatgtgag t    21

<210>  67
<211>  23
<212>  DNA
<213>  Artificial

<220>
<223>  probe 3 for detecting the Arg451Gln polymorphism in the CETP gene

<400> 67
gattatcact caagatgtga gta 23

<210> 68
<211> 21
<212> DNA
<213> Artificial

<220>
<223> probe 4 for detecting the Arg451Gln polymorphism in the CETP gene

<400> 68
attatcactc aagatgtgag t 21

<210> 69
<211> 23
<212> DNA
<213> Artificial

<220>
<223> probe 1 for detecting the 1546 G>T polymorphism in the COL1A1 gene

<400> 69
tcatcccgcc cccattccct ggg 23

<210> 70
<211> 21
<212> DNA
<213> Artificial

<220>
<223> probe 2 for detecting the 1546 G>T polymorphism in the COL1A1 gene

<400> 70
catcccgccc ccattccctg g 21

<210> 71
<211> 23
<212> DNA
<213> Artificial

<220>
<223> probe 3 for detecting the 1546 G>T polymorphism in the COL1A1 gene

<400> 71
tcatcccgcc cacattccct ggg 23

<210> 72
<211> 21
<212> DNA
<213> Artificial

<220>
<223> probe 4 for detecting the 1546 G>T polymorphism in the COL1A1 gene

<400> 72
catcccgccc acattccctg g 21

<210> 73
<211> 23
<212> DNA
<213> Artificial

<220>
<223> probe 1 for detecting the Val158Met polymorphism (Allele*2) in the COMT gene

<400> 73
atttcgctgg cgtgaaggac aag 23

<210> 74
<211> 23
<212> DNA
<213> Artificial

<220>
<223> probe 2 for detecting the Val158Met polymorphism (Allele*2) in the COMT gene

<400> 74
cttgtccttc acgccagcga aat 23

<210> 75
<211> 23
<212> DNA
<213> Artificial

<220>
<223> probe 3 for detecting the Val158Met polymorphism (Allele*2) in the COMT gene

<400> 75
atttcgctgg catgaaggac aag 23

<210> 76
<211> 23
<212> DNA
<213> Artificial

<220>
<223> probe 4 for detecting the Val158Met polymorphism (Allele*2) in the COMT gene

<400> 76
cttgtccttc atgccagcga aat 23

<210> 77
<211> 21
<212> DNA
<213> Artificial

<220>
<223> probe 1 for detecting the -34 A>G polymorphism in the CYP17A1 gene

<400> 77
tctactccac tgctgtctat c 21

<210> 78
<211> 23
<212> DNA
<213> Artificial

<220>
<223> probe 2 for detecting the -34 A>G polymorphism in the CYP17A1 gene

<400> 78
agatagacag cagtggagta gaa 23

<210> 79
<211> 21
<212> DNA
<213> Artificial

<220>
<223> probe 3 for detecting the -34 A>G polymorphism in the CYP17A1 gene

<400> 79
tctactccac cgctgtctat c 21

<210> 80
<211> 23
<212> DNA
<213> Artificial

<220>
<223> probe 4 for detecting the -34 A>G polymorphism in the CYP17A1 gene

<400> 80
agatagacag cggtggagta gaa 23

<210> 81
<211> 23
<212> DNA
<213> Artificial

<220>
<223> probe 1 for detecting the 1558 C>T polymorphism in the CYP19A1 gene

<400> 81
tggtcagtac ccactctgga gca 23

<210> 82
<211> 23
<212> DNA
<213> Artificial

<220>
<223> probe 2 for detecting the 1558 C>T polymorphism in the CYP19A1 gene

<400> 82
tgctccagag tgggtactga cca 23

<210> 83
<211> 23
<212> DNA
<213> Artificial

<220>
<223> probe 3 for detecting the 1558 C>T polymorphism in the CYP19A1 gene

<400> 83
tggtcagtac ctactctgga gca 23

<210> 84
<211> 23
<212> DNA
<213> Artificial

<220>
<223> probe 4 for detecting the 1558 C>T polymorphism in the CYP19A1 gene

<400> 84
tgctccagag taggtactga cca 23

<210> 85
<211> 23
<212> DNA
<213> Artificial

<220>
<223> probe 1 for detecting the Ile462Val polymorphism in the CYP1A1 gene

<400> 85
tcggtgagac cattgcccgc tgg 23

<210> 86
<211> 23
<212> DNA

<213> Artificial

<220>
<223> probe 2 for detecting the Ile462Val polymorphism in the CYP1A1 gene

<400> 86
ccagcgggca atggtctcac cga 23

<210> 87
<211> 23
<212> DNA
<213> Artificial

<220>
<223> probe 3 for detecting the Ile462Val polymorphism in the CYP1A1 gene

<400> 87
tcggtgagac cgttgcccgc tgg 23

<210> 88
<211> 23
<212> DNA
<213> Artificial

<220>
<223> probe 4 for detecting the Ile462Val polymorphism in the CYP1A1 gene

<400> 88
ccagcgggca acggtctcac cga 23

<210> 89
<211> 19
<212> DNA
<213> Artificial

<220>
<223> probe 1 for detecting the T3801C polymorphism in the CYP1A1 gene

<400> 89
tccacctcct gggctcaca 19

<210> 90
<211> 19
<212> DNA
<213> Artificial

<220>
<223> probe 2 for detecting the T3801C polymorphism in the CYP1A1 gene

<400> 90
tccacctccc gggctcaca 19

<210> 91
<211> 19
<212> DNA
<213> Artificial

<220>
<223> probe 3 for detecting the T3801C polymorphism in the CYP1A1 gene

<400> 91
tccacctcct gggctcaca 19

<210> 92
<211> 19
<212> DNA
<213> Artificial

<220>
<223> probe 4 for detecting the T3801C polymorphism in the CYP1A1 gene

<400> 92
tccacctccc gggctcaca 19

<210> 93
<211> 25
<212> DNA
<213> Artificial

<220>
<223> probe 1 for detecting the Leu432Val polymorphism in the CYP1B1 gene

<400> 93
aatcatgacc cactgaagtg gccta 25

<210> 94
<211> 25
<212> DNA
<213> Artificial

<220>
<223> probe 2 for detecting the Leu432Val polymorphism in the CYP1B1 gene

<400> 94
taggccactt cagtgggtca tgatt 25

<210> 95
<211> 25
<212> DNA
<213> Artificial

<220>
<223> probe 3 for detecting the Leu432Val polymorphism in the CYP1B1 gene

<400> 95

aatcatgacc cagtgaagtg gccta 25

<210> 96
<211> 25
<212> DNA
<213> Artificial

<220>
<223> probe 4 for detecting the Leu432Val polymorphism in the CYP1B1 gene

<400> 96
taggccactt cactgggtca tgatt 25

<210> 97
<211> 23
<212> DNA
<213> Artificial

<220>
<223> probe 1 for detecting the Allele*4 (Asn453Ser) polymorphism in the CYP1B1 gene

<400> 97
cggcctcatc aacaaggacc tga 23

<210> 98
<211> 23
<212> DNA
<213> Artificial

<220>
<223> probe 2 for detecting the Allele*4 (Asn453Ser) polymorphism in the CYP1B1 gene

<400> 98
tcaggtcctt gttgatgagg ccg 23

<210> 99
<211> 23
<212> DNA
<213> Artificial

<220>
<223> probe 3 for detecting the Allele*4 (Asn453Ser) polymorphism in the CYP1B1 gene

<400> 99
cggcctcatc agcaaggacc tga 23

<210> 100
<211> 23
<212> DNA
<213> Artificial

<220>
<223> probe 4 for detecting the Allele*4 (Asn453Ser) polymorphism in the CYP1B1 gene

<400> 100
tcaggtcctt gctgatgagg ccg 23

<210> 101
<211> 23
<212> DNA
<213> Artificial

<220>
<223> probe 1 for detecting the Arg144Cys (allele*2) polymorphism in the CYP2C9 gene

<400> 101
gcattgagga ccgtgttcaa gag 23

<210> 102
<211> 23
<212> DNA
<213> Artificial

<220>
<223> probe 2 for detecting the Arg144Cys (allele*2) polymorphism in the CYP2C9 gene

<400> 102
ctcttgaaca cggtcctcaa tgc 23

<210> 103
<211> 23
<212> DNA
<213> Artificial

<220>
<223> probe 3 for detecting the Arg144Cys (allele*2) polymorphism in the CYP2C9 gene

<400> 103
gcattgagga ctgtgttcaa gag 23

<210> 104
<211> 23
<212> DNA
<213> Artificial

<220>
<223> probe 4 for detecting the Arg144Cys (allele*2) polymorphism in the CYP2C9 gene

<400> 104
ctcttgaaca cagtcctcaa tgc 23

<210> 105
<211> 23
<212> DNA
<213> Artificial

<220>
<223> probe 1 for detecting the Ile359Leu (allele*3) polymorphism in the CYP2C9 gene

<400> 105
tccagagata cattgacctt ctc 23

<210> 106
<211> 23
<212> DNA
<213> Artificial

<220>
<223> probe 2 for detecting the Ile359Leu (allele*3) polymorphism in the CYP2C9 gene

<400> 106
gagaaggtca atgtatctct gga 23

<210> 107
<211> 23
<212> DNA
<213> Artificial

<220>
<223> probe 3 for detecting the Ile359Leu (allele*3) polymorphism in the CYP2C9 gene

<400> 107
tccagagata ccttgacctt ctc 23

<210> 108
<211> 23
<212> DNA
<213> Artificial

<220>
<223> probe 4 for detecting the Ile359Leu (allele*3) polymorphism in the CYP2C9 gene

<400> 108
gagaaggtca aggtatctct gga 23

<210> 109
<211> 23
<212> DNA
<213> Artificial

<220>
<223> probe 1 for detecting the 681 G>A (Pro227Pro) (allele*2) polymorphism in the CYP2C19 gene

<400> 109
gattatttcc cgggaaccca taa 23

<210> 110
<211> 21
<212> DNA
<213> Artificial

<220>
<223> probe 2 for detecting the 681 G>A (Pro227Pro) (allele*2) polymorphism in the CYP2C19 gene

<400> 110
attatttccc gggaacccat a 21

<210> 111
<211> 23
<212> DNA
<213> Artificial

<220>
<223> probe 3 for detecting the 681 G>A (Pro227Pro) (allele*2) polymorphism in the CYP2C19 gene

<400> 111
gattatttcc caggaaccca taa 23

<210> 112
<211> 21
<212> DNA
<213> Artificial

<220>
<223> probe 4 for detecting the 681 G>A (Pro227Pro) (allele*2) polymorphism in the CYP2C19 gene

<400> 112
attatttccc aggaacccat a 21

<210> 113
<211> 23
<212> DNA
<213> Artificial

<220>
<223> probe 1 for detecting the 2549 A>del (allele*3) polymorphism in the CYP2D6 gene

<400> 113
ccaggtcatc ctgtgctcag tta 23

<210> 114
<211> 21
<212> DNA

<213> Artificial

<220>
<223> probe 2 for detecting the 2549 A>del (allele*3) polymorphism in the CYP2D6 gene

<400> 114
caggtcatcc tgtgctcagt t 21

<210> 115
<211> 23
<212> DNA
<213> Artificial

<220>
<223> probe 3 for detecting the 2549 A>del (allele*3) polymorphism in the CYP2D6 gene

<400> 115
ccaggtcatc cgtgctcagt tag 23

<210> 116
<211> 21
<212> DNA
<213> Artificial

<220>
<223> probe 4 for detecting the 2549 A>del (allele*3) polymorphism in the CYP2D6 gene

<400> 116
caggtcatcc gtgctcagtt a 21

<210> 117
<211> 21
<212> DNA
<213> Artificial

<220>
<223> probe 1 for detecting the 1846 G>A / 1847 G>A (allele*4) polymorphism in the CYP2D6 gene

<400> 117
cccaccccca ggacgcccct t 21

<210> 118
<211> 19
<212> DNA
<213> Artificial

<220>
<223> probe 2 for detecting the 1846 G>A / 1847 G>A (allele*4) polymorphism in the CYP2D6 gene

<400> 118
ccacccccag gacgcccct 19

<210> 119
<211> 21
<212> DNA
<213> Artificial

<220>
<223> probe 3 for detecting the 1846 G>A / 1847 G>A (allele*4) polymorphism in the CYP2D6 gene

<400> 119
cccaccccca agacgcccct t 21

<210> 120
<211> 19
<212> DNA
<213> Artificial

<220>
<223> probe 4 for detecting the 1846 G>A / 1847 G>A (allele*4) polymorphism in the CYP2D6 gene

<400> 120
ccacccccaa gacgcccct 19

<210> 121
<211> 21
<212> DNA
<213> Artificial

<220>
<223> probe 1 for detecting the 1707 del>T (allele*6) polymorphism in the CYP2D6 gene

<400> 121
gctggagcag tgggtgaccg a 21

<210> 122
<211> 19
<212> DNA
<213> Artificial

<220>
<223> probe 2 for detecting the 1707 del>T (allele*6) polymorphism in the CYP2D6 gene

<400> 122
ctggagcagt gggtgaccg 19

<210> 123
<211> 21
<212> DNA
<213> Artificial

<220>

<223> probe 3 for detecting the 1707 del>T (allele*6) polymorphism in the CYP2D6 gene

<400> 123
cgctggagca ggggtgaccg a 21

<210> 124
<211> 19
<212> DNA
<213> Artificial

<220>
<223> probe 4 for detecting the 1707 del>T (allele*6) polymorphism in the CYP2D6 gene

<400> 124
gctggagcag gggtgaccg 19

<210> 125
<211> 23
<212> DNA
<213> Artificial

<220>
<223> probe 1 for detecting the Ala541Thr polymorphism in the ELAC2 gene

<400> 125
gcaccctggc tgctgtgttt gtg 23

<210> 126
<211> 23
<212> DNA
<213> Artificial

<220>
<223> probe 2 for detecting the Ala541Thr polymorphism in the ELAC2 gene

<400> 126
cacaaacaca gcagccaggg tgc 23

<210> 127
<211> 23
<212> DNA
<213> Artificial

<220>
<223> probe 3 for detecting the Ala541Thr polymorphism in the ELAC2 gene

<400> 127
gcaccctggc tactgtgttt gtg 23

<210> 128

<211> 23
<212> DNA
<213> Artificial

<220>
<223> probe 4 for detecting the Ala541Thr polymorphism in the ELAC2 gene

<400> 128
cacaaacaca gtagccaggg tgc 23

<210> 129
<211> 23
<212> DNA
<213> Artificial

<220>
<223> probe 1 for detecting the -397 T>C (PvuII) polymorphism in the ESR1 IVS1 gene

<400> 129
aatgtcccag ctgttttatg ctt 23

<210> 130
<211> 21
<212> DNA
<213> Artificial

<220>
<223> probe 2 for detecting the -397 T>C (PvuII) polymorphism in the ESR1 IVS1 gene

<400> 130
atgtcccagc tgttttatgc t 21

<210> 131
<211> 23
<212> DNA
<213> Artificial

<220>
<223> probe 3 for detecting the -397 T>C (PvuII) polymorphism in the ESR1 IVS1 gene

<400> 131
aatgtcccag ccgttttatg ctt 23

<210> 132
<211> 21
<212> DNA
<213> Artificial

<220>
<223> probe 4 for detecting the -397 T>C (PvuII) polymorphism in the ESR1 IVS1 gene

<400>  132
atgtcccagc cgttttatgc t                                                21

<210>  133
<211>  23
<212>  DNA
<213>  Artificial

<220>
<223>  probe 1 for detecting the Val34Leu polymorphism in the F13A1 gene

<400>  133
agcttcaggg cgtggtgccc cgg                                              23

<210>  134
<211>  21
<212>  DNA
<213>  Artificial

<220>
<223>  probe 2 for detecting the Val34Leu polymorphism in the F13A1 gene

<400>  134
gcttcagggc gtggtgcccc g                                                21

<210>  135
<211>  23
<212>  DNA
<213>  Artificial

<220>
<223>  probe 3 for detecting the Val34Leu polymorphism in the F13A1 gene

<400>  135
agcttcaggg cttggtgccc cgg                                              23

<210>  136
<211>  21
<212>  DNA
<213>  Artificial

<220>
<223>  probe 4 for detecting the Val34Leu polymorphism in the F13A1 gene

<400>  136
gcttcagggc ttggtgcccc g                                                21

<210>  137
<211>  23
<212>  DNA
<213>  Artificial

<220>
<223>  probe 1 for detecting the -455 G>A polymorphism in the FGB gene

<400> 137
ttgattttaa tggccccttt tga 23

<210> 138
<211> 23
<212> DNA
<213> Artificial

<220>
<223> probe 2 for detecting the -455 G>A polymorphism in the FGB gene

<400> 138
tcaaaagggg ccattaaaat caa 23

<210> 139
<211> 23
<212> DNA
<213> Artificial

<220>
<223> probe 3 for detecting the -455 G>A polymorphism in the FGB gene

<400> 139
ttgattttaa tagccccttt tga 23

<210> 140
<211> 23
<212> DNA
<213> Artificial

<220>
<223> probe 4 for detecting the -455 G>A polymorphism in the FGB gene

<400> 140
tcaaaagggg ctattaaaat caa 23

<210> 141
<211> 23
<212> DNA
<213> Artificial

<220>
<223> probe 1 for detecting the 20210 G>A polymorphism in the FII gene

<400> 141
tgactctcag cgagcctcaa tgc 23

<210> 142
<211> 23
<212> DNA
<213> Artificial

<220>
<223> probe 2 for detecting the 20210 G>A polymorphism in the FII gene

<400> 142
gcattgaggc tcgctgagag tca 23

<210> 143
<211> 23
<212> DNA
<213> Artificial

<220>
<223> probe 3 for detecting the 20210 G>A polymorphism in the FII gene

<400> 143
tgactctcag caagcctcaa tgc 23

<210> 144
<211> 23
<212> DNA
<213> Artificial

<220>
<223> probe 4 for detecting the 20210 G>A polymorphism in the FII gene

<400> 144
gcattgaggc ttgctgagag tca 23

<210> 145
<211> 23
<212> DNA
<213> Artificial

<220>
<223> probe 1 for detecting the Arg506Gln polymorphism in the FV Leiden gene

<400> 145
cctggacagg cgaggaatac agg 23

<210> 146
<211> 23
<212> DNA
<213> Artificial

<220>
<223> probe 2 for detecting the Arg506Gln polymorphism in the FV Leiden gene

<400> 146
cctgtattcc tcgcctgtcc agg 23

<210> 147
<211> 23
<212> DNA
<213> Artificial

<220>

<223> probe 3 for detecting the Arg506Gln polymorphism in the FV Leiden gene

<400> 147
cctggacagg caaggaatac agg 23

<210> 148
<211> 23
<212> DNA
<213> Artificial

<220>
<223> probe 4 for detecting the Arg506Gln polymorphism in the FV Leiden gene

<400> 148
cctgtattcc ttgcctgtcc agg 23

<210> 149
<211> 23
<212> DNA
<213> Artificial

<220>
<223> probe 1 for detecting the Pro319Ser polymorphism in the GJA4 gene

<400> 149
atggccaaaa acccccaagt cgt 23

<210> 150
<211> 23
<212> DNA
<213> Artificial

<220>
<223> probe 2 for detecting the Pro319Ser polymorphism in the GJA4 gene

<400> 150
acgacttggg ggtttttggc cat 23

<210> 151
<211> 23
<212> DNA
<213> Artificial

<220>
<223> probe 3 for detecting the Pro319Ser polymorphism in the GJA4 gene

<400> 151
atggccaaaa atccccaagt cgt 23

<210> 152
<211> 23
<212> DNA
<213> Artificial

<220>
<223> probe 4 for detecting the Pro319Ser polymorphism in the GJA4 gene

<400> 152
acgacttggg gatttttggc cat 23

<210> 153
<211> 23
<212> DNA
<213> Artificial

<220>
<223> probe 1 for detecting the 393 T>C (Ile131Ile) polymorphism in the GNAS gene

<400> 153
gtggactaca ttctgagtgt gat 23

<210> 154
<211> 23
<212> DNA
<213> Artificial

<220>
<223> probe 2 for detecting the 393 T>C (Ile131Ile) polymorphism in the GNAS gene

<400> 154
atcacactca gaatgtagtc cac 23

<210> 155
<211> 23
<212> DNA
<213> Artificial

<220>
<223> probe 3 for detecting the 393 T>C (Ile131Ile) polymorphism in the GNAS gene

<400> 155
gtggactaca tcctgagtgt gat 23

<210> 156
<211> 23
<212> DNA
<213> Artificial

<220>
<223> probe 4 for detecting the 393 T>C (Ile131Ile) polymorphism in the GNAS gene

<400> 156
atcacactca ggatgtagtc cac 23

<210> 157
<211> 23
<212> DNA
<213> Artificial

<220>
<223> probe 1 for detecting the 825 C>T (Ser275Ser) polymorphism in the GNB3 gene

<400> 157
ggcatcacgt ccgtggcctt ctc 23

<210> 158
<211> 23
<212> DNA
<213> Artificial

<220>
<223> probe 2 for detecting the 825 C>T (Ser275Ser) polymorphism in the GNB3 gene

<400> 158
gagaaggcca cggacgtgat gcc 23

<210> 159
<211> 23
<212> DNA
<213> Artificial

<220>
<223> probe 3 for detecting the 825 C>T (Ser275Ser) polymorphism in the GNB3 gene

<400> 159
ggcatcacgt ctgtggcctt ctc 23

<210> 160
<211> 23
<212> DNA
<213> Artificial

<220>
<223> probe 4 for detecting the 825 C>T (Ser275Ser) polymorphism in the GNB3 gene

<400> 160
gagaaggcca cagacgtgat gcc 23

<210> 161
<211> 25
<212> DNA
<213> Artificial

<220>
<223> probe 1 for detecting the GSTM1 polymorphism

<400> 161
cacatattct tggccttctg cagat 25

<210> 162
<211> 25
<212> DNA
<213> Artificial

<220>
<223> probe 2 for detecting the GSTM1 polymorphism

<400> 162
atctgcagaa ggccaagaat atgtg 25

<210> 163
<211> 25
<212> DNA
<213> Artificial

<220>
<223> probe 3 for detecting the GSTM1 polymorphism

<400> 163
cacatattct tgaccttctg cagat 25

<210> 164
<211> 25
<212> DNA
<213> Artificial

<220>
<223> probe 4 for detecting the GSTM1 polymorphism

<400> 164
atctgcagaa ggtcaagaat atgtg 25

<210> 165
<211> 23
<212> DNA
<213> Artificial

<220>
<223> probe 1 for detecting the Ile105Val polymorphism in the GSTP1 gene

<400> 165
gctgcaaata catctccctc atc 23

<210> 166
<211> 23
<212> DNA
<213> Artificial

<220>
<223> probe 2 for detecting the Ile105Val polymorphism in the GSTP1

gene

<400> 166
gatgagggag atgtatttgc agc 23

<210> 167
<211> 23
<212> DNA
<213> Artificial

<220>
<223> probe 3 for detecting the Ile105Val polymorphism in the GSTP1 gene

<400> 167
gctgcaaata cgtctccctc atc 23

<210> 168
<211> 23
<212> DNA
<213> Artificial

<220>
<223> probe 4 for detecting the Ile105Val polymorphism in the GSTP1 gene

<400> 168
gatgagggag acgtatttgc agc 23

<210> 169
<211> 23
<212> DNA
<213> Artificial

<220>
<223> probe 1 for detecting the Ala114Val polymorphism in the GSTP1 gene

<400> 169
ctggcaggag gcgggcaagg atg 23

<210> 170
<211> 21
<212> DNA
<213> Artificial

<220>
<223> probe 2 for detecting the Ala114Val polymorphism in the GSTP1 gene

<400> 170
atccttgccc gcctcctgcc a 21

<210> 171
<211> 23

<212> DNA
<213> Artificial

<220>
<223> probe 3 for detecting the Ala114Val polymorphism in the GSTP1 gene

<400> 171
ctggcaggag gtgggcaagg atg 23

<210> 172
<211> 21
<212> DNA
<213> Artificial

<220>
<223> probe 4 for detecting the Ala114Val polymorphism in the GSTP1 gene

<400> 172
atccttgccc acctcctgcc a 21

<210> 173
<211> 25
<212> DNA
<213> Artificial

<220>
<223> probe 1 for detecting the GSTT1 polymorphism

<400> 173
ctgcctagtg ggttcacctg cccac 25

<210> 174
<211> 25
<212> DNA
<213> Artificial

<220>
<223> probe 2 for detecting the GSTT1 polymorphism

<400> 174
gtgggcaggt gaacccacta ggcag 25

<210> 175
<211> 25
<212> DNA
<213> Artificial

<220>
<223> probe 3 for detecting the GSTT1 polymorphism

<400> 175
ctgcctagtg ggtcacctg cccac 25

<210> 176
<211> 25
<212> DNA
<213> Artificial

<220>
<223> probe 4 for detecting the GSTT1 polymorphism

<400> 176
gtgggcaggt gaccccacta ggcag 25

<210> 177
<211> 23
<212> DNA
<213> Artificial

<220>
<223> probe 1 for detecting the -174 C>G polymorphism in the IL6 gene

<400> 177
ttgtgtcttg cgatgctaaa gga 23

<210> 178
<211> 23
<212> DNA
<213> Artificial

<220>
<223> probe 2 for detecting the -174 C>G polymorphism in the IL6 gene

<400> 178
tcctttagca tcgcaagaca caa 23

<210> 179
<211> 23
<212> DNA
<213> Artificial

<220>
<223> probe 3 for detecting the -174 C>G polymorphism in the IL6 gene

<400> 179
ttgtgtcttg ccatgctaaa gga 23

<210> 180
<211> 23
<212> DNA
<213> Artificial

<220>
<223> probe 4 for detecting the -174 C>G polymorphism in the IL6 gene

<400> 180
tcctttagca tggcaagaca caa 23

<210> 181
<211> 23
<212> DNA
<213> Artificial

<220>
<223> probe 1 for detecting the -1082 G>A polymorphism in the IL10 gene

<400> 181
cttctttggg aaggggaagt agg 23

<210> 182
<211> 23
<212> DNA
<213> Artificial

<220>
<223> probe 2 for detecting the -1082 G>A polymorphism in the IL10 gene

<400> 182
cctacttccc cttcccaaag aag 23

<210> 183
<211> 23
<212> DNA
<213> Artificial

<220>
<223> probe 3 for detecting the -1082 G>A polymorphism in the IL10 gene

<400> 183
cttctttggg agggggaagt agg 23

<210> 184
<211> 23
<212> DNA
<213> Artificial

<220>
<223> probe 4 for detecting the -1082 G>A polymorphism in the IL10 gene

<400> 184
cctacttccc cctcccaaag aag 23

<210> 185
<211> 21
<212> DNA
<213> Artificial

<220>
<223> probe 1 for detecting the Leu33Pro polymorphism in the ITGB3 gene

<400> 185
gccctgcctc tgggctcacc t 21

<210> 186
<211> 23
<212> DNA
<213> Artificial

<220>
<223> probe 2 for detecting the Leu33Pro polymorphism in the ITGB3 gene

<400> 186
gaggtgagcc cagaggcagg gcc 23

<210> 187
<211> 21
<212> DNA
<213> Artificial

<220>
<223> probe 3 for detecting the Leu33Pro polymorphism in the ITGB3 gene

<400> 187
gccctgcctc cgggctcacc t 21

<210> 188
<211> 23
<212> DNA
<213> Artificial

<220>
<223> probe 4 for detecting the Leu33Pro polymorphism in the ITGB3 gene

<400> 188
gaggtgagcc cggaggcagg gcc 23

<210> 189
<211> 23
<212> DNA
<213> Artificial

<220>
<223> probe 1 for detecting the 5A>6A polymorphism in the MMP3 gene

<400> 189
atgggggaa aaaaccatgt ctt 23

<210> 190
<211> 22
<212> DNA
<213> Artificial

<220>
<223> probe 2 for detecting the 5A>6A polymorphism in the MMP3 gene

<400> 190
ggggaaaaaa ccatgtcttg tc 22

<210> 191
<211> 23
<212> DNA
<213> Artificial

<220>
<223> probe 3 for detecting the 5A>6A polymorphism in the MMP3 gene

<400> 191
atggggggaa aaaccatgtc ttg 23

<210> 192
<211> 22
<212> DNA
<213> Artificial

<220>
<223> probe 4 for detecting the 5A>6A polymorphism in the MMP3 gene

<400> 192
ggggaaaaac catgtcttgt cc 22

<210> 193
<211> 21
<212> DNA
<213> Artificial

<220>
<223> probe 1 for detecting the Ala222Val polymorphism in the MTHFR gene

<400> 193
tctgcgggag ccgatttcat c 21

<210> 194
<211> 23
<212> DNA
<213> Artificial

<220>
<223> probe 2 for detecting the Ala222Val polymorphism in the MTHFR gene

<400> 194
tgatgaaatc ggctcccgca gac 23

<210> 195
<211> 21
<212> DNA
<213> Artificial

<220>
<223> probe 3 for detecting the Ala222Val polymorphism in the MTHFR gene

<400> 195

tctgcgggag tcgatttcat c 21

<210> 196
<211> 23
<212> DNA
<213> Artificial

<220>
<223> probe 4 for detecting the Ala222Val polymorphism in the MTHFR gene

<400> 196
tgatgaaatc gactcccgca gac 23

<210> 197
<211> 23
<212> DNA
<213> Artificial

<220>
<223> probe 1 for detecting the R64Q polymorphism in the NAT2 gene

<400> 197
accacccacc ccggtttctt ctt 23

<210> 198
<211> 21
<212> DNA
<213> Artificial

<220>
<223> probe 2 for detecting the R64Q polymorphism in the NAT2 gene

<400> 198
ccacccaccc cggtttcttc t 21

<210> 199
<211> 23
<212> DNA
<213> Artificial

<220>
<223> probe 3 for detecting the R64Q polymorphism in the NAT2 gene

<400> 199
accacccacc ctggtttctt ctt 23

<210> 200
<211> 21
<212> DNA
<213> Artificial

<220>
<223> probe 4 for detecting the R64Q polymorphism in the NAT2 gene

```
<400>  200
ccacccaccc tggtttcttc t                                               21


<210>  201
<211>  25
<212>  DNA
<213>  Artificial

<220>
<223>  probe 1 for detecting the 282 C>T (Y94Y) polymorphism in the NAT2
       gene

<400>  201
agggtatttt tacatccctc cagtt                                           25


<210>  202
<211>  23
<212>  DNA
<213>  Artificial

<220>
<223>  probe 2 for detecting the 282 C>T (Y94Y) polymorphism in the NAT2
       gene

<400>  202
gggtattttt acatccctcc agt                                             23


<210>  203
<211>  25
<212>  DNA
<213>  Artificial

<220>
<223>  probe 3 for detecting the 282 C>T (Y94Y) polymorphism in the NAT2
       gene

<400>  203
agggtatttt tatatccctc cagtt                                           25


<210>  204
<211>  23
<212>  DNA
<213>  Artificial

<220>
<223>  probe 4 for detecting the 282 C>T (Y94Y) polymorphism in the NAT2
       gene

<400>  204
gggtattttt atatccctcc agt                                             23


<210>  205
<211>  23
<212>  DNA
<213>  Artificial
```

<220>
<223>  probe 1 for detecting the I114T polymorphism in the NAT2 gene

<400>  205
gcaggtgacc attgacggca gga                                              23

<210>  206
<211>  21
<212>  DNA
<213>  Artificial

<220>
<223>  probe 2 for detecting the I114T polymorphism in the NAT2 gene

<400>  206
caggtgacca ttgacggcag g                                                21

<210>  207
<211>  23
<212>  DNA
<213>  Artificial

<220>
<223>  probe 3 for detecting the I114T polymorphism in the NAT2 gene

<400>  207
gcaggtgacc actgacggca gga                                              23

<210>  208
<211>  21
<212>  DNA
<213>  Artificial

<220>
<223>  probe 4 for detecting the I114T polymorphism in the NAT2 gene

<400>  208
caggtgacca ctgacggcag g                                                21

<210>  209
<211>  25
<212>  DNA
<213>  Artificial

<220>
<223>  probe 1 for detecting the 481C>T (L161L) polymorphism in the NAT2 gene

<400>  209
ggaatctggt acctggacca aatca                                            25

<210>  210
<211>  27
<212>  DNA

<213> Artificial

<220>
<223> probe 2 for detecting the 481C>T (L161L) polymorphism in the NAT2 gene

<400> 210
aggaatctgg tacctggacc aaatcag 27

<210> 211
<211> 25
<212> DNA
<213> Artificial

<220>
<223> probe 3 for detecting the 481C>T (L161L) polymorphism in the NAT2 gene

<400> 211
ggaatctggt acttggacca aatca 25

<210> 212
<211> 27
<212> DNA
<213> Artificial

<220>
<223> probe 4 for detecting the 481C>T (L161L) polymorphism in the NAT2 gene

<400> 212
aggaatctgg tacttggacc aaatcag 27

<210> 213
<211> 25
<212> DNA
<213> Artificial

<220>
<223> probe 1 for detecting the R197Q polymorphism in the NAT2 gene

<400> 213
cgcttgaacc tcgaacaatt gaaga 25

<210> 214
<211> 23
<212> DNA
<213> Artificial

<220>
<223> probe 2 for detecting the R197Q polymorphism in the NAT2 gene

<400> 214
gcttgaacct cgaacaattg aag 23

<210> 215
<211> 25
<212> DNA
<213> Artificial

<220>
<223> probe 3 for detecting the R197Q polymorphism in the NAT2 gene

<400> 215
cgcttgaacc tcaaacaatt gaaga 25

<210> 216
<211> 23
<212> DNA
<213> Artificial

<220>
<223> probe 4 for detecting the R197Q polymorphism in the NAT2 gene

<400> 216
gcttgaacct caaacaattg aag 23

<210> 217
<211> 25
<212> DNA
<213> Artificial

<220>
<223> probe 1 for detecting the K268R polymorphism in the NAT2 gene

<400> 217
aagaagtgct gaaaaatata tttaa 25

<210> 218
<211> 25
<212> DNA
<213> Artificial

<220>
<223> probe 2 for detecting the K268R polymorphism in the NAT2 gene

<400> 218
ttaaatatat ttttcagcac ttctt 25

<210> 219
<211> 25
<212> DNA
<213> Artificial

<220>
<223> probe 3 for detecting the K268R polymorphism in the NAT2 gene

<400> 219
aagaagtgct gagaaatata tttaa 25

<210> 220
<211> 25
<212> DNA
<213> Artificial

<220>
<223> probe 4 for detecting the K268R polymorphism in the NAT2 gene

<400> 220
ttaaatatat ttctcagcac ttctt 25

<210> 221
<211> 25
<212> DNA
<213> Artificial

<220>
<223> probe 1 for detecting the G286E polymorphism in the NAT2 gene

<400> 221
aacctggtga tggatccctt actat 25

<210> 222
<211> 23
<212> DNA
<213> Artificial

<220>
<223> probe 2 for detecting the G286E polymorphism in the NAT2 gene

<400> 222
acctggtgat ggatccctta cta 23

<210> 223
<211> 25
<212> DNA
<213> Artificial

<220>
<223> probe 3 for detecting the G286E polymorphism in the NAT2 gene

<400> 223
aacctggtga tgaatccctt actat 25

<210> 224
<211> 23
<212> DNA
<213> Artificial

<220>
<223> probe 4 for detecting the G286E polymorphism in the NAT2 gene

<400> 224
acctggtgat gaatccctta cta 23

<210> 225
<211> 23
<212> DNA
<213> Artificial

<220>
<223> probe 1 for detecting the -786 T>C polymorphism in the NOS3 gene

<400> 225
tcttccctgg ctggctgacc ctg 23

<210> 226
<211> 23
<212> DNA
<213> Artificial

<220>
<223> probe 2 for detecting the -786 T>C polymorphism in the NOS3 gene

<400> 226
cagggtcagc cagccaggga aga 23

<210> 227
<211> 23
<212> DNA
<213> Artificial

<220>
<223> probe 3 for detecting the -786 T>C polymorphism in the NOS3 gene

<400> 227
tcttccctgg ccggctgacc ctg 23

<210> 228
<211> 23
<212> DNA
<213> Artificial

<220>
<223> probe 4 for detecting the -786 T>C polymorphism in the NOS3 gene

<400> 228
cagggtcagc cggccaggga aga 23

<210> 229
<211> 23
<212> DNA
<213> Artificial

<220>
<223> probe 1 for detecting the Glu298Asp polymorphism in the NOS3 gene

<400> 229
gccccagatg agcccccaga act 23

<210>  230
<211>  23
<212>  DNA
<213>  Artificial

<220>
<223>  probe 2 for detecting the Glu298Asp polymorphism in the NOS3 gene

<400>  230
agttctgggg gctcatctgg ggc                                                23

<210>  231
<211>  23
<212>  DNA
<213>  Artificial

<220>
<223>  probe 3 for detecting the Glu298Asp polymorphism in the NOS3 gene

<400>  231
gccccagatg atcccccaga act                                                23

<210>  232
<211>  23
<212>  DNA
<213>  Artificial

<220>
<223>  probe 4 for detecting the Glu298Asp polymorphism in the NOS3 gene

<400>  232
agttctgggg gatcatctgg ggc                                                23

<210>  233
<211>  23
<212>  DNA
<213>  Artificial

<220>
<223>  probe 1 for detecting the Leu7Pro polymorphism in the NPY gene

<400>  233
cggacagccc cagtcgcttg tta                                                23

<210>  234
<211>  23
<212>  DNA
<213>  Artificial

<220>
<223>  probe 2 for detecting the Leu7Pro polymorphism in the NPY gene

<400>  234
taacaagcga ctggggctgt ccg                                                23

<210> 235
<211> 23
<212> DNA
<213> Artificial

<220>
<223> probe 3 for detecting the Leu7Pro polymorphism in the NPY gene

<400> 235
cggacagccc cggtcgcttg tta 23

<210> 236
<211> 23
<212> DNA
<213> Artificial

<220>
<223> probe 4 for detecting the Leu7Pro polymorphism in the NPY gene

<400> 236
taacaagcga ccggggctgt ccg 23

<210> 237
<211> 23
<212> DNA
<213> Artificial

<220>
<223> probe 1 for detecting the Cys326Ser polymorphism in the OGG1 gene

<400> 237
cctgcgccaa tcccgccatg ctc 23

<210> 238
<211> 21
<212> DNA
<213> Artificial

<220>
<223> probe 2 for detecting the Cys326Ser polymorphism in the OGG1 gene

<400> 238
ctgcgccaat cccgccatgc t 21

<210> 239
<211> 23
<212> DNA
<213> Artificial

<220>
<223> probe 3 for detecting the Cys326Ser polymorphism in the OGG1 gene

<400> 239
cctgcgccaa tgccgccatg ctc 23

<210>  240
<211>  21
<212>  DNA
<213>  Artificial

<220>
<223>  probe 4 for detecting the Cys326Ser polymorphism in the OGG1 gene

<400>  240
ctgcgccaat gccgccatgc t                                                21

<210>  241
<211>  17
<212>  DNA
<213>  Artificial

<220>
<223>  probe 1 for detecting the 4G>5G polymorphism in the PAI1 gene

<400>  241
ctgactcccc cacgtgt                                                17

<210>  242
<211>  17
<212>  DNA
<213>  Artificial

<220>
<223>  probe 2 for detecting the 4G>5G polymorphism in the PAI1 gene

<400>  242
ggctgactcc cccacgt                                                17

<210>  243
<211>  17
<212>  DNA
<213>  Artificial

<220>
<223>  probe 3 for detecting the 4G>5G polymorphism in the PAI1 gene

<400>  243
ctgactcccc cacgtgt                                                17

<210>  244
<211>  17
<212>  DNA
<213>  Artificial

<220>
<223>  probe 4 for detecting the 4G>5G polymorphism in the PAI1 gene

<400>  244
cggctgactc cccacgt                                                17

<210> 245
<211> 23
<212> DNA
<213> Artificial

<220>
<223> probe 1 for detecting the 331 G>A polymorphism in the PGR gene

<400> 245
cgggagataa aagagccgcg tgt 23

<210> 246
<211> 23
<212> DNA
<213> Artificial

<220>
<223> probe 2 for detecting the 331 G>A polymorphism in the PGR gene

<400> 246
acacgcggct cttttatctc ccg 23

<210> 247
<211> 23
<212> DNA
<213> Artificial

<220>
<223> probe 3 for detecting the 331 G>A polymorphism in the PGR gene

<400> 247
cgggagataa aggagccgcg tgt 23

<210> 248
<211> 23
<212> DNA
<213> Artificial

<220>
<223> probe 4 for detecting the 331 G>A polymorphism in the PGR gene

<400> 248
acacgcggct cctttatctc ccg 23

<210> 249
<211> 23
<212> DNA
<213> Artificial

<220>
<223> probe 1 for detecting the Gln192Arg polymorphism in the PON1 gene

<400> 249
cccctactta caatcctggg aga 23

<210> 250
<211> 23
<212> DNA
<213> Artificial

<220>
<223> probe 2 for detecting the Gln192Arg polymorphism in the PON1 gene

<400> 250
tctcccagga ttgtaagtag ggg 23

<210> 251
<211> 23
<212> DNA
<213> Artificial

<220>
<223> probe 3 for detecting the Gln192Arg polymorphism in the PON1 gene

<400> 251
cccctactta cgatcctggg aga 23

<210> 252
<211> 23
<212> DNA
<213> Artificial

<220>
<223> probe 4 for detecting the Gln192Arg polymorphism in the PON1 gene

<400> 252
tctcccagga tcgtaagtag ggg 23

<210> 253
<211> 23
<212> DNA
<213> Artificial

<220>
<223> probe 1 for detecting the Ala16Val polymorphism in the SOD2 gene

<400> 253
gataccccaa agccggagcc agc 23

<210> 254
<211> 21
<212> DNA
<213> Artificial

<220>
<223> probe 2 for detecting the Ala16Val polymorphism in the SOD2 gene

<400> 254
ataccccaaa gccggagcca g 21

<210> 255
<211> 23
<212> DNA
<213> Artificial

<220>
<223> probe 3 for detecting the Ala16Val polymorphism in the SOD2 gene

<400> 255
gataccccaa aaccggagcc agc 23

<210> 256
<211> 21
<212> DNA
<213> Artificial

<220>
<223> probe 4 for detecting the Ala16Val polymorphism in the SOD2 gene

<400> 256
ataccccaaa accggagcca g 21

<210> 257
<211> 23
<212> DNA
<213> Artificial

<220>
<223> probe 1 for detecting the Ala49Thr polymorphism in the SRD5A2 gene

<400> 257
cccgcctgcc agcccgcgcc gcc 23

<210> 258
<211> 21
<212> DNA
<213> Artificial

<220>
<223> probe 2 for detecting the Ala49Thr polymorphism in the SRD5A2 gene

<400> 258
ccgcctgcca gcccgcgccg c 21

<210> 259
<211> 23
<212> DNA
<213> Artificial

<220>
<223> probe 3 for detecting the Ala49Thr polymorphism in the SRD5A2 gene

<400> 259

cccgcctgcc aacccgcgcc gcc 23

<210> 260
<211> 21
<212> DNA
<213> Artificial

<220>
<223> probe 4 for detecting the Ala49Thr polymorphism in the SRD5A2 gene

<400> 260
ccgcctgcca acccgcgccg c 21

<210> 261
<211> 21
<212> DNA
<213> Artificial

<220>
<223> probe 1 for detecting the Val89Leu polymorphism in the SRD5A2 gene

<400> 261
cctcttctgc gtacattact t 21

<210> 262
<211> 19
<212> DNA
<213> Artificial

<220>
<223> probe 2 for detecting the Val89Leu polymorphism in the SRD5A2 gene

<400> 262
ctcttctgcg tacattact 19

<210> 263
<211> 21
<212> DNA
<213> Artificial

<220>
<223> probe 3 for detecting the Val89Leu polymorphism in the SRD5A2 gene

<400> 263
cctcttctgc ctacattact t 21

<210> 264
<211> 19
<212> DNA
<213> Artificial

<220>
<223> probe 4 for detecting the Val89Leu polymorphism in the SRD5A2 gene

<400> 264
ctcttctgcc tacattact 19

<210> 265
<211> 21
<212> DNA
<213> Artificial

<220>
<223> probe 1 for detecting the Gly595Ala polymorphism in the SREBF2 gene

<400> 265
gctgctgccg gcaacctaca a 21

<210> 266
<211> 21
<212> DNA
<213> Artificial

<220>
<223> probe 2 for detecting the Gly595Ala polymorphism in the SREBF2 gene

<400> 266
ttgtaggttg ccggcagcag c 21

<210> 267
<211> 21
<212> DNA
<213> Artificial

<220>
<223> probe 3 for detecting the Gly595Ala polymorphism in the SREBF2 gene

<400> 267
gctgctgccg ccaacctaca a 21

<210> 268
<211> 21
<212> DNA
<213> Artificial

<220>
<223> probe 4 for detecting the Gly595Ala polymorphism in the SREBF2 gene

<400> 268
ttgtaggttg gcggcagcag c 21

<210> 269
<211> 21
<212> DNA
<213> Artificial

<220>
<223> probe 1 for detecting the Arg213His polymorphism in the SULT1A1 gene

<400> 269
tttgtggggc gctccctgcc a 21

<210> 270
<211> 19
<212> DNA
<213> Artificial

<220>
<223> probe 2 for detecting the Arg213His polymorphism in the SULT1A1 gene

<400> 270
ttgtggggcg ctccctgcc 19

<210> 271
<211> 21
<212> DNA
<213> Artificial

<220>
<223> probe 3 for detecting the Arg213His polymorphism in the SULT1A1 gene

<400> 271
tttgtggggc actccctgcc a 21

<210> 272
<211> 19
<212> DNA
<213> Artificial

<220>
<223> probe 4 for detecting the Arg213His polymorphism in the SULT1A1 gene

<400> 272
ttgtggggca ctccctgcc 19

<210> 273
<211> 23
<212> DNA
<213> Artificial

<220>
<223> probe 1 for detecting the b>B polymorphism in the VDR gene

<400> 273
gacaggcctg cgcattccca ata 23

<210> 274
<211> 23
<212> DNA
<213> Artificial

<220>
<223> probe 2 for detecting the b>B polymorphism in the VDR gene

<400> 274
tattgggaat gcgcaggcct gtc 23

<210> 275
<211> 23
<212> DNA
<213> Artificial

<220>
<223> probe 3 for detecting the b>B polymorphism in the VDR gene

<400> 275
gacaggcctg cacattccca ata 23

<210> 276
<211> 23
<212> DNA
<213> Artificial

<220>
<223> probe 4 for detecting the b>B polymorphism in the VDR gene

<400> 276
tattgggaat gtgcaggcct gtc 23

<210> 277
<211> 20
<212> DNA
<213> Artificial

<220>
<223> oligonucleotide 1 for amplifying the fragment in which the Intron 16 ins/del polymorphism in the ACE gene may exist

<400> 277
gggactctgt aagccactgc 20

<210> 278
<211> 20
<212> DNA
<213> Artificial

<220>
<223> oligonucleotide 2 for amplifying the fragment in which the Intron

16 ins/del polymorphism in the ACE gene may exist

<400> 278
ccatgcccat aacaggtctt 20

<210> 279
<211> 21
<212> DNA
<213> Artificial

<220>
<223> oligonucleotide 1 for amplifying the fragment in which the Gly389Arg polymorphism in the ADRB1 gene may exist

<400> 279
cgccttcaac cccatcatct a 21

<210> 280
<211> 18
<212> DNA
<213> Artificial

<220>
<223> oligonucleotide 2 for amplifying the fragment in which the Gly389Arg polymorphism in the gene ADRB1 may exist

<400> 280
caggctcgag tcgctgtc 18

<210> 281
<211> 18
<212> DNA
<213> Artificial

<220>
<223> oligonucleotide 1 for amplifying the fragment in which the Gln27Glu polymorphism in the ADRB2 gene may exist

<400> 281
gctcacctgc cagactgc 18

<210> 282
<211> 20
<212> DNA
<213> Artificial

<220>
<223> oligonucleotide 2 for amplifying the fragment in which the Gln27Glu polymorphism in the ADRB2 gene may exist

<400> 282
gccaggacga tgagagacat 20

<210> 283
<211> 18

<212> DNA
<213> Artificial

<220>
<223> oligonucleotide 1 for amplifying the fragment in which the Gly16Arg polymorphism in the ADRB2 gene may exist

<400> 283
gctcacctgc cagactgc 18

<210> 284
<211> 20
<212> DNA
<213> Artificial

<220>
<223> oligonucleotide 2 for amplifying the fragment in which the Gly16Arg polymorphism in the ADRB2 gene may exist

<400> 284
gccaggacga tgagagacat 20

<210> 285
<211> 19
<212> DNA
<213> Artificial

<220>
<223> oligonucleotide 1 for amplifying the fragment in which the Trp64Arg polymorphism in the ADRB3 gene may exist

<400> 285
caataccgcc aacaccagt 19

<210> 286
<211> 19
<212> DNA
<213> Artificial

<220>
<223> oligonucleotide 2 for amplifying the fragment in which the Trp64Arg polymorphism in the ADRB3 gene may exist

<400> 286
cgaagtcacg aacacgttg 19

<210> 287
<211> 20
<212> DNA
<213> Artificial

<220>
<223> oligonucleotide 1 for amplifying the fragment in which the Met235Thr polymorphism in the AGT gene may exist

<400> 287

gaactggatg ttgctgctga 20

<210> 288
<211> 20
<212> DNA
<213> Artificial

<220>
<223> oligonucleotide 2 for amplifying the fragment in which the Met235Thr polymorphism in the AGT gene may exist

<400> 288
ttgccttacc ttggaagtgg 20

<210> 289
<211> 20
<212> DNA
<213> Artificial

<220>
<223> oligonucleotide 1 for amplifying the fragment in which the 1166 A>C polymorphism in the AGTR1 gene may exist

<400> 289
ccgcccctca gataatgtaa 20

<210> 290
<211> 20
<212> DNA
<213> Artificial

<220>
<223> oligonucleotide 2 for amplifying the fragment in which the 1166 A>C polymorphism in the AGTR1 gene may exist

<400> 290
gcaaaatgtg gctttgcttt 20

<210> 291
<211> 20
<212> DNA
<213> Artificial

<220>
<223> oligonucleotide 1 for amplifying the fragment in which the -75 G>A polymorphism in the APOA1 gene may exist

<400> 291
cacctccttc tcgcagtctc 20

<210> 292
<211> 20
<212> DNA
<213> Artificial

```
<220>
<223>  oligonucleotide 2 for amplifying the fragment in which the -75
       G>A polymorphism in the APOA1 gene may exist

<400>  292
gggacagagc tgatccttga                                                   20


<210>  293
<211>  29
<212>  DNA
<213>  Artificial

<220>
<223>  oligonucleotide 1 for amplifying the fragment in which the
       Arg3480Trp polymorphism in the APOB gene may exist

<400>  293
agcctcacct cttactttc cattgagtc                                          29


<210>  294
<211>  24
<212>  DNA
<213>  Artificial

<220>
<223>  oligonucleotide 2 for amplifying the fragment in which the
       Arg3480Trp polymorphism in the APOB gene may exist

<400>  294
cgttggtgaa aaagaggccc tcta                                              24


<210>  295
<211>  29
<212>  DNA
<213>  Artificial

<220>
<223>  oligonucleotide 1 for amplifying the fragment in which the
       Arg3500Gln polymorphism in the APOB gene may exist

<400>  295
agcctcacct cttactttc cattgagtc                                          29


<210>  296
<211>  24
<212>  DNA
<213>  Artificial

<220>
<223>  oligonucleotide 2 for amplifying the fragment in which the
       Arg3500Gln polymorphism in the APOB gene may exist

<400>  296
cgttggtgaa aaagaggccc tcta                                              24
```

<210> 297
<211> 29
<212> DNA
<213> Artificial

<220>
<223> oligonucleotide 1 for amplifying the fragment in which the Arg3531Cys polymorphism in the APOB gene may exist

<400> 297
agcctcacct cttacttttc cattgagtc 29

<210> 298
<211> 24
<212> DNA
<213> Artificial

<220>
<223> oligonucleotide 2 for amplifying the fragment in which the Arg3531Cys polymorphism in the APOB gene may exist

<400> 298
cgttggtgaa aaagaggccc tcta 24

<210> 299
<211> 18
<212> DNA
<213> Artificial

<220>
<223> oligonucleotide 1 for amplifying the fragment in which the Cys112Arg polymorphism in the APOE gene may exist

<400> 299
ctgtccaagg agctgcag 18

<210> 300
<211> 18
<212> DNA
<213> Artificial

<220>
<223> oligonucleotide 2 for amplifying the fragment in which the Cys112Arg polymorphism in the APOE gene may exist

<400> 300
ctgttccacc aggggccc 18

<210> 301
<211> 18
<212> DNA
<213> Artificial

<220>
<223> oligonucleotide 1 for amplifying the fragment in which the Arg158Cys polymorphism in the APOE gene may exist

<400> 301
ctgtccaagg agctgcag 18

<210> 302
<211> 18
<212> DNA
<213> Artificial

<220>
<223> oligonucleotide 2 for amplifying the fragment in which the Arg158Cys polymorphism in the APOE gene may exist

<400> 302
ctgttccacc aggggccc 18

<210> 303
<211> 18
<212> DNA
<213> Artificial

<220>
<223> oligonucleotide 1 for amplifying the fragment in which the 833 T>C polymorphism in the CBS gene may exist

<400> 303
gcttttgctg gccttgag 18

<210> 304
<211> 20
<212> DNA
<213> Artificial

<220>
<223> oligonucleotide 2 for amplifying the fragment in which the 833 T>C polymorphism in the CBS gene may exist

<400> 304
gggtgagtta caggctgcac 20

<210> 305
<211> 18
<212> DNA
<213> Artificial

<220>
<223> oligonucleotide 1 for amplifying the fragment in which the 844ins68 polymorphism in the CBS gene may exist

<400> 305
gcttttgctg gccttgag 18

<210> 306
<211> 20
<212> DNA

<213> Artificial

<220>
<223> oligonucleotide 2 for amplifying the fragment in which the 844ins68 polymorphism in the CBS gene may exist

<400> 306
gggtgagtta caggctgcac 20

<210> 307
<211> 20
<212> DNA
<213> Artificial

<220>
<223> oligonucleotide 1 for amplifying the fragment in which the TaqIB polymorphism in the CETP gene may exist

<400> 307
gcaaacagcc aggtataggg 20

<210> 308
<211> 20
<212> DNA
<213> Artificial

<220>
<223> oligonucleotide 2 for amplifying the fragment in which the TaqIB polymorphism in the CETP gene may exist

<400> 308
aagagactga ggcccagaga 20

<210> 309
<211> 20
<212> DNA
<213> Artificial

<220>
<223> oligonucleotide 1 for amplifying the fragment in which the Arg451Gln polymorphism in the CETP gene may exist

<400> 309
gcaaacagcc aggtataggg 20

<210> 310
<211> 20
<212> DNA
<213> Artificial

<220>
<223> oligonucleotide 2 for amplifying the fragment in which the Arg451Gln polymorphism in the CETP gene may exist

<400> 310
aagagactga ggcccagaga 20

<210> 311
<211> 20
<212> DNA
<213> Artificial

<220>
<223> oligonucleotide 1 for amplifying the fragment in which the 1546 G>T polymorphism in the COL1A1 gene may exist

<400> 311
agccgctccc attctcttag 20

<210> 312
<211> 20
<212> DNA
<213> Artificial

<220>
<223> oligonucleotide 2 for amplifying the fragment in which the 1546 G>T polymorphism in the COL1A1 gene may exist

<400> 312
gcgtggtaga gacaggagga 20

<210> 313
<211> 20
<212> DNA
<213> Artificial

<220>
<223> oligonucleotide 1 for amplifying the fragment in which the Val158Met (Allele*2) polymorphism in the COMT gene may exist

<400> 313
gggcctactg tggctactca 20

<210> 314
<211> 20
<212> DNA
<213> Artificial

<220>
<223> oligonucleotide 2 for amplifying the fragment in which the Val158Met (Allele*2) polymorphism in the COMT gene may exist

<400> 314
cccttttcc aggtctgaca 20

<210> 315
<211> 20
<212> DNA
<213> Artificial

<220>

<223> oligonucleotide 1 for amplifying the fragment in which the -34 A>G polymorphism in the CYP17A1 gene may exist

<400> 315
gggctccagg agaatctttc 20

<210> 316
<211> 20
<212> DNA
<213> Artificial

<220>
<223> oligonucleotide 2 for amplifying the fragment in which the -34 A>G polymorphism in the CYP17A1 gene may exist

<400> 316
agggtaagca gcaagagagc 20

<210> 317
<211> 20
<212> DNA
<213> Artificial

<220>
<223> oligonucleotide 1 for amplifying the fragment in which the 1558 C>T polymorphism in the CYP19A1 gene may exist

<400> 317
ccttgcaccc agatgagact 20

<210> 318
<211> 20
<212> DNA
<213> Artificial

<220>
<223> oligonucleotide 2 for amplifying the fragment in which the 1558 C>T polymorphism in the CYP19A1 gene may exist

<400> 318
ggcaaggatg gatgatttgt 20

<210> 319
<211> 20
<212> DNA
<213> Artificial

<220>
<223> oligonucleotide 1 for amplifying the fragment in which the Ile462Val polymorphism in the CYP1A1 gene may exist

<400> 319
tgatggtgct atcgacaagg 20

<210> 320

<211> 20
<212> DNA
<213> Artificial

<220>
<223> oligonucleotide 2 for amplifying the fragment in which the Ile462Val polymorphism in the CYP1A1 gene may exist

<400> 320
tttggaagtg ctcacagcag 20

<210> 321
<211> 20
<212> DNA
<213> Artificial

<220>
<223> oligonucleotide 1 for amplifying the fragment in which the T3801C polymorphism in the CYP1A1 gene may exist

<400> 321
ccgctgcact taagcagtct 20

<210> 322
<211> 19
<212> DNA
<213> Artificial

<220>
<223> oligonucleotide 2 for amplifying the fragment in which the T3801C polymorphism in the CYP1A1 gene may exist

<400> 322
ggccccaact actcagagg 19

<210> 323
<211> 20
<212> DNA
<213> Artificial

<220>
<223> oligonucleotide 1 for amplifying the fragment in which the Leu432Val polymorphism in the CYP1B1 gene may exist

<400> 323
acctctgtct tgggctacca 20

<210> 324
<211> 20
<212> DNA
<213> Artificial

<220>
<223> oligonucleotide 2 for amplifying the fragment in which the Leu432Val polymorphism in the CYP1B1 gene may exist

<400> 324
gccaggatgg agatgaagag 20

<210> 325
<211> 20
<212> DNA
<213> Artificial

<220>
<223> oligonucleotide 1 for amplifying the fragment in which the Allele*4 (Asn453Ser) polymorphism in the CYP1B1 gene may exist

<400> 325
acctctgtct tgggctacca 20

<210> 326
<211> 20
<212> DNA
<213> Artificial

<220>
<223> oligonucleotide 2 for amplifying the fragment in which the Allele*4 (Asn453Ser) polymorphism in the CYP1B1 gene may exist

<400> 326
gccaggatgg agatgaagag 20

<210> 327
<211> 20
<212> DNA
<213> Artificial

<220>
<223> oligonucleotide 1 for amplifying the fragment in which the Arg144Cys (allele*2) polymorphism in the CYP2C9 gene may exist

<400> 327
cctgggatct ccctcctagt 20

<210> 328
<211> 20
<212> DNA
<213> Artificial

<220>
<223> oligonucleotide 2 for amplifying the fragment in which the Arg144Cys (allele*2) polymorphism in the CYP2C9 gene may exist

<400> 328
ccacccttgg tttttctcaa 20

<210> 329
<211> 20
<212> DNA
<213> Artificial

<220>
<223> oligonucleotide 1 for amplifying the fragment in which the Ile359Leu (allele*3) polymorphism in the CYP2C9 gene may exist

<400> 329
ccacatgccc tacacagatg 20

<210> 330
<211> 20
<212> DNA
<213> Artificial

<220>
<223> oligonucleotide 2 for amplifying the fragment in which the p Ile359Leu (allele*3) olymorphism in the CYP2C9 gene may exist

<400> 330
tcgaaaacat ggagttgcag 20

<210> 331
<211> 21
<212> DNA
<213> Artificial

<220>
<223> oligonucleotide 1 for amplifying the fragment in which the 681 G>A (Pro227Pro) (allele*2) polymorphism in the CYP2C19 gene may exist

<400> 331
caaccagagc ttggcatatt g 21

<210> 332
<211> 20
<212> DNA
<213> Artificial

<220>
<223> oligonucleotide 2 for amplifying the fragment in which the 681 G>A (Pro227Pro) (allele*2) polymorphism in the CYP2C19 gene may exist

<400> 332
taaagtcccg agggttgttg 20

<210> 333
<211> 20
<212> DNA
<213> Artificial

<220>
<223> oligonucleotide 1 for amplifying the fragment in which the 2549 A>del (allele*3) polymorphism in the CYP2D6 gene may exist

<400> 333

```
gggcctgaga cttgtccagg                                                  20


<210>  334
<211>  20
<212>  DNA
<213>  Artificial

<220>
<223>  oligonucleotide 2 for amplifying the fragment in which the 2549
       A>del (allele*3) polymorphism in the CYP2D6 gene may exist

<400>  334
gccgagagca tactcgggac                                                  20


<210>  335
<211>  22
<212>  DNA
<213>  Artificial

<220>
<223>  oligonucleotide 1 for amplifying the fragment in which the 1846
       G>A / 1847 G>A (allele*4) polymorphism in the CYP2D6 gene may
       exist

<400>  335
ccacgcgcac gtgcccgtcc ca                                               22


<210>  336
<211>  24
<212>  DNA
<213>  Artificial

<220>
<223>  oligonucleotide 2 for amplifying the fragment in which the 1846
       G>A / 1847 G>A (allele*4) polymorphism in the CYP2D6 gene may
       exist

<400>  336
cctgcagaga ctcctcggtc tctc                                             24


<210>  337
<211>  22
<212>  DNA
<213>  Artificial

<220>
<223>  oligonucleotide 1 for amplifying the fragment in which the 1707
       del>T (allele*6) polymorphism in the CYP2D6 gene may exist

<400>  337
ccacgcgcac gtgcccgtcc ca                                               22


<210>  338
<211>  24
<212>  DNA
```

<213> Artificial

<220>
<223> oligonucleotide 2 for amplifying the fragment in which the 1707 del>T (allele*6) polymorphism in the CYP2D6 gene may exist

<400> 338
cctgcagaga ctcctcggtc tctc 24

<210> 339
<211> 20
<212> DNA
<213> Artificial

<220>
<223> oligonucleotide 1 for amplifying the fragment in which the Ala541Thr polymorphism in the ELAC2 gene may exist

<400> 339
ccgacacgtc tctgctactg 20

<210> 340
<211> 20
<212> DNA
<213> Artificial

<220>
<223> oligonucleotide 2 for amplifying the fragment in which the Ala541Thr polymorphism in the ELAC2 gene may exist

<400> 340
aacaaaagct ctgggcaagt 20

<210> 341
<211> 20
<212> DNA
<213> Artificial

<220>
<223> oligonucleotide 1 for amplifying the fragment in which the -397 T>C (PvuII) polymorphism in the ESR1 IVS1 gene may exist

<400> 341
agggttatgt ggcaatgacg 20

<210> 342
<211> 20
<212> DNA
<213> Artificial

<220>
<223> oligonucleotide 2 for amplifying the fragment in which the -397 T>C (PvuII) polymorphism in the ESR1 IVS1 gene may exist

<400> 342
accaatgctc atcccaactc 20

<210> 343
<211> 23
<212> DNA
<213> Artificial

<220>
<223> oligonucleotide 1 for amplifying the fragment in which the Val34Leu polymorphism in the F13A1 gene may exist

<400> 343
catgcctttt ctgttgtctt ctt 23

<210> 344
<211> 20
<212> DNA
<213> Artificial

<220>
<223> oligonucleotide 2 for amplifying the fragment in which the Val34Leu polymorphism in the F13A1 gene may exist

<400> 344
cccagtggag acagaggatg 20

<210> 345
<211> 25
<212> DNA
<213> Artificial

<220>
<223> oligonucleotide 1 for amplifying the fragment in which the -455 G>A polymorphism in the FGB gene may exist

<400> 345
gggtctttct gatgtgtatt tttca 25

<210> 346
<211> 21
<212> DNA
<213> Artificial

<220>
<223> oligonucleotide 2 for amplifying the fragment in which the -455 G>A polymorphism in the FGB gene may exist

<400> 346
gacctactca caaggcaacc a 21

<210> 347
<211> 20
<212> DNA
<213> Artificial

<220>

<223> oligonucleotide 1 for amplifying the fragment in which the 20210 G>A polymorphism in the FII gene may exist

<400> 347
gagagtaggg ggccactcat 20

<210> 348
<211> 18
<212> DNA
<213> Artificial

<220>
<223> oligonucleotide 2 for amplifying the fragment in which the 20210 G>A polymorphism in the FII gene may exist

<400> 348
cctgagccca gagagctg 18

<210> 349
<211> 20
<212> DNA
<213> Artificial

<220>
<223> oligonucleotide 1 for amplifying the fragment in which the Arg506Gln polymorphism in the FV Leiden gene may exist

<400> 349
gcccagtgct taacaagacc 20

<210> 350
<211> 22
<212> DNA
<213> Artificial

<220>
<223> oligonucleotide 2 for amplifying the fragment in which the Arg506Gln polymorphism in the FV Leiden gene may exist

<400> 350
cccattattt agccaggaga cc 22

<210> 351
<211> 20
<212> DNA
<213> Artificial

<220>
<223> oligonucleotide 1 for amplifying the fragment in which the Pro319Ser polymorphism in the GJA4 gene may exist

<400> 351
cctcctcaga cccttacacg 20

<210> 352

<211> 20
<212> DNA
<213> Artificial

<220>
<223> oligonucleotide 2 for amplifying the fragment in which the Pro319Ser polymorphism in the GJA4 gene may exist

<400> 352
gcagccagac ttctcaggac 20

<210> 353
<211> 20
<212> DNA
<213> Artificial

<220>
<223> oligonucleotide 1 for amplifying the fragment in which the 393 T>C (Ile131Ile) polymorphism in the GNAS gene may exist

<400> 353
agtacgtgct ggctccttgt 20

<210> 354
<211> 20
<212> DNA
<213> Artificial

<220>
<223> oligonucleotide 2 for amplifying the fragment in which the 393 T>C (Ile131Ile) polymorphism in the GNAS gene may exist

<400> 354
cacaagtcgg ggtgtagctt 20

<210> 355
<211> 18
<212> DNA
<213> Artificial

<220>
<223> oligonucleotide 1 for amplifying the fragment in which the 825 C>T (Ser275Ser) polymorphism in the GNB3 gene may exist

<400> 355
ctgccgcttg tttgacct 18

<210> 356
<211> 20
<212> DNA
<213> Artificial

<220>
<223> oligonucleotide 2 for amplifying the fragment in which the 825 C>T (Ser275Ser) polymorphism in the GNB3 gene may exist

<400> 356
cacacgctca gacttcatgg 20

<210> 357
<211> 21
<212> DNA
<213> Artificial

<220>
<223> oligonucleotide 1 for amplifying the fragment in which the GSTM1 polymorphism may exist

<400> 357
tgcttcacgt gttatggagg t 21

<210> 358
<211> 20
<212> DNA
<213> Artificial

<220>
<223> oligonucleotide 2 for amplifying the fragment in which the GSTM1 polymorphism may exist

<400> 358
gggctcaaat atacggtgga 20

<210> 359
<211> 20
<212> DNA
<213> Artificial

<220>
<223> oligonucleotide 1 for amplifying the fragment in which the Ile105Val polymorphism in the GSTP1 gene may exist

<400> 359
ctctatggga aggaccagca 20

<210> 360
<211> 20
<212> DNA
<213> Artificial

<220>
<223> oligonucleotide 2 for amplifying the fragment in which the Ile105Val polymorphism in the GSTP1 gene may exist

<400> 360
gaagcccctt tctttgttca 20

<210> 361
<211> 20
<212> DNA
<213> Artificial

<220>
<223> oligonucleotide 1 for amplifying the fragment in which the Ala114Val polymorphism in the GSTP1 gene may exist

<400> 361
gcaagcagag gagaatctgg 20

<210> 362
<211> 20
<212> DNA
<213> Artificial

<220>
<223> oligonucleotide 2 for amplifying the fragment in which the Ala114Val polymorphism in the GSTP1 gene may exist

<400> 362
ctcacctggt ctcccacaat 20

<210> 363
<211> 20
<212> DNA
<213> Artificial

<220>
<223> oligonucleotide 1 for amplifying the fragment in which the GSTT1 polymorphism may exist

<400> 363
ggcagcataa gcaggacttc 20

<210> 364
<211> 20
<212> DNA
<213> Artificial

<220>
<223> oligonucleotide 2 for amplifying the fragment in which the GSTT1 polymorphism may exist

<400> 364
ctgcagttgc tcgaggacaa 20

<210> 365
<211> 20
<212> DNA
<213> Artificial

<220>
<223> oligonucleotide 1 for amplifying the fragment in which the -174 C>G polymorphism in the IL6 gene may exist

<400> 365
gcctcaatga cgacctaagc 20

<210> 366
<211> 20
<212> DNA
<213> Artificial

<220>
<223> oligonucleotide 2 for amplifying the fragment in which the -174 C>G polymorphism in the IL6 gene may exist

<400> 366
tcatgggaaa atcccacatt 20

<210> 367
<211> 20
<212> DNA
<213> Artificial

<220>
<223> oligonucleotide 1 for amplifying the fragment in which the -1082 G>A polymorphism in the IL10 gene may exist

<400> 367
tccccaggta gagcaacact 20

<210> 368
<211> 20
<212> DNA
<213> Artificial

<220>
<223> oligonucleotide 2 for amplifying the fragment in which the -1082 G>A polymorphism in the IL10 gene may exist

<400> 368
atggaggctg gataggaggt 20

<210> 369
<211> 20
<212> DNA
<213> Artificial

<220>
<223> oligonucleotide 1 for amplifying the fragment in which the Leu33Pro polymorphism in the ITGB3 gene may exist

<400> 369
gctccaatgt acggggtaaa 20

<210> 370
<211> 20
<212> DNA
<213> Artificial

<220>
<223> oligonucleotide 2 for amplifying the fragment in which the

Leu33Pro polymorphism in the ITGB3 gene may exist

<400> 370
actcactggg aactcgatgg 20

<210> 371
<211> 20
<212> DNA
<213> Artificial

<220>
<223> oligonucleotide 1 for amplifying the fragment in which the 5A>6A polymorphism in the MMP3 gene may exist

<400> 371
tcactgccac cactctgttc 20

<210> 372
<211> 20
<212> DNA
<213> Artificial

<220>
<223> oligonucleotide 2 for amplifying the fragment in which the 5A>6A polymorphism in the MMP3 gene may exist

<400> 372
gcctcaacct ctcaaagtgc 20

<210> 373
<211> 20
<212> DNA
<213> Artificial

<220>
<223> oligonucleotide 1 for amplifying the fragment in which the Ala222Val polymorphism in the MTHFR gene may exist

<400> 373
gcctctcctg actgtcatcc 20

<210> 374
<211> 20
<212> DNA
<213> Artificial

<220>
<223> oligonucleotide 2 for amplifying the fragment in which the Ala222Val polymorphism in the MTHFR gene may exist

<400> 374
caaagcggaa gaatgtgtca 20

<210> 375
<211> 20

<212> DNA
<213> Artificial

<220>
<223> oligonucleotide 1 for amplifying the fragment in which the R64Q polymorphism in the NAT2 gene may exist

<400> 375
ccatggagtt gggcttagag 20

<210> 376
<211> 20
<212> DNA
<213> Artificial

<220>
<223> oligonucleotide 2 for amplifying the fragment in which the R64Q polymorphism in the NAT2 gene may exist

<400> 376
ccatgccagt gctgtatttg 20

<210> 377
<211> 20
<212> DNA
<213> Artificial

<220>
<223> oligonucleotide 1 for amplifying the fragment in which the 282 C>T (Y94Y) polymorphism in the NAT2 gene may exist

<400> 377
ccatggagtt gggcttagag 20

<210> 378
<211> 20
<212> DNA
<213> Artificial

<220>
<223> oligonucleotide 2 for amplifying the fragment in which the 282 C>T (Y94Y) polymorphism in the NAT2 gene may exist

<400> 378
ccatgccagt gctgtatttg 20

<210> 379
<211> 20
<212> DNA
<213> Artificial

<220>
<223> oligonucleotide 1 for amplifying the fragment in which the I114T polymorphism in the NAT2 gene may exist

<400> 379

ccatggagtt gggcttagag 20

<210> 380
<211> 20
<212> DNA
<213> Artificial

<220>
<223> oligonucleotide 2 for amplifying the fragment in which the I114T polymorphism in the NAT2 gene may exist

<400> 380
ccatgccagt gctgtatttg 20

<210> 381
<211> 19
<212> DNA
<213> Artificial

<220>
<223> oligonucleotide 1 for amplifying the fragment in which the 481C>T (L161L) polymorphism in the NAT2 gene may exist

<400> 381
caggtgcctt gcattttct 19

<210> 382
<211> 20
<212> DNA
<213> Artificial

<220>
<223> oligonucleotide 2 for amplifying the fragment in which the 481C>T (L161L) polymorphism in the NAT2 gene may exist

<400> 382
gatgaagccc accaaacagt 20

<210> 383
<211> 19
<212> DNA
<213> Artificial

<220>
<223> oligonucleotide 1 for amplifying the fragment in which the R197Q polymorphism in the NAT2 gene may exist

<400> 383
caggtgcctt gcattttct 19

<210> 384
<211> 20
<212> DNA
<213> Artificial

<220>
<223>  oligonucleotide 2 for amplifying the fragment in which the R197Q polymorphism in the NAT2 gene may exist

<400>  384
gatgaagccc accaaacagt    20

<210>  385
<211>  24
<212>  DNA
<213>  Artificial

<220>
<223>  oligonucleotide 1 for amplifying the fragment in which the K268R polymorphism in the NAT2 gene may exist

<400>  385
aaagacaata cagatctggt cgag    24

<210>  386
<211>  24
<212>  DNA
<213>  Artificial

<220>
<223>  oligonucleotide 2 for amplifying the fragment in which the K268R polymorphism in the NAT2 gene may exist

<400>  386
tcttcaaaat aacgtgaggg taga    24

<210>  387
<211>  24
<212>  DNA
<213>  Artificial

<220>
<223>  oligonucleotide 1 for amplifying the fragment in which the G286E polymorphism in the NAT2 gene may exist

<400>  387
aaagacaata cagatctggt cgag    24

<210>  388
<211>  24
<212>  DNA
<213>  Artificial

<220>
<223>  oligonucleotide 2 for amplifying the fragment in which the G286E polymorphism in the NAT2 gene may exist

<400>  388
tcttcaaaat aacgtgaggg taga    24

<210> 389
<211> 20
<212> DNA
<213> Artificial

<220>
<223> oligonucleotide 1 for amplifying the fragment in which the -786 T>C polymorphism in the NOS3 gene may exist

<400> 389
gtgtacccca cctgcattct 20

<210> 390
<211> 19
<212> DNA
<213> Artificial

<220>
<223> oligonucleotide 2 for amplifying the fragment in which the -786 T>C polymorphism in the NOS3 gene may exist

<400> 390
cccaccctgt cattcagtg 19

<210> 391
<211> 20
<212> DNA
<213> Artificial

<220>
<223> oligonucleotide 1 for amplifying the fragment in which the Glu298Asp polymorphism in the NOS3 gene may exist

<400> 391
gaaggcagga gacagtggat 20

<210> 392
<211> 20
<212> DNA
<213> Artificial

<220>
<223> oligonucleotide 2 for amplifying the fragment in which the Glu298Asp polymorphism in the NOS3 gene may exist

<400> 392
cagtcaatcc ctttggtgct 20

<210> 393
<211> 20
<212> DNA
<213> Artificial

<220>
<223> oligonucleotide 1 for amplifying the fragment in which the Leu7Pro polymorphism in the NPY gene may exist

<400> 393
ctctgcctgg tgatgaggtt 20

<210> 394
<211> 19
<212> DNA
<213> Artificial

<220>
<223> oligonucleotide 2 for amplifying the fragment in which the Leu7Pro polymorphism in the NPY gene may exist

<400> 394
ggtgctctga atccccaag 19

<210> 395
<211> 20
<212> DNA
<213> Artificial

<220>
<223> oligonucleotide 1 for amplifying the fragment in which the Cys326Ser polymorphism in the OGG1 gene may exist

<400> 395
tagtctcacc agccctgacc 20

<210> 396
<211> 20
<212> DNA
<213> Artificial

<220>
<223> oligonucleotide 2 for amplifying the fragment in which the Cys326Ser polymorphism in the OGG1 gene may exist

<400> 396
tggggaattt ctttgtccag 20

<210> 397
<211> 20
<212> DNA
<213> Artificial

<220>
<223> oligonucleotide 1 for amplifying the fragment in which the 4G>5G polymorphism in the PAI1 gene may exist

<400> 397
caacctcagc cagacaaggt 20

<210> 398
<211> 20
<212> DNA

<213> Artificial

<220>
<223> oligonucleotide 2 for amplifying the fragment in which the 4G>5G polymorphism in the PAI1 gene may exist

<400> 398
cagccacgtg attgtctagg 20

<210> 399
<211> 20
<212> DNA
<213> Artificial

<220>
<223> oligonucleotide 1 for amplifying the fragment in which the 331 G>A polymorphism in the PGR gene may exist

<400> 399
gcttcacagc atgcacgagt 20

<210> 400
<211> 20
<212> DNA
<213> Artificial

<220>
<223> oligonucleotide 2 for amplifying the fragment in which the 331 G>A polymorphism in the PGR gene may exist

<400> 400
tattgttgct gtgggacctg 20

<210> 401
<211> 20
<212> DNA
<213> Artificial

<220>
<223> oligonucleotide 1 for amplifying the fragment in which the Gln192Arg polymorphism in the PON1 gene may exist

<400> 401
tattgttgct gtgggacctg 20

<210> 402
<211> 20
<212> DNA
<213> Artificial

<220>
<223> oligonucleotide 2 for amplifying the fragment in which the Gln192Arg polymorphism in the PON1 gene may exist

<400> 402
caaatccttc tgccaccact 20

<210> 403
<211> 20
<212> DNA
<213> Artificial

<220>
<223> oligonucleotide 1 for amplifying the fragment in which the Ala16Val polymorphism in the SOD2 gene may exist

<400> 403
ggctgtgctt tctcgtcttc 20

<210> 404
<211> 19
<212> DNA
<213> Artificial

<220>
<223> oligonucleotide 2 for amplifying the fragment in which the Ala16Val polymorphism in the SOD2 gene may exist

<400> 404
ccgtagtcgt agggcaggt 19

<210> 405
<211> 18
<212> DNA
<213> Artificial

<220>
<223> oligonucleotide 1 for amplifying the fragment in which the Ala49Thr polymorphism in the SRD5A2 gene may exist

<400> 405
agcacacgga gagcctga 18

<210> 406
<211> 20
<212> DNA
<213> Artificial

<220>
<223> oligonucleotide 2 for amplifying the fragment in which the Ala49Thr polymorphism in the SRD5A2 gene may exist

<400> 406
aggggaaaaa cgctacctgt 20

<210> 407
<211> 18
<212> DNA
<213> Artificial

<220>

<223> oligonucleotide 1 for amplifying the fragment in which the Val89Leu polymorphism in the SRD5A2 gene may exist

<400> 407
agcacacgga gagcctga 18

<210> 408
<211> 20
<212> DNA
<213> Artificial

<220>
<223> oligonucleotide 2 for amplifying the fragment in which the Val89Leu polymorphism in the SRD5A2 gene may exist

<400> 408
aggggaaaaa cgctacctgt 20

<210> 409
<211> 20
<212> DNA
<213> Artificial

<220>
<223> oligonucleotide 1 for amplifying the fragment in which the Gly595Ala polymorphism in the SREBF2 gene may exist

<400> 409
ggccagtgac cattaacacc 20

<210> 410
<211> 20
<212> DNA
<213> Artificial

<220>
<223> oligonucleotide 2 for amplifying the fragment in which the Gly595Ala polymorphism in the SREBF2 gene may exist

<400> 410
tcttcaaagc ctgcctcagt 20

<210> 411
<211> 20
<212> DNA
<213> Artificial

<220>
<223> oligonucleotide 1 for amplifying the fragment in which the Arg213His polymorphism in the SULT1A1 gene may exist

<400> 411
gtaatccgag cctccactga 20

<210> 412

<211> 20
<212> DNA
<213> Artificial

<220>
<223> oligonucleotide 2 for amplifying the fragment in which the Arg213His polymorphism in the SULT1A1 gene may exist

<400> 412
gctgtggtcc atgaactcct 20

<210> 413
<211> 20
<212> DNA
<213> Artificial

<220>
<223> oligonucleotide 1 for amplifying the fragment in which the b>B polymorphism in the VDR gene may exist

<400> 413
cctcactgcc cttagctctg 20

<210> 414
<211> 20
<212> DNA
<213> Artificial

<220>
<223> oligonucleotide 2 for amplifying the fragment in which the b>B polymorphism in the VDR gene may exist

<400> 414
cccgcaagaa acctcaaata 20

<210> 415
<211> 50
<212> DNA
<213> Artificial

<220>
<223> nucleotide sequence of the external control CEH

<400> 415
gtcgtcaaga tgctaccgtt caggagtcgt caagatgcta ccgttcagga 50

<210> 416
<211> 19
<212> DNA
<213> Artificial

<220>
<223> oligonucleotide 1 for detecting the external control

<400> 416
cttgacgact cctgaacgg 19

<210> 417
<211> 19
<212> DNA
<213> Artificial

<220>
<223> oligonucleotido 2 for detecting the external control

<400> 417
cttgacgaca cctgaacgg 19

87

<110> Sabiobbi, S.L.
Progenika Biopharma, S.A,

<120> Método y producto para genotipado "in vitro" con aplicación en medicina antienvejecimiento

<130> P2700ES

<160> 417

<170> PatentIn version 3.3

<210> 1
<211> 20
<212> ADN
<213> Artificial

<220>
<223> sonda 1 para detectar el polimorfismo Intron 16 ins/del en el gen ACE

<400> 1
gattacaggc gtgatacagt cac 23

<210> 2
<211> 20
<212> ADN
<213> Artificial

<220>
<223> sonda 2 para detectar el polimorfismo Intron 16 ins/del en el gen ACE

<400> 2
gtgactgtat cacgcctgta atc 23

<210> 3
<211> 20
<212> ADN
<213> Artificial

<220>
<223> sonda 3 para detectar el polimorfismo Intron 16 ins/del en el gen ACE

<400> 3
agacctgctg cctatacagt cac 23

<210> 4
<211> 20
<212> ADN
<213> Artificial

<220>
<223> sonda 4 para detectar el polimorfismo Intron 16 ins/del en el gen ACE

<400> 4
gtgactgtat aggcagcagg tct 23

<210> 5
<211> 23
<212> ADN
<213> Artificial

<220>
<223> sonda 1 para detectar el polimorfismo ADRB1 Gly389Arg en el gen ADRB1

<400> 5
aggccttcca gcgactgctc tgc 23

<210> 6
<211> 23
<212> ADN
<213> Artificial

<220>
<223> sonda 2 para detectar el polimorfismo ADRB1 Gly389Arg en el gen ADRB1

<400> 6
gcagagcagt cgctggaagg cct 23

<210> 7
<211> 23
<212> ADN
<213> Artificial

<220>
<223> sonda 3 para detectar el polimorfismo ADRB1 Gly389Arg en el gen ADRB1

<400> 7
aggccttcca gggactgctc tgc 23

<210> 8
<211> 23
<212> ADN
<213> Artificial

<220>
<223> sonda 8 para detectar el polimorfismo ADRB1 Gly389Arg en el gen ADRB1

<400> 8
gcagagcagt ccctggaagg cct 23

<210> 9
<211> 21
<212> ADN
<213> Artificial

<220>
<223> sonda 1 para detectar el polimorfismo Gln27Glu en el gen ADRB2

<400> 9
acgtcacgca ggaaagggac gag 23

<210> 10
<211> 23
<212> ADN
<213> Artificial

<220>
<223> sonda 2 para detectar el polimorfismo Gln27Glu en el gen ADRB2

<400> 10
cgtcacgcag gaaagggacg a 21

<210> 11
<211> 21
<212> ADN
<213> Artificial

<220>
<223> sonda 3 para detectar el polimorfismo Gln27Glu en el gen ADRB2

<400> 11
acgtcacgca gcaaagggac gag 23

<210> 12
<211> 23
<212> ADN
<213> Artificial

<220>
<223> sonda 4 para detectar el polimorfismo Gln27Glu en el gen ADRB2

<400> 12
cgtcacgcag caaagggacg a 21

<210> 13
<211> 23
<212> ADN
<213> Artificial

<220>
<223> sonda 1 para detectar el polimorfismo Gly16Arg en el gen ADRB2

<400> 13
tggcacccaa tagaagccat gcg 23

<210> 14
<211> 25
<212> ADN
<213> Artificial

<220>
<223> sonda 2 para detectar el polimorfismo Gly16Arg en el gen ADRB2

<400> 14
ctggcaccca atagaagcca tgcgc 25

<210> 15
<211> 23
<212> ADN
<213> Artificial

<220>
<223> sonda 3 para detectar el polimorfismo Gly16Arg en el gen ADRB2

<400> 15
tggcacccaa tggaagccat gcg 23

<210> 16
<211> 25
<212> ADN
<213> Artificial

<220>
<223> sonda 4 para detectar el polimorfismo Gly16Arg en el gen ADRB2

<400> 16
ctggcaccca atggaagcca tgcgc 25

<210> 17
<211> 23
<212> ADN
<213> Artificial

<220>
<223> sonda 1 para detectar el polimorfismo Trp64Arg en el gen ADRB3

<400> 17
tggccatcgc ctggactccg aga 23

<210> 18
<211> 23
<212> ADN
<213> Artificial

<220>
<223> sonda 2 para detectar el polimorfismo Trp64Arg en el gen ADRB3

<400> 18
tctcggagtc caggcgatgg cca 23

<210> 19
<211> 23
<212> ADN
<213> Artificial

<220>

<223> sonda 3 para detectar el polimorfismo Trp64Arg en el gen ADRB3

<400> 19
tggccatcgc ccggactccg aga 23

<210> 20
<211> 23
<212> ADN
<213> Artificial

<220>
<223> sonda 4 para detectar el polimorfismo Trp64Arg en el gen ADRB3

<400> 20
tctcggagtc cgggcgatgg cca 23

<210> 21
<211> 25
<212> ADN
<213> Artificial

<220>
<223> sonda 1 para detectar el polimorfismo Met235Thr en el gen AGT

<400> 21
gctgctccct gacgggagcc agtgt 25

<210> 22
<211> 25
<212> ADN
<213> Artificial

<220>
<223> sonda 2 para detectar el polimorfismo Met235Thr en el gen AGT

<400> 22
cacactggct cccgtcaggg agcagcc 27

<210> 23
<211> 25
<212> ADN
<213> Artificial

<220>
<223> sonda 3 para detectar el polimorfismo Met235Thr en el gen AGT

<400> 23
gctgctccct gatgggagcc agtgt 25

<210> 24
<211> 25
<212> ADN
<213> Artificial

<220>
<223> sonda 4 para detectar el polimorfismo Met235Thr en el gen AGT

<400> 24
cacactggct cccatcaggg agcagcc 27

<210> 25
<211> 23
<212> ADN
<213> Artificial

<220>
<223> sonda 1 para detectar el polimorfismo 1166 A>C en el gen AGTR1

<400> 25
accaaatgag cattagctac ttt 23

<210> 26
<211> 23
<212> ADN
<213> Artificial

<220>
<223> sonda 2 para detectar el polimorfismo 1166 A>C en el gen AGTR1

<400> 26
aaagtagcta atgctcattt ggt 23

<210> 27
<211> 23
<212> ADN
<213> Artificial

<220>
<223> sonda 3 para detectar el polimorfismo 1166 A>C en el gen AGTR1

<400> 27
accaaatgag ccttagctac ttt 23

<210> 28
<211> 23
<212> ADN
<213> Artificial

<220>
<223> sonda 4 para detectar el polimorfismo 1166 A>C en el gen AGTR1

<400> 28
aaagtagcta aggctcattt ggt 23

<210> 29
<211> 21
<212> ADN
<213> Artificial

<220>
<223> sonda 1 para detectar el polimorfismo -75 G>A en el gen APOA1

<400> 29
agcccagccc cggccctgtt g 21

<210> 30
<211> 19
<212> ADN
<213> Artificial

<220>
<223> sonda 2 para detectar el polimorfismo -75 G>A en el gen APOA1

<400> 30
gcccagcccc ggccctgtt 19

<210> 31
<211> 21
<212> ADN
<213> Artificial

<220>
<223> sonda 3 para detectar el polimorfismo -75 G>A en el gen APOA1

<400> 31
agcccagccc tggccctgtt g 21

<210> 32
<211> 19
<212> ADN
<213> Artificial

<220>
<223> sonda 4 para detectar el polimorfismo -75 G>A en el gen APOA1

<400> 32
gcccagccct ggccctgtt 19

<210> 33
<211> 23
<212> ADN
<213> Artificial

<220>
<223> sonda 1 para detectar el polimorfismo Arg3480Trp en el gen APOB

<400> 33
cggttctttc tcgggaatat tca 23

<210> 34
<211> 23
<212> ADN
<213> Artificial

<220>
<223> sonda 2 para detectar el polimorfismo Arg3480Trp en el gen APOB

<400> 34

tgaatattcc cgagaaagaa ccg 23

<210> 35
<211> 23
<212> ADN
<213> Artificial

<220>
<223> sonda 3 para detectar el polimorfismo Arg3480Trp en el gen APOB

<400> 35
cggttctttc ttgggaatat tca 23

<210> 36
<211> 23
<212> ADN
<213> Artificial

<220>
<223> sonda 4 para detectar el polimorfismo Arg3480Trp en el gen APOB

<400> 36
tgaatattcc caagaaagaa ccg 23

<210> 37
<211> 23
<212> ADN
<213> Artificial

<220>
<223> sonda 1 para detectar el polimorfismo Arg3500Gln en el gen APOB

<400> 37
caagagcaca cggtcttcag tga 23

<210> 38
<211> 23
<212> ADN
<213> Artificial

<220>
<223> sonda 2 para detectar el polimorfismo Arg3500Gln en el gen APOB

<400> 38
tcactgaaga ccgtgtgctc ttg 23

<210> 39
<211> 23
<212> ADN
<213> Artificial

<220>
<223> sonda 3 para detectar el polimorfismo Arg3500Gln en el gen APOB

<400> 39
caagagcaca cagtcttcag tga 23

<210> 40
<211> 23
<212> ADN
<213> Artificial

<220>
<223> sonda 4 para detectar el polimorfismo Arg3500Gln en el gen APOB

<400> 40
tcactgaaga ctgtgtgctc ttg 23

<210> 41
<211> 23
<212> ADN
<213> Artificial

<220>
<223> sonda 1 para detectar el polimorfismo Arg3531Cys en el gen APOB

<400> 41
ccacactcca acgcatatat tcc 23

<210> 42
<211> 23
<212> ADN
<213> Artificial

<220>
<223> sonda 2 para detectar el polimorfismo Arg3531Cys en el gen APOB

<400> 42
ggaatatatg cgttggagtg tgg 23

<210> 43
<211> 23
<212> ADN
<213> Artificial

<220>
<223> sonda 3 para detectar el polimorfismo Arg3531Cys en el gen APOB

<400> 43
ccacactcca atgcatatat tcc 23

<210> 44
<211> 23
<212> ADN
<213> Artificial

<220>
<223> sonda 4 para detectar el polimorfismo Arg3531Cys en el gen APOB

<400> 44
ggaatatatg cattggagtg tgg 23

<210> 45
<211> 23
<212> ADN
<213> Artificial

<220>
<223> sonda 1 para detectar el polimorfismo Cys112Arg en el gen APOE

<400> 45
atggaggacg tgtgcggccg cctgg 25

<210> 46
<211> 23
<212> ADN
<213> Artificial

<220>
<223> sonda 2 para detectar el polimorfismo Cys112Arg en el gen APOE

<400> 46
ccaggcggcc gcacacgtcc tccat 25

<210> 47
<211> 23
<212> ADN
<213> Artificial

<220>
<223> sonda 3 para detectar el polimorfismo Cys112Arg en el gen APOE

<400> 47
atggaggacg tgcgcggccg cctgg 25

<210> 48
<211> 23
<212> ADN
<213> Artificial

<220>
<223> sonda 4 para detectar el polimorfismo Cys112Arg en el gen APOE

<400> 48
ccaggcggcc gcgcacgtcc tccat 25

<210> 49
<211> 23
<212> ADN
<213> Artificial

<220>
<223> sonda 1 para detectar el polimorfismo Arg158Cys en el gen APOE

<400> 49
gacctgcaga agcgcctggc agtgt 25

<210> 50
<211> 23
<212> ADN
<213> Artificial

<220>
<223> sonda 2 para detectar el polimorfismo Arg158Cys en el gen APOE

<400> 50
acactgccag gcgcttctgc aggtc 25

<210> 51
<211> 23
<212> ADN
<213> Artificial

<220>
<223> sonda 3 para detectar el polimorfismo Arg158Cys en el gen APOE

<400> 51
gacctgcaga agtgcctggc agtgt 25

<210> 52
<211> 23
<212> ADN
<213> Artificial

<220>
<223> sonda 4 para detectar el polimorfismo Arg158Cys en el gen APOE

<400> 52
acactgccag gcacttctgc aggtc 25

<210> 53
<211> 23
<212> ADN
<213> Artificial

<220>
<223> sonda 1 para detectar el polimorfismo 833 T>C en el gen CBS

<400> 53
gatccacccc agtgatctgc aga 23

<210> 54
<211> 21
<212> ADN
<213> Artificial

<220>
<223> sonda 2 para detectar el polimorfismo 833 T>C en el gen CBS

<400> 54
atccacccca gtgatctgca g 21

<210> 55

<211> 23
<212> ADN
<213> Artificial

<220>
<223> sonda 3 para detectar el polimorfismo 833 T>C en el gen CBS

<400> 55
gatccacccc aatgatctgc aga 23

<210> 56
<211> 21
<212> ADN
<213> Artificial

<220>
<223> sonda 4 para detectar el polimorfismo 833 T>C en el gen CBS

<400> 56
atccacccca atgatctgca g 21

<210> 57
<211> 21
<212> ADN
<213> Artificial

<220>
<223> sonda 1 para detectar el polimorfismo 844ins68 en el gen CBS

<400> 57
tggggtggat catccaggtg ggg 23

<210> 58
<211> 21
<212> ADN
<213> Artificial

<220>
<223> sonda 2 para detectar el polimorfismo 844ins68 en el gen CBS

<400> 58
ccccacctgg atgatccacc cca 23

<210> 59
<211> 21
<212> ADN
<213> Artificial

<220>
<223> sonda 3 para detectar el polimorfismo 844ins68 en el gen CBS

<400> 59
tggggtggat cccgaagggt cca 23

<210> 60
<211> 21

<212> ADN
<213> Artificial

<220>
<223> sonda 4 para detectar el polimorfismo 844ins68 en el gen CBS

<400> 60
tggacccttc gggatccacc cca 23

<210> 61
<211> 23
<212> ADN
<213> Artificial

<220>
<223> sonda 1 para detectar el polimorfismo TaqIB en el gen CETP

<400> 61
cactggggtt cgagttaggg ttc 23

<210> 62
<211> 23
<212> ADN
<213> Artificial

<220>
<223> sonda 2 para detectar el polimorfismo TaqIB en el gen CETP

<400> 62
gaaccctaac tcgaacccca gtg 23

<210> 63
<211> 23
<212> ADN
<213> Artificial

<220>
<223> sonda 3 para detectar el polimorfismo TaqIB en el gen CETP

<400> 63
cactggggtt caagttaggg ttc 23

<210> 64
<211> 23
<212> ADN
<213> Artificial

<220>
<223> sonda 4 para detectar el polimorfismo TaqIB en el gen CETP

<400> 64
gaaccctaac ttgaacccca gtg 23

<210> 65
<211> 23
<212> ADN

<213> Artificial

<220>
<223> sonda 1 para detectar el polimorfismo Arg451Gln en el gen CETP

<400> 65
gattatcact cgagatgtga gta 23

<210> 66
<211> 23
<212> ADN
<213> Artificial

<220>
<223> sonda 2 para detectar el polimorfismo Arg451Gln en el gen CETP

<400> 66
attatcactc gagatgtgag t 21

<210> 67
<211> 23
<212> ADN
<213> Artificial

<220>
<223> sonda 3 para detectar el polimorfismo Arg451Gln en el gen CETP

<400> 67
gattatcact caagatgtga gta 23

<210> 68
<211> 23
<212> ADN
<213> Artificial

<220>
<223> sonda 4 para detectar el polimorfismo Arg451Gln en el gen CETP

<400> 68
attatcactc aagatgtgag t 21

<210> 69
<211> 23
<212> ADN
<213> Artificial

<220>
<223> sonda 1 para detectar el polimorfismo 1546 G>T en el gen COL1A1

<400> 69
tcatcccgcc cccattccct ggg 23

<210> 70
<211> 21
<212> ADN
<213> Artificial

<220>
<223> sonda 2 para detectar el polimorfismo 1546 G>T en el gen COL1A1

<400> 70
catcccgccc ccattccctg g 21

<210> 71
<211> 23
<212> ADN
<213> Artificial

<220>
<223> sonda 3 para detectar el polimorfismo 1546 G>T en el gen COL1A1

<400> 71
tcatcccgcc cacattccct ggg 23

<210> 72
<211> 21
<212> ADN
<213> Artificial

<220>
<223> sonda 4 para detectar el polimorfismo 1546 G>T en el gen COL1A1

<400> 72
catcccgccc acattccctg g 21

<210> 73
<211> 23
<212> ADN
<213> Artificial

<220>
<223> sonda 1 para detectar el polimorfismo Val158Met (Alelo*2) en el gen COMT

<400> 73
atttcgctgg cgtgaaggac aag 23

<210> 74
<211> 23
<212> ADN
<213> Artificial

<220>
<223> sonda 2 para detectar el polimorfismo Val158Met (Alelo*2) en el gen COMT

<400> 74
cttgtccttc acgccagcga aat 23

<210> 75
<211> 23
<212> ADN

<213> Artificial

<220>
<223> sonda 3 para detectar el polimorfismo Val158Met (Alelo*2) en el gen COMT

<400> 75
atttcgctgg catgaaggac aag 23

<210> 76
<211> 23
<212> ADN
<213> Artificial

<220>
<223> sonda 4 para detectar el polimorfismo Val158Met (Alelo*2) en el gen COMT

<400> 76
cttgtccttc atgccagcga aat 23

<210> 77
<211> 21
<212> ADN
<213> Artificial

<220>
<223> sonda 1 para detectar el polimorfismo -34 A>G en el gen CYP17A1

<400> 77
tctactccac tgctgtctat c 21

<210> 78
<211> 23
<212> ADN
<213> Artificial

<220>
<223> sonda 2 para detectar el polimorfismo -34 A>G en el gen CYP17A1

<400> 78
agatagacag cagtggagta gaa 23

<210> 79
<211> 21
<212> ADN
<213> Artificial

<220>
<223> sonda 3 para detectar el polimorfismo -34 A>G en el gen CYP17A1

<400> 79
tctactccac cgctgtctat c 21

<210> 80
<211> 23

<212> ADN
<213> Artificial

<220>
<223> sonda 4 para detectar el polimorfismo -34 A>G en el gen CYP17A1

<400> 80
agatagacag cggtggagta gaa 23

<210> 81
<211> 23
<212> ADN
<213> Artificial

<220>
<223> sonda 1 para detectar el polimorfismo 1558 C>T en el gen CYP19A1

<400> 81
tggtcagtac ccactctgga gca 23

<210> 82
<211> 23
<212> ADN
<213> Artificial

<220>
<223> sonda 2 para detectar el polimorfismo 1558 C>T en el gen CYP19A1

<400> 82
tgctccagag tgggtactga cca 23

<210> 83
<211> 23
<212> ADN
<213> Artificial

<220>
<223> sonda 3 para detectar el polimorfismo 1558 C>T en el gen CYP19A1

<400> 83
tggtcagtac ctactctgga gca 23

<210> 84
<211> 23
<212> ADN
<213> Artificial

<220>
<223> sonda 4 para detectar el polimorfismo 1558 C>T en el gen CYP19A1

<400> 84
tgctccagag taggtactga cca 23

<210> 85
<211> 21
<212> ADN

<213> Artificial

<220>
<223> sonda 1 para detectar el polimorfismo Ile462Val en el gen CYP1A1

<400> 85
tcggtgagac cattgcccgc tgg 23

<210> 86
<211> 21
<212> ADN
<213> Artificial

<220>
<223> sonda 2 para detectar el polimorfismo Ile462Val en el gen CYP1A1

<400> 86
ccagcgggca atggtctcac cga 23

<210> 87
<211> 21
<212> ADN
<213> Artificial

<220>
<223> sonda 3 para detectar el polimorfismo Ile462Val en el gen CYP1A1

<400> 87
tcggtgagac cgttgcccgc tgg 23

<210> 88
<211> 21
<212> ADN
<213> Artificial

<220>
<223> sonda 4 para detectar el polimorfismo Ile462Val en el gen CYP1A1

<400> 88
ccagcgggca acggtctcac cga 23

<210> 89
<211> 19
<212> ADN
<213> Artificial

<220>
<223> sonda 1 para detectar el polimorfismo T3801C en el gen CYP1A1

<400> 89
tccacctcct gggctcaca 19

<210> 90
<211> 19
<212> ADN
<213> Artificial

<220>
<223> sonda 2 para detectar el polimorfismo T3801C en el gen CYP1A1

<400> 90
tccacctccc gggctcaca 19

<210> 91
<211> 19
<212> ADN
<213> Artificial

<220>
<223> sonda 3 para detectar el polimorfismo T3801C en el gen CYP1A1

<400> 91
tccacctcct gggctcaca 19

<210> 92
<211> 19
<212> ADN
<213> Artificial

<220>
<223> sonda 4 para detectar el polimorfismo T3801C en el gen CYP1A1

<400> 92
tccacctccc gggctcaca 19

<210> 93
<211> 21
<212> ADN
<213> Artificial

<220>
<223> sonda 1 para detectar el polimorfismo Leu432Val en el gen CYP1B1

<400> 93
aatcatgacc cactgaagtg gccta 25

<210> 94
<211> 21
<212> ADN
<213> Artificial

<220>
<223> sonda 2 para detectar el polimorfismo Leu432Val en el gen CYP1B1

<400> 94
taggccactt cagtgggtca tgatt 25

<210> 95
<211> 21
<212> ADN
<213> Artificial

<220>
<223> sonda 3 para detectar el polimorfismo Leu432Val en el gen CYP1B1

<400> 95
aatcatgacc cagtgaagtg gccta 25

<210> 96
<211> 21
<212> ADN
<213> Artificial

<220>
<223> sonda 4 para detectar el polimorfismo Leu432Val en el gen CYP1B1

<400> 96
taggccactt cactgggtca tgatt 25

<210> 97
<211> 23
<212> ADN
<213> Artificial

<220>
<223> sonda 1 para detectar el polimorfismo Alelo*4 (Asn453Ser)en el gen CYP1B1

<400> 97
cggcctcatc aacaaggacc tga 23

<210> 98
<211> 23
<212> ADN
<213> Artificial

<220>
<223> sonda 2 para detectar el polimorfismo Alelo*4 (Asn453Ser)en el gen CYP1B1

<400> 98
tcaggtcctt gttgatgagg ccg 23

<210> 99
<211> 23
<212> ADN
<213> Artificial

<220>
<223> sonda 3 para detectar el polimorfismo Alelo*4 (Asn453Ser)en el gen CYP1B1

<400> 99
cggcctcatc agcaaggacc tga 23

<210> 100
<211> 23
<212> ADN

<213> Artificial

<220>
<223> sonda 4 para detectar el polimorfismo Alelo*4 (Asn453Ser)en el gen CYP1B1

<400> 100
tcaggtcctt gctgatgagg ccg 23

<210> 101
<211> 23
<212> ADN
<213> Artificial

<220>
<223> sonda 1 para detectar el polimorfismo Arg144Cys (alelo*2) en el gen CYP2C9

<400> 101
gcattgagga ccgtgttcaa gag 23

<210> 102
<211> 23
<212> ADN
<213> Artificial

<220>
<223> sonda 2 para detectar el polimorfismo Arg144Cys (alelo*2) en el gen CYP2C9

<400> 102
ctcttgaaca cggtcctcaa tgc 23

<210> 103
<211> 23
<212> ADN
<213> Artificial

<220>
<223> sonda 3 para detectar el polimorfismo Arg144Cys (alelo*2) en el gen CYP2C9

<400> 103
gcattgagga ctgtgttcaa gag 23

<210> 104
<211> 23
<212> ADN
<213> Artificial

<220>
<223> sonda 4 para detectar el polimorfismo Arg144Cys (alelo*2) en el gen CYP2C9

<400> 104
ctcttgaaca cagtcctcaa tgc 23

<210> 105
<211> 23
<212> ADN
<213> Artificial

<220>
<223> sonda 1 para detectar el polimorfismo Ile359Leu (alelo*3) en el gen CYP2C9

<400> 105
tccagagata cattgacctt ctc 23

<210> 106
<211> 23
<212> ADN
<213> Artificial

<220>
<223> sonda 2 para detectar el polimorfismo Ile359Leu (alelo*3) en el gen CYP2C9

<400> 106
gagaaggtca atgtatctct gga 23

<210> 107
<211> 23
<212> ADN
<213> Artificial

<220>
<223> sonda 3 para detectar el polimorfismo Ile359Leu (alelo*3) en el gen CYP2C9

<400> 107
tccagagata ccttgacctt ctc 23

<210> 108
<211> 23
<212> ADN
<213> Artificial

<220>
<223> sonda 4 para detectar el polimorfismo Ile359Leu (alelo*3) en el gen CYP2C9

<400> 108
gagaaggtca aggtatctct gga 23

<210> 109
<211> 23
<212> ADN
<213> Artificial

<220>
<223> sonda 1 para detectar el polimorfismo 681 G>A (Pro227Pro) (alelo*2) en el gen CYP2C19

<400> 109
gattatttcc cgggaaccca taa 23

<210> 110
<211> 21
<212> ADN
<213> Artificial

<220>
<223> sonda 2 para detectar el polimorfismo 681 G>A (Pro227Pro) (alelo*2) en el gen CYP2C19

<400> 110
attatttccc gggaacccat a 21

<210> 111
<211> 23
<212> ADN
<213> Artificial

<220>
<223> sonda 3 para detectar el polimorfismo 681 G>A (Pro227Pro) (alelo*2) en el gen CYP2C19

<400> 111
gattatttcc caggaaccca taa 23

<210> 112
<211> 21
<212> ADN
<213> Artificial

<220>
<223> sonda 4 para detectar el polimorfismo 681 G>A (Pro227Pro) (alelo*2) en el gen CYP2C19

<400> 112
attatttccc aggaacccat a 21

<210> 113
<211> 23
<212> ADN
<213> Artificial

<220>
<223> sonda 1 para detectar el polimorfismo 2549 A>del (alelo*3) en el gen CYP2D6

<400> 113
ccaggtcatc ctgtgctcag tta 23

<210> 114
<211> 21
<212> ADN
<213> Artificial

<220>
<223> sonda 2 para detectar el polimorfismo 2549 A>del (alelo*3) en el gen CYP2D6

<400> 114
caggtcatcc tgtgctcagt t 21

<210> 115
<211> 23
<212> ADN
<213> Artificial

<220>
<223> sonda 3 para detectar el polimorfismo 2549 A>del (alelo*3) en el gen CYP2D6

<400> 115
ccaggtcatc cgtgctcagt tag 23

<210> 116
<211> 21
<212> ADN
<213> Artificial

<220>
<223> sonda 4 para detectar el polimorfismo 2549 A>del (alelo*3) en el gen CYP2D6

<400> 116
caggtcatcc gtgctcagtt a 21

<210> 117
<211> 21
<212> ADN
<213> Artificial

<220>
<223> sonda 1 para detectar el polimorfismo 1846 G>A / 1847 G>A (alelo*4) en el gen CYP2D6

<400> 117
cccaccccca ggacgcccct t 21

<210> 118
<211> 19
<212> ADN
<213> Artificial

<220>
<223> sonda 2 para detectar el polimorfismo 1846 G>A / 1847 G>A (alelo*4) en el gen CYP2D6

<400> 118
ccacccccag gacgcccct 19

<210> 119
<211> 21
<212> ADN
<213> Artificial

<220>
<223> sonda 3 para detectar el polimorfismo 1846 G>A / 1847 G>A (alelo*4) en el gen CYP2D6

<400> 119
cccacccccca agacgcccct t 21

<210> 120
<211> 19
<212> ADN
<213> Artificial

<220>
<223> sonda 4 para detectar el polimorfismo 1846 G>A / 1847 G>A (alelo*4) en el gen CYP2D6

<400> 120
ccaccccccaa gacgcccct 19

<210> 121
<211> 21
<212> ADN
<213> Artificial

<220>
<223> sonda 1 para detectar el polimorfismo 1707 del>T (alelo*6) en el gen CYP2D6

<400> 121
gctggagcag tgggtgaccg a 21

<210> 122
<211> 21
<212> ADN
<213> Artificial

<220>
<223> sonda 2 para detectar el polimorfismo 1707 del>T (alelo*6) en el gen CYP2D6

<400> 122
ctggagcagt gggtgaccg 19

<210> 123
<211> 21
<212> ADN
<213> Artificial

<220>
<223> sonda 3 para detectar el polimorfismo 1707 del>T (alelo*6) en el gen CYP2D6

<400> 123
cgctggagca ggggtgaccg a 21

<210> 124
<211> 21
<212> ADN
<213> Artificial

<220>
<223> sonda 4 para detectar el polimorfismo 1707 del>T (alelo*6) en el gen CYP2D6

<400> 124
gctggagcag gggtgaccg 19

<210> 125
<211> 23
<212> ADN
<213> Artificial

<220>
<223> sonda 1 para detectar el polimorfismo Ala541Thr en el gen ELAC2

<400> 125
gcaccctggc tgctgtgttt gtg 23

<210> 126
<211> 23
<212> ADN
<213> Artificial

<220>
<223> sonda 2 para detectar el polimorfismo Ala541Thr en el gen ELAC2

<400> 126
cacaaacaca gcagccaggg tgc 23

<210> 127
<211> 23
<212> ADN
<213> Artificial

<220>
<223> sonda 3 para detectar el polimorfismo Ala541Thr en el gen ELAC2

<400> 127
gcaccctggc tactgtgttt gtg 23

<210> 128
<211> 23
<212> ADN
<213> Artificial

<220>
<223> sonda 4 para detectar el polimorfismo Ala541Thr en el gen ELAC2

<400> 128
cacaaacaca gtagccaggg tgc 23

<210> 129
<211> 23
<212> ADN
<213> Artificial

<220>
<223> sonda 1 para detectar el polimorfismo -397 T>C (PvuII) en el gen ESR1 IVS1

<400> 129
aatgtcccag ctgttttatg ctt 23

<210> 130
<211> 21
<212> ADN
<213> Artificial

<220>
<223> sonda 2 para detectar el polimorfismo -397 T>C (PvuII) en el gen ESR1 IVS1

<400> 130
atgtcccagc tgttttatgc t 21

<210> 131
<211> 23
<212> ADN
<213> Artificial

<220>
<223> sonda 3 para detectar el polimorfismo -397 T>C (PvuII) en el gen ESR1 IVS1

<400> 131
aatgtcccag ccgttttatg ctt 23

<210> 132
<211> 21
<212> ADN
<213> Artificial

<220>
<223> sonda 4 para detectar el polimorfismo -397 T>C (PvuII) en el gen ESR1 IVS1

<400> 132
atgtcccagc cgttttatgc t 21

<210> 133
<211> 23
<212> ADN
<213> Artificial

<220>
<223> sonda 1 para detectar el polimorfismo Val34Leu en el gen F13A1

<400> 133
agcttcaggg cgtggtgccc cgg 23

<210> 134
<211> 21
<212> ADN
<213> Artificial

<220>
<223> sonda 2 para detectar el polimorfismo Val34Leu en el gen F13A1

<400> 134
gcttcagggc gtggtgcccc g 21

<210> 135
<211> 23
<212> ADN
<213> Artificial

<220>
<223> sonda 3 para detectar el polimorfismo Val34Leu en el gen F13A1

<400> 135
agcttcaggg cttggtgccc cgg 23

<210> 136
<211> 21
<212> ADN
<213> Artificial

<220>
<223> sonda 4 para detectar el polimorfismo Val34Leu en el gen F13A1

<400> 136
gcttcagggc ttggtgcccc g 21

<210> 137
<211> 23
<212> ADN
<213> Artificial

<220>
<223> sonda 1 para detectar el polimorfismo -455 G>A en el gen FGB

<400> 137
ttgattttaa tggccccttt tga 23

<210> 138
<211> 23
<212> ADN
<213> Artificial

<220>

<223> sonda 2 para detectar el polimorfismo -455 G>A en el gen FGB

<400> 138
tcaaaagggg ccattaaaat caa 23

<210> 139
<211> 23
<212> ADN
<213> Artificial

<220>
<223> sonda 3 para detectar el polimorfismo -455 G>A en el gen FGB

<400> 139
ttgattttaa tagccccttt tga 23

<210> 140
<211> 23
<212> ADN
<213> Artificial

<220>
<223> sonda 4 para detectar el polimorfismo -455 G>A en el gen FGB

<400> 140
tcaaaagggg ctattaaaat caa 23

<210> 141
<211> 23
<212> ADN
<213> Artificial

<220>
<223> sonda 1 para detectar el polimorfismo 20210 G>A en el gen FII

<400> 141
tgactctcag cgagcctcaa tgc 23

<210> 142
<211> 23
<212> ADN
<213> Artificial

<220>
<223> sonda 2 para detectar el polimorfismo 20210 G>A en el gen FII

<400> 142
gcattgaggc tcgctgagag tca 23

<210> 143
<211> 23
<212> ADN
<213> Artificial

<220>
<223> sonda 3 para detectar el polimorfismo 20210 G>A en el gen FII

<400> 143
tgactctcag caagcctcaa tgc 23

<210> 144
<211> 23
<212> ADN
<213> Artificial

<220>
<223> sonda 4 para detectar el polimorfismo 20210 G>A en el gen FII

<400> 144
gcattgaggc ttgctgagag tca 23

<210> 145
<211> 23
<212> ADN
<213> Artificial

<220>
<223> sonda 1 para detectar el polimorfismo Arg506Gln en el gen FV Leiden

<400> 145
cctggacagg cgaggaatac agg 23

<210> 146
<211> 23
<212> ADN
<213> Artificial

<220>
<223> sonda 2 para detectar el polimorfismo Arg506Gln en el gen FV Leiden

<400> 146
cctgtattcc tcgcctgtcc agg 23

<210> 147
<211> 23
<212> ADN
<213> Artificial

<220>
<223> sonda 3 para detectar el polimorfismo Arg506Gln en el gen FV Leiden

<400> 147
cctggacagg caaggaatac agg 23

<210> 148
<211> 23
<212> ADN
<213> Artificial

<220>
<223> sonda 4 para detectar el polimorfismo Arg506Gln en el gen FV Leiden

<400> 148
cctgtattcc ttgcctgtcc agg 23

<210> 149
<211> 23
<212> ADN
<213> Artificial

<220>
<223> sonda 1 para detectar el polimorfismo Pro319Ser en el gen GJA4

<400> 149
atggccaaaa acccccaagt cgt 23

<210> 150
<211> 23
<212> ADN
<213> Artificial

<220>
<223> sonda 2 para detectar el polimorfismo Pro319Ser en el gen GJA4

<400> 150
acgacttggg ggtttttggc cat 23

<210> 151
<211> 23
<212> ADN
<213> Artificial

<220>
<223> sonda 3 para detectar el polimorfismo Pro319Ser en el gen GJA4

<400> 151
atggccaaaa atccccaagt cgt 23

<210> 152
<211> 23
<212> ADN
<213> Artificial

<220>
<223> sonda 4 para detectar el polimorfismo Pro319Ser en el gen GJA4

<400> 152
acgacttggg gatttttggc cat 23

<210> 153
<211> 23
<212> ADN
<213> Artificial

<220>
<223> sonda 1 para detectar el polimorfismo 393 T>C (Ile131Ile) en el gen GNAS

<400> 153
gtggactaca ttctgagtgt gat 23

<210> 154
<211> 23
<212> ADN
<213> Artificial

<220>
<223> sonda 2 para detectar el polimorfismo 393 T>C (Ile131Ile) en el gen GNAS

<400> 154
atcacactca gaatgtagtc cac 23

<210> 155
<211> 23
<212> ADN
<213> Artificial

<220>
<223> sonda 3 para detectar el polimorfismo 393 T>C (Ile131Ile) en el gen GNAS

<400> 155
gtggactaca tcctgagtgt gat 23

<210> 156
<211> 23
<212> ADN
<213> Artificial

<220>
<223> sonda 4 para detectar el polimorfismo 393 T>C (Ile131Ile) en el gen GNAS

<400> 156
atcacactca ggatgtagtc cac 23

<210> 157
<211> 23
<212> ADN
<213> Artificial

<220>
<223> sonda 1 para detectar el polimorfismo 825 C>T (Ser275Ser) en el gen GNB3

<400> 157
ggcatcacgt ccgtggcctt ctc 23

<210> 158

<211> 23
<212> ADN
<213> Artificial

<220>
<223> sonda 2 para detectar el polimorfismo 825 C>T (Ser275Ser) en el gen GNB3

<400> 158
gagaaggcca cggacgtgat gcc 23

<210> 159
<211> 23
<212> ADN
<213> Artificial

<220>
<223> sonda 3 para detectar el polimorfismo 825 C>T (Ser275Ser) en el gen GNB3

<400> 159
ggcatcacgt ctgtggcctt ctc 23

<210> 160
<211> 23
<212> ADN
<213> Artificial

<220>
<223> sonda 4 para detectar el polimorfismo 825 C>T (Ser275Ser) en el gen GNB3

<400> 160
gagaaggcca cagacgtgat gcc 23

<210> 161
<211> 23
<212> ADN
<213> Artificial

<220>
<223> sonda 1 para detectar el polimorfismo GSTM1

<400> 161
cacatattct tggccttctg cagat 25

<210> 162
<211> 23
<212> ADN
<213> Artificial

<220>
<223> sonda 2 para detectar el polimorfismo GSTM1

<400> 162
atctgcagaa ggccaagaat atgtg 25

<210> 163
<211> 23
<212> ADN
<213> Artificial

<220>
<223> sonda 3 para detectar el polimorfismo GSTM1

<400> 163
cacatattct tgaccttctg cagat 25

<210> 164
<211> 23
<212> ADN
<213> Artificial

<220>
<223> sonda 4 para detectar el polimorfismo GSTM1

<400> 164
atctgcagaa ggtcaagaat atgtg 25

<210> 165
<211> 23
<212> ADN
<213> Artificial

<220>
<223> sonda 1 para detectar el polimorfismo Ile105Val en el gen GSTP1

<400> 165
gctgcaaata catctccctc atc 23

<210> 166
<211> 23
<212> ADN
<213> Artificial

<220>
<223> sonda 2 para detectar el polimorfismo Ile105Val en el gen GSTP1

<400> 166
gatgagggag atgtatttgc agc 23

<210> 167
<211> 23
<212> ADN
<213> Artificial

<220>
<223> sonda 3 para detectar el polimorfismo Ile105Val en el gen GSTP1

<400> 167
gctgcaaata cgtctccctc atc 23

<210> 168
<211> 23
<212> ADN
<213> Artificial

<220>
<223> sonda 4 para detectar el polimorfismo Ile105Val en el gen GSTP1

<400> 168
gatgagggag acgtatttgc agc 23

<210> 169
<211> 23
<212> ADN
<213> Artificial

<220>
<223> sonda 1 para detectar el polimorfismo Ala114Val en el gen GSTP1

<400> 169
ctggcaggag gcgggcaagg atg 23

<210> 170
<211> 23
<212> ADN
<213> Artificial

<220>
<223> sonda 2 para detectar el polimorfismo Ala114Val en el gen GSTP1

<400> 170
atccttgccc gcctcctgcc a 21

<210> 171
<211> 23
<212> ADN
<213> Artificial

<220>
<223> sonda 3 para detectar el polimorfismo Ala114Val en el gen GSTP1

<400> 171
ctggcaggag gtgggcaagg atg 23

<210> 172
<211> 23
<212> ADN
<213> Artificial

<220>
<223> sonda 4 para detectar el polimorfismo Ala114Val en el gen GSTP1

<400> 172
atccttgccc acctcctgcc a 21

<210> 173

<211> 23
<212> ADN
<213> Artificial

<220>
<223> sonda 1 para detectar el polimorfismo GSTT1

<400> 173
ctgcctagtg ggttcacctg cccac 25

<210> 174
<211> 23
<212> ADN
<213> Artificial

<220>
<223> sonda 2 para detectar el polimorfismo GSTT1

<400> 174
gtgggcaggt gaacccacta ggcag 25

<210> 175
<211> 23
<212> ADN
<213> Artificial

<220>
<223> sonda 3 para detectar el polimorfismo GSTT1

<400> 175
ctgcctagtg gggtcacctg cccac 25

<210> 176
<211> 23
<212> ADN
<213> Artificial

<220>
<223> sonda 4 para detectar el polimorfismo GSTT1

<400> 176
gtgggcaggt gaccccacta ggcag 25

<210> 177
<211> 23
<212> ADN
<213> Artificial

<220>
<223> sonda 1 para detectar el polimorfismo -174 C>G en el gen IL6

<400> 177
ttgtgtcttg cgatgctaaa gga 23

<210> 178
<211> 23

<212> ADN
<213> Artificial

<220>
<223> sonda 2 para detectar el polimorfismo -174 C>G en el gen IL6

<400> 178
tcctttagca tcgcaagaca caa 23

<210> 179
<211> 23
<212> ADN
<213> Artificial

<220>
<223> sonda 3 para detectar el polimorfismo -174 C>G en el gen IL6

<400> 179
ttgtgtcttg ccatgctaaa gga 23

<210> 180
<211> 23
<212> ADN
<213> Artificial

<220>
<223> sonda 4 para detectar el polimorfismo -174 C>G en el gen IL6

<400> 180
tcctttagca tggcaagaca caa 23

<210> 181
<211> 23
<212> ADN
<213> Artificial

<220>
<223> sonda 1 para detectar el polimorfismo -1082 G>A en el gen IL10

<400> 181
cttctttggg aaggggaagt agg 23

<210> 182
<211> 23
<212> ADN
<213> Artificial

<220>
<223> sonda 2 para detectar el polimorfismo -1082 G>A en el gen IL10

<400> 182
cctacttccc cttcccaaag aag 23

<210> 183
<211> 23
<212> ADN

<213> Artificial

<220>
<223> sonda 3 para detectar el polimorfismo -1082 G>A en el gen IL10

<400> 183
cttctttggg agggggaagt agg 23

<210> 184
<211> 23
<212> ADN
<213> Artificial

<220>
<223> sonda 4 para detectar el polimorfismo -1082 G>A en el gen IL10

<400> 184
cctacttccc cctcccaaag aag 23

<210> 185
<211> 21
<212> ADN
<213> Artificial

<220>
<223> sonda 1 para detectar el polimorfismo Leu33Pro en el gen ITGB3

<400> 185
gccctgcctc tgggctcacc t 21

<210> 186
<211> 23
<212> ADN
<213> Artificial

<220>
<223> sonda 2 para detectar el polimorfismo Leu33Pro en el gen ITGB3

<400> 186
gaggtgagcc cagaggcagg gcc 23

<210> 187
<211> 21
<212> ADN
<213> Artificial

<220>
<223> sonda 3 para detectar el polimorfismo Leu33Pro en el gen ITGB3

<400> 187
gccctgcctc cgggctcacc t 21

<210> 188
<211> 23
<212> ADN
<213> Artificial

<220>
<223> sonda 4 para detectar el polimorfismo Leu33Pro en el gen ITGB3

<400> 188
gaggtgagcc cggaggcagg gcc 23

<210> 189
<211> 23
<212> ADN
<213> Artificial

<220>
<223> sonda 1 para detectar el polimorfismo 5A>6A en el gen MMP3

<400> 189
atggggggaa aaaaccatgt ctt 23

<210> 190
<211> 22
<212> ADN
<213> Artificial

<220>
<223> sonda 2 para detectar el polimorfismo 5A>6A en el gen MMP3

<400> 190
ggggaaaaaa ccatgtcttg tc 22

<210> 191
<211> 23
<212> ADN
<213> Artificial

<220>
<223> sonda 3 para detectar el polimorfismo 5A>6A en el gen MMP3

<400> 191
atggggggaa aaaccatgtc ttg 23

<210> 192
<211> 22
<212> ADN
<213> Artificial

<220>
<223> sonda 4 para detectar el polimorfismo 5A>6A en el gen MMP3

<400> 192
ggggaaaaac catgtcttgt cc 22

<210> 193
<211> 23
<212> ADN
<213> Artificial

<220>
<223> sonda 1 para detectar el polimorfismo Ala222Val en el gen MTHFR

<400> 193
tctgcgggag ccgatttcat c 21

<210> 194
<211> 23
<212> ADN
<213> Artificial

<220>
<223> sonda 2 para detectar el polimorfismo Ala222Val en el gen MTHFR

<400> 194
tgatgaaatc ggctcccgca gac 23

<210> 195
<211> 23
<212> ADN
<213> Artificial

<220>
<223> sonda 3 para detectar el polimorfismo Ala222Val en el gen MTHFR

<400> 195
tctgcgggag tcgatttcat c 21

<210> 196
<211> 23
<212> ADN
<213> Artificial

<220>
<223> sonda 4 para detectar el polimorfismo Ala222Val en el gen MTHFR

<400> 196
tgatgaaatc gactcccgca gac 23

<210> 197
<211> 23
<212> ADN
<213> Artificial

<220>
<223> sonda 1 para detectar el polimorfismo R64Q en el gen NAT2

<400> 197
accacccacc ccggtttctt ctt 23

<210> 198
<211> 21
<212> ADN
<213> Artificial

<220>

<223> sonda 2 para detectar el polimorfismo R64Q en el gen NAT2

<400> 198
ccacccaccc cggtttcttc t 21

<210> 199
<211> 23
<212> ADN
<213> Artificial

<220>
<223> sonda 3 para detectar el polimorfismo R64Q en el gen NAT2

<400> 199
accacccacc ctggtttctt ctt 23

<210> 200
<211> 21
<212> ADN
<213> Artificial

<220>
<223> sonda 4 para detectar el polimorfismo R64Q en el gen NAT2

<400> 200
ccacccaccc tggtttcttc t 21

<210> 201
<211> 25
<212> ADN
<213> Artificial

<220>
<223> sonda 1 para detectar el polimorfismo 282 C>T (Y94Y) en el gen NAT2

<400> 201
agggtatttt tacatccctc cagtt 25

<210> 202
<211> 23
<212> ADN
<213> Artificial

<220>
<223> sonda 2 para detectar el polimorfismo 282 C>T (Y94Y) en el gen NAT2

<400> 202
gggtattttt acatccctcc agt 23

<210> 203
<211> 25
<212> ADN
<213> Artificial

<220>
<223> sonda 3 para detectar el polimorfismo 282 C>T (Y94Y) en el gen NAT2

<400> 203
agggtatttt tatatccctc cagtt 25

<210> 204
<211> 23
<212> ADN
<213> Artificial

<220>
<223> sonda 4 para detectar el polimorfismo 282 C>T (Y94Y) en el gen NAT2

<400> 204
gggtattttt atatccctcc agt 23

<210> 205
<211> 23
<212> ADN
<213> Artificial

<220>
<223> sonda 1 para detectar el polimorfismo I114T en el gen NAT2

<400> 205
gcaggtgacc attgacggca gga 23

<210> 206
<211> 21
<212> ADN
<213> Artificial

<220>
<223> sonda 2 para detectar el polimorfismo I114T en el gen NAT2

<400> 206
caggtgacca ttgacggcag g 21

<210> 207
<211> 23
<212> ADN
<213> Artificial

<220>
<223> sonda 3 para detectar el polimorfismo I114T en el gen NAT2

<400> 207
gcaggtgacc actgacggca gga 23

<210> 208
<211> 21
<212> ADN
<213> Artificial

<220>
<223> sonda 4 para detectar el polimorfismo I114T en el gen NAT2

<400> 208
caggtgacca ctgacggcag g 21

<210> 209
<211> 25
<212> ADN
<213> Artificial

<220>
<223> sonda 1 para detectar el polimorfismo 481C>T (L161L) en el gen NAT2

<400> 209
ggaatctggt acctggacca aatca 25

<210> 210
<211> 27
<212> ADN
<213> Artificial

<220>
<223> sonda 2 para detectar el polimorfismo 481C>T (L161L) en el gen NAT2

<400> 210
aggaatctgg tacctggacc aaatcag 27

<210> 211
<211> 25
<212> ADN
<213> Artificial

<220>
<223> sonda 3 para detectar el polimorfismo 481C>T (L161L) en el gen NAT2

<400> 211
ggaatctggt acttggacca aatca 25

<210> 212
<211> 27
<212> ADN
<213> Artificial

<220>
<223> sonda 4 para detectar el polimorfismo 481C>T (L161L) en el gen NAT2

<400> 212
aggaatctgg tacttggacc aaatcag 27

<210> 213

<211> 25
<212> ADN
<213> Artificial

<220>
<223> sonda 1 para detectar el polimorfismo R197Q en el gen NAT2

<400> 213
cgcttgaacc tcgaacaatt gaaga 25

<210> 214
<211> 23
<212> ADN
<213> Artificial

<220>
<223> sonda 2 para detectar el polimorfismo R197Q en el gen NAT2

<400> 214
gcttgaacct cgaacaattg aag 23

<210> 215
<211> 25
<212> ADN
<213> Artificial

<220>
<223> sonda 3 para detectar el polimorfismo R197Q en el gen NAT2

<400> 215
cgcttgaacc tcaaacaatt gaaga 25

<210> 216
<211> 23
<212> ADN
<213> Artificial

<220>
<223> sonda 4 para detectar el polimorfismo R197Q en el gen NAT2

<400> 216
gcttgaacct caaacaattg aag 23

<210> 217
<211> 25
<212> ADN
<213> Artificial

<220>
<223> sonda 1 para detectar el polimorfismo K268R en el gen NAT2

<400> 217
aagaagtgct gaaaaatata tttaa 25

<210> 218
<211> 25

<212> ADN
<213> Artificial

<220>
<223> sonda 2 para detectar el polimorfismo K268R en el gen NAT2

<400> 218
ttaaatatat ttttcagcac ttctt 25

<210> 219
<211> 25
<212> ADN
<213> Artificial

<220>
<223> sonda 3 para detectar el polimorfismo K268R en el gen NAT2

<400> 219
aagaagtgct gagaaatata tttaa 25

<210> 220
<211> 25
<212> ADN
<213> Artificial

<220>
<223> sonda 4 para detectar el polimorfismo K268R en el gen NAT2

<400> 220
ttaaatatat ttctcagcac ttctt 25

<210> 221
<211> 25
<212> ADN
<213> Artificial

<220>
<223> sonda 1 para detectar el polimorfismo G286E en el gen NAT2

<400> 221
aacctggtga tggatccctt actat 25

<210> 222
<211> 23
<212> ADN
<213> Artificial

<220>
<223> sonda 2 para detectar el polimorfismo G286E en el gen NAT2

<400> 222
acctggtgat ggatccctta cta 23

<210> 223
<211> 25
<212> ADN

<213> Artificial

<220>
<223> sonda 3 para detectar el polimorfismo G286E en el gen NAT2

<400> 223
aacctggtga tgaatcccctt actat 25

<210> 224
<211> 23
<212> ADN
<213> Artificial

<220>
<223> sonda 4 para detectar el polimorfismo G286E en el gen NAT2

<400> 224
acctggtgat gaatccctta cta 23

<210> 225
<211> 23
<212> ADN
<213> Artificial

<220>
<223> sonda 1 para detectar el polimorfismo -786 T>C en el gen NOS3

<400> 225
tcttccctgg ctggctgacc ctg 23

<210> 226
<211> 23
<212> ADN
<213> Artificial

<220>
<223> sonda 2 para detectar el polimorfismo -786 T>C en el gen NOS3

<400> 226
cagggtcagc cagccaggga aga 23

<210> 227
<211> 23
<212> ADN
<213> Artificial

<220>
<223> sonda 3 para detectar el polimorfismo -786 T>C en el gen NOS3

<400> 227
tcttccctgg ccggctgacc ctg 23

<210> 228
<211> 23
<212> ADN
<213> Artificial

<220>
<223> sonda 4 para detectar el polimorfismo -786 T>C en el gen NOS3

<400> 228
caggtcagc cggccaggga aga 23

<210> 229
<211> 23
<212> ADN
<213> Artificial

<220>
<223> sonda 1 para detectar el polimorfismo Glu298Asp en el gen NOS3

<400> 229
gccccagatg agcccccaga act 23

<210> 230
<211> 23
<212> ADN
<213> Artificial

<220>
<223> sonda 2 para detectar el polimorfismo Glu298Asp en el gen NOS3

<400> 230
agttctgggg gctcatctgg ggc 23

<210> 231
<211> 23
<212> ADN
<213> Artificial

<220>
<223> sonda 3 para detectar el polimorfismo Glu298Asp en el gen NOS3

<400> 231
gccccagatg atcccccaga act 23

<210> 232
<211> 23
<212> ADN
<213> Artificial

<220>
<223> sonda 4 para detectar el polimorfismo Glu298Asp en el gen NOS3

<400> 232
agttctgggg gatcatctgg ggc 23

<210> 233
<211> 23
<212> ADN
<213> Artificial

<220>
<223> sonda 1 para detectar el polimorfismo Leu7Pro en el gen NPY

<400> 233
cggacagccc cagtcgcttg tta 23

<210> 234
<211> 23
<212> ADN
<213> Artificial

<220>
<223> sonda 2 para detectar el polimorfismo Leu7Pro en el gen NPY

<400> 234
taacaagcga ctggggctgt ccg 23

<210> 235
<211> 23
<212> ADN
<213> Artificial

<220>
<223> sonda 3 para detectar el polimorfismo Leu7Pro en el gen NPY

<400> 235
cggacagccc cggtcgcttg tta 23

<210> 236
<211> 23
<212> ADN
<213> Artificial

<220>
<223> sonda 4 para detectar el polimorfismo Leu7Pro en el gen NPY

<400> 236
taacaagcga ccggggctgt ccg 23

<210> 237
<211> 23
<212> ADN
<213> Artificial

<220>
<223> sonda 1 para detectar el polimorfismo Cys326Ser en el gen OGG1

<400> 237
cctgcgccaa tcccgccatg ctc 23

<210> 238
<211> 21
<212> ADN
<213> Artificial

<220>

<223> sonda 2 para detectar el polimorfismo Cys326Ser en el gen OGG1

<400> 238
ctgcgccaat cccgccatgc t 21

<210> 239
<211> 23
<212> ADN
<213> Artificial

<220>
<223> sonda 3 para detectar el polimorfismo Cys326Ser en el gen OGG1

<400> 239
cctgcgccaa tgccgccatg ctc 23

<210> 240
<211> 21
<212> ADN
<213> Artificial

<220>
<223> sonda 4 para detectar el polimorfismo Cys326Ser en el gen OGG1

<400> 240
ctgcgccaat gccgccatgc t 21

<210> 241
<211> 21
<212> ADN
<213> Artificial

<220>
<223> sonda 1 para detectar el polimorfismo 4G>5G en el gen PAI1

<400> 241
ctgactcccc cacgtgt 17

<210> 242
<211> 17
<212> ADN
<213> Artificial

<220>
<223> sonda 2 para detectar el polimorfismo 4G>5G en el gen PAI1

<400> 242
ctgactcccc acgtgtc 17

<210> 243
<211> 21
<212> ADN
<213> Artificial

<220>
<223> sonda 3 para detectar el polimorfismo 4G>5G en el gen PAI1

<400> 243
ctgactcccc cacgtgt 17

<210> 244
<211> 17
<212> ADN
<213> Artificial

<220>
<223> sonda 4 para detectar el polimorfismo 4G>5G en el gen PAI1

<400> 244
ctgactcccc acgtgtc 17

<210> 245
<211> 23
<212> ADN
<213> Artificial

<220>
<223> sonda 1 para detectar el polimorfismo 331 G>A en el gen PGR

<400> 245
cgggagataa aagagccgcg tgt 23

<210> 246
<211> 23
<212> ADN
<213> Artificial

<220>
<223> sonda 2 para detectar el polimorfismo 331 G>A en el gen PGR

<400> 246
acacgcggct cttttatctc ccg 23

<210> 247
<211> 23
<212> ADN
<213> Artificial

<220>
<223> sonda 3 para detectar el polimorfismo 331 G>A en el gen PGR

<400> 247
cgggagataa aggagccgcg tgt 23

<210> 248
<211> 23
<212> ADN
<213> Artificial

<220>
<223> sonda 4 para detectar el polimorfismo 331 G>A en el gen PGR

<400> 248
acacgcggct cctttatctc ccg 23

<210> 249
<211> 23
<212> ADN
<213> Artificial

<220>
<223> sonda 1 para detectar el polimorfismo Gln192Arg en el gen PON1

<400> 249
cccctactta caatcctggg aga 23

<210> 250
<211> 23
<212> ADN
<213> Artificial

<220>
<223> sonda 2 para detectar el polimorfismo Gln192Arg en el gen PON1

<400> 250
tctcccagga ttgtaagtag ggg 23

<210> 251
<211> 23
<212> ADN
<213> Artificial

<220>
<223> sonda 3 para detectar el polimorfismo Gln192Arg en el gen PON1

<400> 251
cccctactta cgatcctggg aga 23

<210> 252
<211> 23
<212> ADN
<213> Artificial

<220>
<223> sonda 4 para detectar el polimorfismo Gln192Arg en el gen PON1

<400> 252
tctcccagga tcgtaagtag ggg 23

<210> 253
<211> 23
<212> ADN
<213> Artificial

<220>
<223> sonda 1 para detectar el polimorfismo Ala16Val en el gen SOD2

<400> 253

gataccccaa agccggagcc agc 23

<210> 254
<211> 21
<212> ADN
<213> Artificial

<220>
<223> sonda 2 para detectar el polimorfismo Ala16Val en el gen SOD2

<400> 254
ataccccaaa gccggagcca g 21

<210> 255
<211> 23
<212> ADN
<213> Artificial

<220>
<223> sonda 3 para detectar el polimorfismo Ala16Val en el gen SOD2

<400> 255
gataccccaa aaccggagcc agc 23

<210> 256
<211> 21
<212> ADN
<213> Artificial

<220>
<223> sonda 4 para detectar el polimorfismo Ala16Val en el gen SOD2

<400> 256
ataccccaaa accggagcca g 21

<210> 257
<211> 21
<212> ADN
<213> Artificial

<220>
<223> sonda 1 para detectar el polimorfismo Ala49Thr en el gen SRD5A2

<400> 257
cccgcctgcc agcccgcgcc gcc 23

<210> 258
<211> 19
<212> ADN
<213> Artificial

<220>
<223> sonda 2 para detectar el polimorfismo Ala49Thr en el gen SRD5A2

<400> 258
ccgcctgcca gcccgcgccg c 21

<210> 259
<211> 21
<212> ADN
<213> Artificial

<220>
<223> sonda 3 para detectar el polimorfismo Ala49Thr en el gen SRD5A2

<400> 259
cccgcctgcc aacccgcgcc gcc 23

<210> 260
<211> 19
<212> ADN
<213> Artificial

<220>
<223> sonda 4 para detectar el polimorfismo Ala49Thr en el gen SRD5A2

<400> 260
ccgcctgcca acccgcgccg c 21

<210> 261
<211> 21
<212> ADN
<213> Artificial

<220>
<223> sonda 1 para detectar el polimorfismo Val89Leu en el gen SRD5A2

<400> 261
cctcttctgc gtacattact t 21

<210> 262
<211> 19
<212> ADN
<213> Artificial

<220>
<223> sonda 2 para detectar el polimorfismo Val89Leu en el gen SRD5A2

<400> 262
ctcttctgcg tacattact 19

<210> 263
<211> 21
<212> ADN
<213> Artificial

<220>
<223> sonda para 3 detectar el polimorfismo Val89Leu en el gen SRD5A2

<400> 263
cctcttctgc ctacattact t 21

<210> 264
<211> 19
<212> ADN
<213> Artificial

<220>
<223> sonda 4 para detectar el polimorfismo Val89Leu en el gen SRD5A2

<400> 264
ctcttctgcc tacattact 19

<210> 265
<211> 21
<212> ADN
<213> Artificial

<220>
<223> sonda 1 para detectar el polimorfismo Gly595Ala en el gen SREBF2

<400> 265
gctgctgccg gcaacctaca a 21

<210> 266
<211> 21
<212> ADN
<213> Artificial

<220>
<223> sonda 2 para detectar el polimorfismo Gly595Ala en el gen SREBF2

<400> 266
ttgtaggttg ccggcagcag c 21

<210> 267
<211> 21
<212> ADN
<213> Artificial

<220>
<223> sonda 3 para detectar el polimorfismo Gly595Ala en el gen SREBF2

<400> 267
gctgctgccg ccaacctaca a 21

<210> 268
<211> 21
<212> ADN
<213> Artificial

<220>
<223> sonda 4 para detectar el polimorfismo Gly595Ala en el gen SREBF2

<400> 268
ttgtaggttg gcggcagcag c 21

<210> 269
<211> 21
<212> ADN
<213> Artificial

<220>
<223> sonda 1 para detectar el polimorfismo Arg213His en el gen SULT1A1

<400> 269
tttgtggggc gctccctgcc a 21

<210> 270
<211> 23
<212> ADN
<213> Artificial

<220>
<223> sonda 2 para detectar el polimorfismo Arg213His en el gen SULT1A1

<400> 270
ttgtggggcg ctccctgcc 19

<210> 271
<211> 21
<212> ADN
<213> Artificial

<220>
<223> sonda 3 para detectar el polimorfismo Arg213His en el gen SULT1A1

<400> 271
tttgtggggc actccctgcc a 21

<210> 272
<211> 23
<212> ADN
<213> Artificial

<220>
<223> sonda 4 para detectar el polimorfismo Arg213His en el gen SULT1A1

<400> 272
ttgtggggca ctccctgcc 19

<210> 273
<211> 23
<212> ADN
<213> Artificial

<220>
<223> sonda 1 para detectar el polimorfismo b>B en el gen VDR

<400> 273
gacaggcctg cgcattccca ata 23

<210> 274

<211> 23
<212> ADN
<213> Artificial

<220>
<223> sonda 2 para detectar el polimorfismo b>B en el gen VDR

<400> 274
tattgggaat gcgcaggcct gtc 23

<210> 275
<211> 23
<212> ADN
<213> Artificial

<220>
<223> sonda 3 para detectar el polimorfismo b>B en el gen VDR

<400> 275
gacaggcctg cacattccca ata 23

<210> 276
<211> 23
<212> ADN
<213> Artificial

<220>
<223> sonda 4 para detectar el polimorfismo b>B en el gen VDR

<400> 276
tattgggaat gtgcaggcct gtc 23

<210> 277
<211> 20
<212> ADN
<213> Artificial

<220>
<223> oligonucleótido 1 para amplificar el fragmento en el que puede existir el polimorfismo Intron 16 ins/del en el gen ACE

<400> 277
gggactctgt aagccactgc 20

<210> 278
<211> 20
<212> ADN
<213> Artificial

<220>
<223> oligonucleótido 2 para amplificar el fragmento en el que puede existir el polimorfismo Intron 16 ins/del en el gen ACE

<400> 278
ccatgcccat aacaggtctt 20

<210> 279
<211> 20
<212> ADN
<213> Artificial

<220>
<223> oligonucleótido 1 para amplificar el fragmento en el que puede existir el polimorfismo Gly389Arg en el gen ADRB1

<400> 279
cgccttcaac cccatcatct a 21

<210> 280
<211> 18
<212> ADN
<213> Artificial

<220>
<223> oligonucleótido 2 para amplificar el fragmento en el que puede existir el polimorfismo Gly389Arg en el gen ADRB1

<400> 280
ccggtctccg tgggtcgcgt 20

<210> 281
<211> 18
<212> ADN
<213> Artificial

<220>
<223> oligonucleótido 1 para amplificar el fragmento en el que puede existir el polimorfismo Gln27Glu en el gen ADRB2

<400> 281
gctcacctgc cagactgc 18

<210> 282
<211> 20
<212> ADN
<213> Artificial

<220>
<223> oligonucleótido 2 para amplificar el fragmento en el que puede existir el polimorfismo Gln27Glu en el gen ADRB2

<400> 282
gccaggacga tgagagacat 20

<210> 283
<211> 18
<212> ADN
<213> Artificial

<220>
<223> oligonucleótido 1 para amplificar el fragmento en el que puede existir el polimorfismo Gly16Arg en el gen ADRB2

<400> 283
gctcacctgc cagactgc 18

<210> 284
<211> 20
<212> ADN
<213> Artificial

<220>
<223> oligonucleótido 2 para amplificar el fragmento en el que puede existir el polimorfismo Gly16Arg en el gen ADRB2

<400> 284
gccaggacga tgagagacat 20

<210> 285
<211> 19
<212> ADN
<213> Artificial

<220>
<223> oligonucleótido 1 para amplificar el fragmento en el que puede existir el polimorfismo Trp64Arg en el gen ADRB3

<400> 285
caataccgcc aacaccagt 19

<210> 286
<211> 19
<212> ADN
<213> Artificial

<220>
<223> oligonucleótido 2 para amplificar el fragmento en el que puede existir el polimorfismo Trp64Arg en el gen ADRB3

<400> 286
cgaagtcacg aacacgttg 19

<210> 287
<211> 20
<212> ADN
<213> Artificial

<220>
<223> oligonucleótido 1 para amplificar el fragmento en el que puede existir el polimorfismo Met235Thr en el gen AGT

<400> 287
gaactggatg ttgctgctga 20

<210> 288
<211> 20
<212> ADN
<213> Artificial

<220>
<223> oligonucleótido 2 para amplificar el fragmento en el que puede existir el polimorfismo Met235Thr en el gen AGT

<400> 288
ttgccttacc ttggaagtgg 20

<210> 289
<211> 20
<212> ADN
<213> Artificial

<220>
<223> oligonucleótido 1 para amplificar el fragmento en el que puede existir el polimorfismo 1166 A>C en el gen AGTR1

<400> 289
ccgcccctca gataatgtaa 20

<210> 290
<211> 20
<212> ADN
<213> Artificial

<220>
<223> oligonucleótido 2 para amplificar el fragmento en el que puede existir el polimorfismo 1166 A>C en el gen AGTR1

<400> 290
gcaaaatgtg gctttgcttt 20

<210> 291
<211> 20
<212> ADN
<213> Artificial

<220>
<223> oligonucleótido 1 para amplificar el fragmento en el que puede existir el polimorfismo -75 G>A en el gen APOA1

<400> 291
cacctccttc tcgcagtctc 20

<210> 292
<211> 20
<212> ADN
<213> Artificial

<220>
<223> oligonucleótido 2 para amplificar el fragmento en el que puede existir el polimorfismo -75 G>A en el gen APOA1

<400> 292
gggacagagc tgatccttga 20

<210> 293

<211> 29
<212> ADN
<213> Artificial

<220>
<223> oligonucleótido 1 para amplificar el fragmento en el que puede existir el polimorfismo Arg3480Trp en el gen APOB

<400> 293
agcctcacct cttacttttc cattgagtc 29

<210> 294
<211> 24
<212> ADN
<213> Artificial

<220>
<223> oligonucleótido 2 para amplificar el fragmento en el que puede existir el polimorfismo Arg3480Trp en el gen APOB

<400> 294
cgttggtgaa aaagaggccc tcta 24

<210> 295
<211> 29
<212> ADN
<213> Artificial

<220>
<223> oligonucleótido 1 para amplificar el fragmento en el que puede existir el polimorfismo Arg3500Gln en el gen APOB

<400> 295
agcctcacct cttacttttc cattgagtc 29

<210> 296
<211> 24
<212> ADN
<213> Artificial

<220>
<223> oligonucleótido 2 para amplificar el fragmento en el que puede existir el polimorfismo Arg3500Gln en el gen APOB

<400> 296
cgttggtgaa aaagaggccc tcta 24

<210> 297
<211> 29
<212> ADN
<213> Artificial

<220>
<223> oligonucleótido 1 para amplificar el fragmento en el que puede existir el polimorfismo Arg3531Cys en el gen APOB

<400> 297

agcctcacct cttacttttc cattgagtc 29

<210> 298
<211> 24
<212> ADN
<213> Artificial

<220>
<223> oligonucleótido 2 para amplificar el fragmento en el que puede existir el polimorfismo Arg3531Cys en el gen APOB

<400> 298
cgttggtgaa aaagaggccc tcta 24

<210> 299
<211> 18
<212> ADN
<213> Artificial

<220>
<223> oligonucleótido 1 para amplificar el fragmento en el que puede existir el polimorfismo Cys112Arg en el gen APOE

<400> 299
ctgtccaagg agctgcag 18

<210> 300
<211> 18
<212> ADN
<213> Artificial

<220>
<223> oligonucleótido 2 para amplificar el fragmento en el que puede existir el polimorfismo Cys112Arg en el gen APOE

<400> 300
ctgttccacc aggggccc 18

<210> 301
<211> 18
<212> ADN
<213> Artificial

<220>
<223> oligonucleótido 1 para amplificar el fragmento en el que puede existir el polimorfismo Arg158Cys en el gen APOE

<400> 301
ctgtccaagg agctgcag 18

<210> 302
<211> 18
<212> ADN
<213> Artificial

<220>

<223> oligonucleótido 2 para amplificar el fragmento en el que puede existir el polimorfismo Arg158Cys en el gen APOE

<400> 302
ctgttccacc aggggccc 18

<210> 303
<211> 18
<212> ADN
<213> Artificial

<220>
<223> oligonucleótido 1 para amplificar el fragmento en el que puede existir el polimorfismo 833 T>C en el gen CBS

<400> 303
gcttttgctg gccttgag 18

<210> 304
<211> 20
<212> ADN
<213> Artificial

<220>
<223> oligonucleótido 2 para amplificar el fragmento en el que puede existir el polimorfismo 833 T>C en el gen CBS

<400> 304
gggtgagtta caggctgcac 20

<210> 305
<211> 18
<212> ADN
<213> Artificial

<220>
<223> oligonucleótido 1 para amplificar el fragmento en el que puede existir el polimorfismo 844ins68 en el gen CBS

<400> 305
gcttttgctg gccttgag 18

<210> 306
<211> 20
<212> ADN
<213> Artificial

<220>
<223> oligonucleótido 2 para amplificar el fragmento en el que puede existir el polimorfismo 844ins68 en el gen CBS

<400> 306
gggtgagtta caggctgcac 20

<210> 307
<211> 20

<212> ADN
<213> Artificial

<220>
<223> oligonucleótido 1 para amplificar el fragmento en el que puede existir el polimorfismo TaqIB en el gen CETP

<400> 307
gcaaacagcc aggtataggg 20

<210> 308
<211> 20
<212> ADN
<213> Artificial

<220>
<223> oligonucleótido 2 para amplificar el fragmento en el que puede existir el polimorfismo TaqIB en el gen CETP

<400> 308
aagagactga ggcccagaga 20

<210> 309
<211> 20
<212> ADN
<213> Artificial

<220>
<223> oligonucleótido 1 para amplificar el fragmento en el que puede existir el polimorfismo Arg451Gln en el gen CETP

<400> 309
agccctcatg aacagcaaag 20

<210> 310
<211> 20
<212> ADN
<213> Artificial

<220>
<223> oligonucleótido 2 para amplificar el fragmento en el que puede existir el polimorfismo Arg451Gln en el gen CETP

<400> 310
aatcctgtct gggcctctct 20

<210> 311
<211> 20
<212> ADN
<213> Artificial

<220>
<223> oligonucleótido 1 para amplificar el fragmento en el que puede existir el polimorfismo 1546 G>T en el gen COL1A1

<400> 311
agccgctccc attctcttag 20

<210> 312
<211> 20
<212> ADN
<213> Artificial

<220>
<223> oligonucleótido 2 para amplificar el fragmento en el que puede existir el polimorfismo 1546 G>T en el gen COL1A1

<400> 312
gcgtggtaga gacaggagga 20

<210> 313
<211> 20
<212> ADN
<213> Artificial

<220>
<223> oligonucleótido 1 para amplificar el fragmento en el que puede existir el polimorfismo Val158Met (Alelo*2) en el gen COMT

<400> 313
gggcctactg tggctactca 20

<210> 314
<211> 20
<212> ADN
<213> Artificial

<220>
<223> oligonucleótido 2 para amplificar el fragmento en el que puede existir el polimorfismo Val158Met (Alelo*2) en el gen COMT

<400> 314
cccttttcc aggtctgaca 20

<210> 315
<211> 20
<212> ADN
<213> Artificial

<220>
<223> oligonucleótido 1 para amplificar el fragmento en el que puede existir el polimorfismo -34 A>G en el gen CYP17A1

<400> 315
gggctccagg agaatctttc 20

<210> 316
<211> 20
<212> ADN
<213> Artificial

<220>
<223> oligonucleótido 2 para amplificar el fragmento en el que puede

existir el polimorfismo -34 A>G en el gen CYP17A1

<400> 316
agggtaagca gcaagagagc 20

<210> 317
<211> 20
<212> ADN
<213> Artificial

<220>
<223> oligonucleótido 1 para amplificar el fragmento en el que puede existir el polimorfismo 1558 C>T en el gen CYP19A1

<400> 317
ccttgcaccc agatgagact 20

<210> 318
<211> 20
<212> ADN
<213> Artificial

<220>
<223> oligonucleótido 2 para amplificar el fragmento en el que puede existir el polimorfismo 1558 C>T en el gen CYP19A1

<400> 318
ggcaaggatg gatgatttgt 20

<210> 319
<211> 20
<212> ADN
<213> Artificial

<220>
<223> oligonucleótido 1 para amplificar el fragmento en el que puede existir el polimorfismo Ile462Val en el gen CYP1A1

<400> 319
tgatggtgct atcgacaagg 20

<210> 320
<211> 20
<212> ADN
<213> Artificial

<220>
<223> oligonucleótido 2 para amplificar el fragmento en el que puede existir el polimorfismo Ile462Val en el gen CYP1A1

<400> 320
tttggaagtg ctcacagcag 20

<210> 321
<211> 20
<212> ADN

<213> Artificial

<220>
<223> oligonucleótido 1 para amplificar el fragmento en el que puede existir el polimorfismo T3801C en el gen CYP1A1

<400> 321
ccgctgcact taagcagtct 20

<210> 322
<211> 19
<212> ADN
<213> Artificial

<220>
<223> oligonucleótido 2 para amplificar el fragmento en el que puede existir el polimorfismo T3801C en el gen CYP1A1

<400> 322
ggccccaact actcagagg 19

<210> 323
<211> 20
<212> ADN
<213> Artificial

<220>
<223> oligonucleótido 1 para amplificar el fragmento en el que puede existir el polimorfismo Leu432Val en el gen CYP1B1

<400> 323
acctctgtct tgggctacca 20

<210> 324
<211> 20
<212> ADN
<213> Artificial

<220>
<223> oligonucleótido 2 para amplificar el fragmento en el que puede existir el polimorfismo Leu432Val en el gen CYP1B1

<400> 324
gccaggatgg agatgaagag 20

<210> 325
<211> 20
<212> ADN
<213> Artificial

<220>
<223> oligonucleótido 1 para amplificar el fragmento en el que puede existir el polimorfismo Alelo*4 (Asn453Ser) en el gen CYP1B1

<400> 325
acctctgtct tgggctacca 20

<210> 326
<211> 20
<212> ADN
<213> Artificial

<220>
<223> oligonucleótido 2 para amplificar el fragmento en el que puede existir el polimorfismo Alelo*4 (Asn453Ser) en el gen CYP1B1

<400> 326
gccaggatgg agatgaagag 20

<210> 327
<211> 20
<212> ADN
<213> Artificial

<220>
<223> oligonucleótido 1 para amplificar el fragmento en el que puede existir el polimorfismo Arg144Cys (alelo*2) en el gen CYP2C9

<400> 327
cctgggatct ccctcctagt 20

<210> 328
<211> 20
<212> ADN
<213> Artificial

<220>
<223> oligonucleótido 2 para amplificar el fragmento en el que puede existir el polimorfismo Arg144Cys (alelo*2) en el gen CYP2C9

<400> 328
ccacccttgg tttttctcaa 20

<210> 329
<211> 20
<212> ADN
<213> Artificial

<220>
<223> oligonucleótido 1 para amplificar el fragmento en el que puede existir el polimorfismo Ile359Leu (alelo*3)en el gen CYP2C9

<400> 329
ccacatgccc tacacagatg 20

<210> 330
<211> 20
<212> ADN
<213> Artificial

<220>
<223> oligonucleótido 2 para amplificar el fragmento en el que puede existir el polimorfismo Ile359Leu (alelo*3)en el gen CYP2C9

<400> 330
tcgaaaacat ggagttgcag 20

<210> 331
<211> 21
<212> ADN
<213> Artificial

<220>
<223> oligonucleótido 1 para amplificar el fragmento en el que puede existir el polimorfismo 681 G>A (Pro227Pro) (alelo*2) en el gen CYP2C19

<400> 331
caaccagagc ttggcatatt g 21

<210> 332
<211> 20
<212> ADN
<213> Artificial

<220>
<223> oligonucleótido 2 para amplificar el fragmento en el que puede existir el polimorfismo 681 G>A (Pro227Pro) (alelo*2) en el gen CYP2C19

<400> 332
taaagtcccg agggttgttg 20

<210> 333
<211> 24
<212> ADN
<213> Artificial

<220>
<223> oligonucleótido 1 para amplificar el fragmento en el que puede existir el polimorfismo 2549 A>del (alelo*3) en el gen CYP2D6

<400> 333
gggcctgaga cttgtccagg 20

<210> 334
<211> 23
<212> ADN
<213> Artificial

<220>
<223> oligonucleótido 2 para amplificar el fragmento en el que puede existir el polimorfismo 2549 A>del (alelo*3) en el gen CYP2D6

<400> 334
gccgagagc atactcggga c 21

<210> 335
<211> 19

<212> ADN
<213> Artificial

<220>
<223> oligonucleótido 1 para amplificar el fragmento en el que puede existir el polimorfismo 1846 G>A / 1847 G>A (alelo*4) en el gen CYP2D6

<400> 335
ccacgcgcac gtgcccgtcc ca 22

<210> 336
<211> 20
<212> ADN
<213> Artificial

<220>
<223> oligonucleótido 2 para amplificar el fragmento en el que puede existir el polimorfismo 1846 G>A / 1847 G>A (alelo*4) en el gen CYP2D6

<400> 336
cctgcagaga ctcctcggtc tctc 24

<210> 337
<211> 19
<212> ADN
<213> Artificial

<220>
<223> oligonucleótido 1 para amplificar el fragmento en el que puede existir el polimorfismo 1707 del>T (alelo*6) en el gen CYP2D6

<400> 337
ccacgcgcac gtgcccgtcc ca 22

<210> 338
<211> 20
<212> ADN
<213> Artificial

<220>
<223> oligonucleótido 2 para amplificar el fragmento en el que puede existir el polimorfismo 1707 del>T (alelo*6) en el gen CYP2D6

<400> 338
cctgcagaga ctcctcggtc tctc 24

<210> 339
<211> 20
<212> ADN
<213> Artificial

<220>
<223> oligonucleótido 1 para amplificar el fragmento en el que puede existir el polimorfismo Ala541Thr en el gen ELAC2

<400> 339
ccgacacgtc tctgctactg 20

<210> 340
<211> 20
<212> ADN
<213> Artificial

<220>
<223> oligonucleótido 2 para amplificar el fragmento en el que puede existir el polimorfismo Ala541Thr en el gen ELAC2

<400> 340
aacaaaagct ctgggcaagt 20

<210> 341
<211> 20
<212> ADN
<213> Artificial

<220>
<223> oligonucleótido 1 para amplificar el fragmento en el que puede existir el polimorfismo -397 T>C (PvuII) en el gen ESR1 IVS1

<400> 341
agggttatgt ggcaatgacg 20

<210> 342
<211> 20
<212> ADN
<213> Artificial

<220>
<223> oligonucleótido 2 para amplificar el fragmento en el que puede existir el polimorfismo -397 T>C (PvuII) en el gen ESR1 IVS1

<400> 342
accaatgctc atcccaactc 20

<210> 343
<211> 23
<212> ADN
<213> Artificial

<220>
<223> oligonucleótido 1 para amplificar el fragmento en el que puede existir el polimorfismo Val34Leu en el gen F13A1

<400> 343
catgcctttt ctgttgtctt ctt 23

<210> 344
<211> 20
<212> ADN
<213> Artificial

<220>
<223> oligonucleótido 2 para amplificar el fragmento en el que puede existir el polimorfismo Val34Leu en el gen F13A1

<400> 344
cccagtggag acagaggatg 20

<210> 345
<211> 26
<212> ADN
<213> Artificial

<220>
<223> oligonucleótido 1 para amplificar el fragmento en el que puede existir el polimorfismo -455 G>A en el gen FGB

<400> 345
gggtctttct gatgtgtatt tttca 25

<210> 346
<211> 20
<212> ADN
<213> Artificial

<220>
<223> oligonucleótido 2 para amplificar el fragmento en el que puede existir el polimorfismo -455 G>A en el gen FGB

<400> 346
gacctactca caaggcaacc a 21

<210> 347
<211> 25
<212> ADN
<213> Artificial

<220>
<223> oligonucleótido 1 para amplificar el fragmento en el que puede existir el polimorfismo 20210 G>A en el gen FII

<400> 347
gagagtaggg ggccactcat 20

<210> 348
<211> 21
<212> ADN
<213> Artificial

<220>
<223> oligonucleótido 2 para amplificar el fragmento en el que puede existir el polimorfismo 20210 G>A en el gen FII

<400> 348
cctgagccca gagagctg 18

<210> 349

<211> 20
<212> ADN
<213> Artificial

<220>
<223> oligonucleótido 1 para amplificar el fragmento en el que puede existir el polimorfismo Arg506Gln en el gen FV Leiden

<400> 349
gcccagtgct taacaagacc 20

<210> 350
<211> 22
<212> ADN
<213> Artificial

<220>
<223> oligonucleótido 2 para amplificar el fragmento en el que puede existir el polimorfismo Arg506Gln en el gen FV Leiden

<400> 350
cccattattt agccaggaga cc 22

<210> 351
<211> 20
<212> ADN
<213> Artificial

<220>
<223> oligonucleótido 1 para amplificar el fragmento en el que puede existir el polimorfismo Pro319Ser en el gen GJA4

<400> 351
cctcctcaga cccttacacg 20

<210> 352
<211> 20
<212> ADN
<213> Artificial

<220>
<223> oligonucleótido 2 para amplificar el fragmento en el que puede existir el polimorfismo Pro319Ser en el gen GJA4

<400> 352
gcagccagac ttctcaggac 20

<210> 353
<211> 20
<212> ADN
<213> Artificial

<220>
<223> oligonucleótido 1 para amplificar el fragmento en el que puede existir el polimorfismo 393 T>C (Ile131Ile) en el gen GNAS

<400> 353

agtacgtgct ggctccttgt 20

<210> 354
<211> 20
<212> ADN
<213> Artificial

<220>
<223> oligonucleótido 2 para amplificar el fragmento en el que puede existir el polimorfismo 393 T>C (Ile131Ile) en el gen GNAS

<400> 354
cacaagtcgg ggtgtagctt 20

<210> 355
<211> 18
<212> ADN
<213> Artificial

<220>
<223> oligonucleótido 1 para amplificar el fragmento en el que puede existir el polimorfismo 825 C>T (Ser275Ser) en el gen GNB3

<400> 355
ctgccgcttg tttgacct 18

<210> 356
<211> 20
<212> ADN
<213> Artificial

<220>
<223> oligonucleótido 2 para amplificar el fragmento en el que puede existir el polimorfismo 825 C>T (Ser275Ser) en el gen GNB3

<400> 356
cacacgctca gacttcatgg 20

<210> 357
<211> 20
<212> ADN
<213> Artificial

<220>
<223> oligonucleótido 1 para amplificar el fragmento en el que puede existir el polimorfismo GSTM1

<400> 357
tgcttcacgt gttatggagg t 21

<210> 358
<211> 20
<212> ADN
<213> Artificial

<220>

<223> oligonucleótido 2 para amplificar el fragmento en el que puede existir el polimorfismo GSTM1

<400> 358
gggctcaaat atacggtgga 20

<210> 359
<211> 21
<212> ADN
<213> Artificial

<220>
<223> oligonucleótido 1 para amplificar el fragmento en el que puede existir el polimorfismo Ile105Val en el gen GSTP1

<400> 359
ctctatggga aggaccagca 20

<210> 360
<211> 20
<212> ADN
<213> Artificial

<220>
<223> oligonucleótido 2 para amplificar el fragmento en el que puede existir el polimorfismo Ile105Val en el gen GSTP1

<400> 360
gaagcccctt tctttgttca 20

<210> 361
<211> 20
<212> ADN
<213> Artificial

<220>
<223> oligonucleótido 1 para amplificar el fragmento en el que puede existir el polimorfismo Ala114Val en el gen GSTP1

<400> 361
gcaagcagag gagaatctgg 20

<210> 362
<211> 20
<212> ADN
<213> Artificial

<220>
<223> oligonucleótido 2 para amplificar el fragmento en el que puede existir el polimorfismo Ala114Val en el gen GSTP1

<400> 362
ctcacctggt ctcccacaat 20

<210> 363
<211> 20

<212> ADN
<213> Artificial

<220>
<223> oligonucleótido 1 para amplificar el fragmento en el que puede existir el polimorfismo GSTT1

<400> 363
ggcagcataa gcaggacttc 20

<210> 364
<211> 20
<212> ADN
<213> Artificial

<220>
<223> oligonucleótido 2 para amplificar el fragmento en el que puede existir el polimorfismo GSTT1

<400> 364
ctgcagttgc tcgaggacaa 20

<210> 365
<211> 20
<212> ADN
<213> Artificial

<220>
<223> oligonucleótido 1 para amplificar el fragmento en el que puede existir el polimorfismo -174 C>G en el gen IL6

<400> 365
gcctcaatga cgacctaagc 20

<210> 366
<211> 20
<212> ADN
<213> Artificial

<220>
<223> oligonucleótido 2 para amplificar el fragmento en el que puede existir el polimorfismo -174 C>G en el gen IL6

<400> 366
tcatgggaaa atcccacatt 20

<210> 367
<211> 20
<212> ADN
<213> Artificial

<220>
<223> oligonucleótido 1 para amplificar el fragmento en el que puede existir el polimorfismo -1082 G>A en el gen IL10

<400> 367
tccccaggta gagcaacact 20

<210> 368
<211> 20
<212> ADN
<213> Artificial

<220>
<223> oligonucleótido 2 para amplificar el fragmento en el que puede existir el polimorfismo -1082 G>A en el gen IL10

<400> 368
atggaggctg gataggaggt 20

<210> 369
<211> 20
<212> ADN
<213> Artificial

<220>
<223> oligonucleótido 1 para amplificar el fragmento en el que puede existir el polimorfismo Leu33Pro en el gen ITGB3

<400> 369
gctccaatgt acggggtaaa 20

<210> 370
<211> 20
<212> ADN
<213> Artificial

<220>
<223> oligonucleótido 2 para amplificar el fragmento en el que puede existir el polimorfismo Leu33Pro en el gen ITGB3

<400> 370
actcactggg aactcgatgg 20

<210> 371
<211> 20
<212> ADN
<213> Artificial

<220>
<223> oligonucleótido 1 para amplificar el fragmento en el que puede existir el polimorfismo 5A>6A en el gen MMP3

<400> 371
tcactgccac cactctgttc 20

<210> 372
<211> 20
<212> ADN
<213> Artificial

<220>
<223> oligonucleótido 2 para amplificar el fragmento en el que puede

existir el polimorfismo 5A>6A en el gen MMP3

<400> 372
gcctcaacct ctcaaagtgc 20

<210> 373
<211> 20
<212> ADN
<213> Artificial

<220>
<223> oligonucleótido 1 para amplificar el fragmento en el que puede existir el polimorfismo Ala222Val en el gen MTHFR

<400> 373
gcctctcctg actgtcatcc 20

<210> 374
<211> 20
<212> ADN
<213> Artificial

<220>
<223> oligonucleótido 2 para amplificar el fragmento en el que puede existir el polimorfismo Ala222Val en el gen MTHFR

<400> 374
caaagcggaa gaatgtgtca 20

<210> 375
<211> 20
<212> ADN
<213> Artificial

<220>
<223> oligonucleótido 1 para amplificar el fragmento en el que puede existir el polimorfismo R64Q en el gen NAT2

<400> 375
ccatggagtt gggcttagag 20

<210> 376
<211> 20
<212> ADN
<213> Artificial

<220>
<223> oligonucleótido 2 para amplificar el fragmento en el que puede existir el polimorfismo R64Q en el gen NAT2

<400> 376
ggctgatcct tcccagaaat 20

<210> 377
<211> 20
<212> ADN

<213> Artificial

<220>
<223> oligonucleótido 1 para amplificar el fragmento en el que puede existir el polimorfismo 282 C>T (Y94Y) en el gen NAT2

<400> 377
ccatggagtt gggcttagag 20

<210> 378
<211> 20
<212> ADN
<213> Artificial

<220>
<223> oligonucleótido 2 para amplificar el fragmento en el que puede existir el polimorfismo 282 C>T (Y94Y) en el gen NAT2

<400> 378
ccatgccagt gctgtatttg 20

<210> 379
<211> 20
<212> ADN
<213> Artificial

<220>
<223> oligonucleótido 1 para amplificar el fragmento en el que puede existir el polimorfismo I114T en el gen NAT2

<400> 379
ccatggagtt gggcttagag 20

<210> 380
<211> 20
<212> ADN
<213> Artificial

<220>
<223> oligonucleótido 2 para amplificar el fragmento en el que puede existir el polimorfismo I114T en el gen NAT2

<400> 380
ccatgccagt gctgtatttg 20

<210> 381
<211> 19
<212> ADN
<213> Artificial

<220>
<223> oligonucleótido 1 para amplificar el fragmento en el que puede existir el polimorfismo 481C>T (L161L) en el gen NAT2

<400> 381
caggtgcctt gcattttct 19

<210> 382
<211> 20
<212> ADN
<213> Artificial

<220>
<223> oligonucleótido 2 para amplificar el fragmento en el que puede existir el polimorfismo 481C>T (L161L) en el gen NAT2

<400> 382
gatgaagccc accaaacagt 20

<210> 383
<211> 19
<212> ADN
<213> Artificial

<220>
<223> oligonucleótido 1 para amplificar el fragmento en el que puede existir el polimorfismo R197Q en el gen NAT2

<400> 383
caggtgcctt gcattttct 19

<210> 384
<211> 20
<212> ADN
<213> Artificial

<220>
<223> oligonucleótido 2 para amplificar el fragmento en el que puede existir el polimorfismo R197Q en el gen NAT2

<400> 384
gatgaagccc accaaacagt 20

<210> 385
<211> 24
<212> ADN
<213> Artificial

<220>
<223> oligonucleótido 1 para amplificar el fragmento en el que puede existir el polimorfismo K268R en el gen NAT2

<400> 385
aaagacaata cagatctggt cgag 24

<210> 386
<211> 24
<212> ADN
<213> Artificial

<220>
<223> oligonucleótido 2 para amplificar el fragmento en el que puede existir el polimorfismo K268R en el gen NAT2

<400> 386
tcttcaaaat aacgtgaggg taga 24

<210> 387
<211> 24
<212> ADN
<213> Artificial

<220>
<223> oligonucleótido 1 para amplificar el fragmento en el que puede existir el polimorfismo G286E en el gen NAT2

<400> 387
aaagacaata cagatctggt cgag 24

<210> 388
<211> 24
<212> ADN
<213> Artificial

<220>
<223> oligonucleótido 2 para amplificar el fragmento en el que puede existir el polimorfismo G286E en el gen NAT2

<400> 388
tcttcaaaat aacgtgaggg taga 24

<210> 389
<211> 20
<212> ADN
<213> Artificial

<220>
<223> oligonucleótido 1 para amplificar el fragmento en el que puede existir el polimorfismo -786 T>C en el gen NOS3

<400> 389
gtgtacccca cctgcattct 20

<210> 390
<211> 19
<212> ADN
<213> Artificial

<220>
<223> oligonucleótido 2 para amplificar el fragmento en el que puede existir el polimorfismo -786 T>C en el gen NOS3

<400> 390
cccaccctgt cattcagtg 19

<210> 391
<211> 20
<212> ADN
<213> Artificial

<220>
<223> oligonucleótido 1 para amplificar el fragmento en el que puede existir el polimorfismo Glu298Asp en el gen NOS3

<400> 391
gaaggcagga gacagtggat 20

<210> 392
<211> 20
<212> ADN
<213> Artificial

<220>
<223> oligonucleótido 2 para amplificar el fragmento en el que puede existir el polimorfismo Glu298Asp en el gen NOS3

<400> 392
cagtcaatcc ctttggtgct 20

<210> 393
<211> 20
<212> ADN
<213> Artificial

<220>
<223> oligonucleótido 1 para amplificar el fragmento en el que puede existir el polimorfismo Leu7Pro en el gen NPY

<400> 393
ctctgcctgg tgatgaggtt 20

<210> 394
<211> 19
<212> ADN
<213> Artificial

<220>
<223> oligonucleótido 2 para amplificar el fragmento en el que puede existir el polimorfismo Leu7Pro en el gen NPY

<400> 394
gcagaggagg gaggtgct 18

<210> 395
<211> 20
<212> ADN
<213> Artificial

<220>
<223> oligonucleótido 1 para amplificar el fragmento en el que puede existir el polimorfismo Cys326Ser en el gen OGG1

<400> 395
tagtctcacc agccctgacc 20

<210> 396
<211> 20
<212> ADN
<213> Artificial

<220>
<223> oligonucleótido 2 para amplificar el fragmento en el que puede existir el polimorfismo Cys326Ser en el gen OGG1

<400> 396
tggggaattt ctttgtccag 20

<210> 397
<211> 20
<212> ADN
<213> Artificial

<220>
<223> oligonucleótido 1 para amplificar el fragmento en el que puede existir el polimorfismo 4G>5G en el gen PAI1

<400> 397
caacctcagc cagacaaggt 20

<210> 398
<211> 20
<212> ADN
<213> Artificial

<220>
<223> oligonucleótido 2 para amplificar el fragmento en el que puede existir el polimorfismo 4G>5G en el gen PAI1

<400> 398
cagccacgtg attgtctagg 20

<210> 399
<211> 20
<212> ADN
<213> Artificial

<220>
<223> oligonucleótido 1 para amplificar el fragmento en el que puede existir el polimorfismo 331 G>A en el gen PGR

<400> 399
gcttcacagc atgcacgagt 20

<210> 400
<211> 20
<212> ADN
<213> Artificial

<220>
<223> oligonucleótido 2 para amplificar el fragmento en el que puede existir el polimorfismo 3 Gln192Arg en el gen PON1

<400> 400
gaggactgga gacgcagagt 20

<210> 401
<211> 20
<212> ADN
<213> Artificial

<220>
<223> oligonucleótido 1 para amplificar el fragmento en el que puede existir el polimorfismo Gln192Arg en el gen PON1

<400> 401
tattgttgct gtgggacctg 20

<210> 402
<211> 20
<212> ADN
<213> Artificial

<220>
<223> oligonucleótido 2 para amplificar el fragmento en el que puede existir el polimorfismo Gln192Arg en el gen PON1

<400> 402
caaatccttc tgccaccact 20

<210> 403
<211> 20
<212> ADN
<213> Artificial

<220>
<223> oligonucleótido 1 para amplificar el fragmento en el que puede existir el polimorfismo Ala16Val en el gen SOD2

<400> 403
ggctgtgctt tctcgtcttc 20

<210> 404
<211> 19
<212> ADN
<213> Artificial

<220>
<223> oligonucleótido 2 para amplificar el fragmento en el que puede existir el polimorfismo Ala16Val en el gen SOD2

<400> 404
ccgtagtcgt agggcaggt 19

<210> 405
<211> 18
<212> ADN
<213> Artificial

<220>
<223> oligonucleótido 1 para amplificar el fragmento en el que puede existir el polimorfismo Ala49Thr en el gen SRD5A2

<400> 405
agcacacgga gagcctga 18

<210> 406
<211> 20
<212> ADN
<213> Artificial

<220>
<223> oligonucleótido 2 para amplificar el fragmento en el que puede existir el polimorfismo Ala49Thr en el gen SRD5A2

<400> 406
aggggaaaaa cgctacctgt 20

<210> 407
<211> 18
<212> ADN
<213> Artificial

<220>
<223> oligonucleótido 1 para amplificar el fragmento en el que puede existir el polimorfismo Val89Leu en el gen SRD5A2

<400> 407
agcacacgga gagcctga 18

<210> 408
<211> 20
<212> ADN
<213> Artificial

<220>
<223> oligonucleótido 2 para amplificar el fragmento en el que puede existir el polimorfismo Val89Leu en el gen SRD5A2

<400> 408
aggggaaaaa cgctacctgt 20

<210> 409
<211> 20
<212> ADN
<213> Artificial

<220>
<223> oligonucleótido 1 para amplificar el fragmento en el que puede existir el polimorfismo Gly595Ala en el gen SREBF2

<400> 409
ggccagtgac cattaacacc 20

<210> 410

<211> 20
<212> ADN
<213> Artificial

<220>
<223> oligonucleótido 2 para amplificar el fragmento en el que puede existir el polimorfismo Gly595Ala en el gen SREBF2

<400> 410
tcttcaaagc ctgcctcagt 20

<210> 411
<211> 20
<212> ADN
<213> Artificial

<220>
<223> oligonucleótido 1 para amplificar el fragmento en el que puede existir el polimorfismo Arg213His en el gen SULT1A1

<400> 411
gtaatccgag cctccactga 20

<210> 412
<211> 20
<212> ADN
<213> Artificial

<220>
<223> oligonucleótido 2 para amplificar el fragmento en el que puede existir el polimorfismo Arg213His en el gen SULT1A1

<400> 412
gctgtggtcc atgaactcct 20

<210> 413
<211> 20
<212> ADN
<213> Artificial

<220>
<223> oligonucleótido 1 para amplificar el fragmento en el que puede existir el polimorfismo b>B en el gen VDR

<400> 413
cctcactgcc cttagctctg 20

<210> 414
<211> 20
<212> ADN
<213> Artificial

<220>
<223> oligonucleótido 2 para amplificar el fragmento en el que puede existir el polimorfismo b>B en el gen VDR

<400> 414

```
cccgcaagaa acctcaaata                                                   20


<210>  415
<211>  50
<212>  ADN
<213>  Artificial

<220>
<223>  secuencia de nucleótidos del control externo CEH

<400>  415
gtcgtcaaga tgctaccgtt caggagtcgt caagatgcta ccgttcagga                  50


<210>  416
<211>  19
<212>  ADN
<213>  Artificial

<220>
<223>  oligonucleótido 1 para detección del control externo

<400>  416
cttgacgact cctgaacgg                                                    19


<210>  417
<211>  19
<212>  ADN
<213>  Artificial

<220>
<223>  oligonucleotido 2 para detección del control externo

<400>  417
cttgacgaca cctgaacgg                                                    19
```

**Description**

Field of the Invention

The invention is comprised in the technical-industrial sector of the extracorporeal *in vitro* diagnosis of biological samples for the detection of gene variants, for example, polymorphisms or genetic mutations, associated with diseases associated with aging, or associated with the response to pharmacological treatments, with application in anti-aging medicine; the invention particularly relates to an *in vitro* method for determining the global genetic risk a subject has of developing a pathology associated with aging from a combination of particular genetic risks. The invention also relates to reagents and kits for putting said method into practice.

Background of the Invention

As a result of the knowledge obtained from the analysis of the human genome, many examples of alleles defined by single nucleotide polymorphisms or SNPs which can affect the good functioning of a certain system and others which, on the contrary, have a beneficial effect are currently known. It is important to bear in mind that many of these genes interact with one another and, for this reason, some antagonistic effects usually mutually compensate their expression, which can clinically be translated into the suppression of a certain sign or symptom within the clinical symptomatology. Nevertheless, in other cases the effects of some genes are mutually enhanced and as a result of this synergy, there may be both clinical and therapeutic response complications or peculiarities which explain the differences observed in the evolution of several cases with one and the same disease.

These differences are also shown in the predisposition to suffer from various common diseases and to the development of their complications. For example, the genetic susceptibility to dyslipidemias will most likely lead to a shorter life, on the other hand, inheriting gene variants in genes protecting against coronary diseases, against oxidative damage or against cancer will without a doubt aid to prolonging life. In this sense, there is a balance which can be established between genes with negative or deleterious effects (predisposing to diseases) and genes with positive effects (certain protective genotypes) in the maintenance of life reserves. Of course, it must never be forgotten that other non-genetic risk factors with a negative effect (unhealthy lifestyles and habits) in contrast to those with a positive effect (control of said habits, specific pharmacological intervention) which shift the balance in one direction or the other, play an important direct role in this genetic interaction, completing the modulation of the final clinical phenotype and finally determining the greater or lower life expectancy.

Medical treatments also have an effect among these environmental factors capable of modulating the expression of the genes. If they are the suitable ones, they would contribute to increasing survival once any disease has developed.

A genetic analysis can facilitate the very early detection of the particular vulnerability of each individual analyzed and at the same time it offers the possibility of providing a scientific basis to a treatment, which stops being empirical and general to become completely objective, since it will be formulated according to the principles of pharmacogenetics: a state-of-the-art tool which is gradually becoming the latest great revolution of modern medicine: the era of the personalized medicine.

If, furthermore, there is the possibility of analyzing the genetic polymorphisms involved in the etiopathogenesis of the disease, a comprehensive analysis of the problem could be conducted, under a unitary perspective including, on one hand, classic risk factors and on the other hand, the data obtained from the gene variants studied.

Until the mid twentieth century, it has been assumed that the diseases to which elderly people were more vulnerable, such as for example osteoporosis, were inevitable attributes of the aging process. It is true that aging predisposes to increasing the vulnerability to the disease, however, a large amount of research aimed at obtaining information about the biology of aging and longevity is currently being conducted.

Anti-aging medicine can be defined as any intervention delaying the development of pathologies related to aging and other adverse changes related to age and which are officially not listed as such diseases.

A number of molecular markers in the genome which are related to pathologies associated with aging have been described in recent years. Given that the list of genetic risk factors for developing a pathology associated with aging is increasingly numerous and the interest for considering its importance in the determinism of the disease continues to increase, it is currently necessary to have tools which allow quickly conducting the analysis of all these genetic factors as a whole.

The most relevant diseases associated with aging are those which occupy the first places among the main causes of morbimortality among people above 65 years of age, including cardiovascular diseases, cancer and osteoporosis. Aging has also been defined as the process resulting from an imperfect protection of the main cell components against oxidative stress. Furthermore, as people get older, drugs remain more time in the organism due to the decrease of the amount of water, therefore the prescription of suitable doses according to the response of the patient to the drugs becomes more important in order to prevent adverse reactions to such drugs.

Vascular disease

Vascular disease (VD) is one of the main causes of mortality and morbidity, therefore the development of models for predicting the risk of suffering from this type of disease, both for attempting to know the possible mechanisms affecting the increase of the risk and for being able to intervene early on and prevent them, is of great interest.

It is important from the perspective of the global assessment of vascular risk to consider VD as a systemic process pathogenically related to endothelial dysfunction, on which there act various risk factors which will determine interindividual expression variability (dyslipidemia, blood hypercoagulability, hyperhomocysteinemia) but which by no means will lead to it being manifested as an organ disease at different levels: cardiovascular, cerebrovascular, peripheral vascular and/or renal level.

The association between coronary and cerebrovascular disease has been partly explained and its study and knowledge has been slow, since the interest for the analysis of the risk factors in cerebrovascular disease has been scarce, which explains why its study began later. Despite the fact that there was a tendency to consider that familial hypercholesterolemia, a disease prototype which indicated a high coronary risk, was not accompanied by ictus, recently conducted meticulous studies demonstrate that what actually happens is that atheromatous cerebrovascular disease develops more slowly than coronary disease, therefore for example in familial hypercholesterolemia, since the onset of the ischemic cardiopathy itself is earlier, it does not allow the development of the cerebrovascular disease in most cases.

According to the foregoing, a thorough stratification must be performed in the evaluation of vascular risk in order to be as objective as possible when evaluating each case. The following are within the large sections which must be analyzed:

a) Metabolic risk: dyslipidemia, hyperhomocysteinemia;
b) Blood hypercoagulability;
c) Endothelial vulnerability; and
d) Hemodynamic status (renin-angiotensin system)

Dyslipidemia

The predisposition to dyslipidemia or lipid metabolism alteration is also very heterogeneous at molecular level and it is important to evaluate the entire set since among each of the representatives of every genetic polymorphism (presence of allele A or B) which are inherited in an individual, synergies or antagonisms may be established which will determine highly variable and particular risks and therefore vulnerabilities which enable individualizing each case not only in its global assessment, but also in relation to the therapeutic strategy to be used.

Hyperhomocysteinemia

Homocysteine (HCT) is a demethylated amino acid derived from Methionine and, therefore, an intermediate of the methionine cycle. It is metabolized by remethylation to methionine or by sulfuration to cysteine. For the remethylation, the methionine synthase needs vitamin B12 as a cofactor and folic acid as a substrate. For the transsulfuration, a cystathionine beta-synthase (CBS) and vitamin B6 as a cofactor are required. A defect in the remethylation or the transsulfuration leads to a hyperhomocysteinemia. Various studies have demonstrated that hyperhomocysteinemia, even when it is mild to moderate (greater than 12 nmol/mL) is an independent factor for brain ischemia, myocardial infarction, peripheral artery disease and carotid stenosis. Although the causes coming from the external environment (non-genetic) are important among the causes thereof, there are important genetic alterations to be considered because they determine both the prognosis and the degree of therapeutic response of each case.

The renin-angiotensin system and adrenergic receptors are also factors predisposing to high blood pressure and cardiovascular disease in general.

Blood hypercoagulability

According to the classic Virchow's triad, three interrelated factors must be taken into account in the formation of a thrombus: alteration of the blood vessel wall, of the blood flow and of the blood coagulability. It is precisely the alteration of this latter factor which favors the coagulation of the blood, or hypercoagulability or prothrombotic state, which is defined as thrombophilia.

As a general rule, a hypercoagulability state must be suspected in individuals with recurrent episodes of deep vein thromboses, pulmonary embolism, family history of thrombotic events, unusual sites of arterial and venous thrombosis and in children, adolescents or young adults with thrombotic events in general.

Endothelial vulnerability

The most evident function of the vascular endothelium is that of maintaining a dilated vascular tone in the exact proportion to preserve the blood pressure at normal values and allow tissue perfusion. This vasodilating function is exerted by the endothelium by means of the synthesis and secretion of relaxation factors such as nitric oxide (NO). Furthermore, the endothelium is an important element for maintaining the balance with platelets and coagulation factors and thus maintaining the fluidity of the blood in what is referred to as homeostatic balance (hemostasis) since the imbalance in one direction or the other will cause hemorrhage or thrombosis.

Most of the factors capable of attacking and damaging the vascular endothelium come from the external environment and one of the most harmful among them is smoking. Nevertheless, there are several genetic polymorphisms which determine a greater vulnerability to this damage and therefore contribute considerably to the general increase of vascular risk. These polymorphisms even worsen the damage which would already be caused by classic non-genetic risk factors themselves such as smoking.

Oxidative stress

Oxidative stress is another factor which can also affect to a great extent the better or worse response at endothelial level and at vascular level in general, thus, another important pillar to be considered in the molecular etiopathogenesis of general vascular disease is the degree of defensive potential against oxidative stress.

Ischemic cardiopathy and acute myocardial infarction can be the expression of a process starting with an excess of free radicals, which start the atherosclerotic process by damage in vascular wall, causing the penetration into the subendothelial space of low density lipoproteins (LDL) and therefore into the atherosclerotic plaque.

Various scientific publications analyze the mechanisms of the human organism to produce and at the same time limit the production of reactive oxygen species. An excess of free radicals usually starts the damage of the vascular wall and LDL-cholesterol is involved in this process. A decrease in the incidence of cardiovascular diseases with individual antioxidant supplements has been demonstrated.

Carcinogenic risk

This risk relates to the susceptibility with a polygenic and multifactorial basis, not to the monogenic variants of hereditary cancer, therefore adapting each risk to the personal clinical situation and to the family history of each case is recommended.

Risk of adverse reactions to drugs

The elderly are more prone to suffering from chronic diseases and take a larger amount of drugs than the young, they are therefore more prone to adverse reactions to the drug.

As people get older, the amount of water of the organism decreases. Drugs reach higher concentrations in the elderly. Once in the body, many drugs are dissolved in the fluids of the organism but in these people there is less water for diluting them. Furthermore, the kidneys are much less effective in the excretion of drugs through urine and the liver has a lower capacity for metabolizing them.

For this reason, as people get older, the prescription of suitable doses according to the response of the patient to the drugs becomes more important in order to prevent adverse reactions to such drugs.

It is therefore necessary to develop a method which allows the simultaneous, sensitive, specific and reproducible detection of gene variants associated with pathologies associated with aging (vascular risk, carcinogenic risk, risk of osteoporosis, risk against oxidative stress and risk of adverse reactions to drugs) and which is a tool useful in medicine, particularly in anti-aging medicine. Thus, the clinical and practical translation of this analysis requires the corresponding algorithm integrating the real value of all these gene variants, taking into account the synergies and antagonisms occurring between them, presenting a risk in absolute values which is different depending on the individual analyzed.

The real value of this risk must be considered in the global context of each case taking into account all the classic (non-genetic) risk factors. An objective analysis and unitary vision of a complex and multifactorial disease such as for example a disease associated with aging will only be assured in this way.

Detailed Description of the Invention

The authors of the present invention have developed a method for determining the global genetic risk of a subject to develope a pathology associated with aging. Said method is based on the combination of particular genetic risks of developing common pathologies associated with aging. Said particular genetic risks are determined from the results obtained from the simultaneous genotyping of certain gene variants, particularly of SNPs associated with said pathologies associated with aging and the main objective of which is the use thereof in anti-aging medicine.

Aging is a multifactorial process taking place during the last stage of the life cycle and characterized by the progressive decrease of the functional capacity on all the tissues and organs of the body, and of the consequent ability to adapt to environmental stimuli. Life cycle is a specific characteristic, defined by a maximum potential duration between conception and death and a series of stages during which ontogenetic processes take place: growth, development, maturation and involution. Ontogenetic processes, the sequence in which they occur and their phenotypic expression are genetically programmed and environmentally limited. The sequential and differential expression of one and the same set of genes in specific environments causes the continuum of successive phenotypes corresponding to one and the same individual throughout his or her life cycle. The involutive processes associated with aging are manifested at molecular, cell and functional level with an evident expression in the visible phenotype.

Anti-aging medicine is the part of medicine based on the application of scientific research and of technologies for the prevention and early treatment of diseases related to age or caused by aging, with the objective of lengthening the life expectancy and at the same time improving the quality of life.

For the purpose of achieving an integral and objective assessment of the greater or lower adaptive capacity and capacity of resistance or vulnerability of a subject against most common diseases associated with aging, the inventors of the present invention have developed a method allowing a global assessment of the genetic risk a subject has of suffering from a pathology associated with aging from the calculation of the particular genetic risk of developing certain pathologies associated with aging, particularly, from the calculation of the following particular genetic risks:

1. Vascular risk;
2. Risk of osteoporosis
3. Carcinogenic risk; and
4. Risk of environmental stress and oxidative damage; and, optionally
5. Risk of adverse reactions to drugs.

Thus, the main objective of the present invention is developing an *in vitro* method for determining the global genetic risk of a subject to develope a pathology associated with aging from a combination of particular genetic risks, particularly, vascular risk, oncogenic risk, risk of osteoporosis, risk of environmental stress and oxidative damage and risk of adverse reactions to drugs.

Therefore, in one aspect, the invention relates to an *in vitro* method for determining the global genetic risk of a subject to develope a pathology associated with aging from a combination of particular genetic risks, hereinafter method of the invention, comprising:

i) simultaneously genotyping multiple human gene variants present in one or more genes of a subject associated with a pathology associated with aging in a biological sample of said subject;
ii) determining each particular genetic risk; and
iii) determining said global genetic risk according to the value of each particular genetic risk obtained in step ii).

As used in the present description, the term "gene variant" includes mutations, polymorphisms and allelic variants. A genetic variant is found among individuals within populations and among populations within species. In a particular embodiment, the authors of the present invention have selected a total of 69 human gene variants of 49 human genes associated with pathologies associated with aging (Table 1); nevertheless, different additional human gene variants in the same genes or in other human genes, associated with pathologies associated with aging, can be analyzed.

The term "gene mutation" relates to a variation in the nucleotide sequence of a nucleic acid wherein each possible sequence is present in a proportion less than 1% in a population.

The term "polymorphism" relates to a variation in the nucleotide sequence of a nucleic acid wherein each possible sequence is present in a proportion equal to or greater than 1% in a population; in a particular case, when said variation is the nucleotide sequence occurring in single nucleotide (A, C, T or G) it is called SNP.

The terms "allelic variant" or "allele" are used indistinctly in the present description and relate to a polymorphism occurring in one and the same locus in one and the same population.

For the purpose of simultaneously genotyping said human gene variants present in one or more genes of a subject associated with a pathology associated with aging by means of the method of the invention, in a first step the nucleic acid is extracted from a biological sample of the subject to be analyzed.

The extraction of the nucleic acid (e.g., DNA) from a biological sample containing it and coming from a subject, such as a human being, can be carried out by conventional methods optionally using commercial products useful for extracting said nucleic acid. Virtually any biological sample containing nucleic acid can be used to put the invention into practice; by way of a non-limiting illustration, said biological sample can be a sample of blood, saliva, plasma, serum, secretions, tissue, etc.

Once the nucleic acid is obtained, those regions of said nucleic acid containing the gene variants to be identified are amplified. As has been previously mentioned, as used in this description, the term "gene variant" includes polymorphisms (e.g., SNPs), mutations and allelic variants. To amplify the regions of nucleic acid containing the gene variants to be identified, specific oligonucleotide primers amplifying the genome fragments which can contain said gene variants are used. Said oligonucleotide primers are described in detail below, they form part of the present invention and form an additional aspect thereof. If desired, said amplification products can be optionally labeled during the amplification reaction to obtain labeled amplification products containing the gene variants to be identified.

Thus, the DNA regions containing the gene variants to be identified (target DNA regions) are subjected to an amplification reaction to obtain amplification products containing the gene variants to be identified. Although any technique or method allowing the amplification of all the DNA sequences containing the gene variants to be identified can be used, in a particular embodiment, said sequences are amplified by means of a multiplex amplification, which allows simultaneously genotyping said human gene variants to be identified present in one or more genes.

To perform a multiplex amplification, the use of pairs of oligonucleotide primers or primers capable of amplifying said target DNA regions containing the gene variants to be identified as has been previously explained is required. Virtually any pair of oligonucleotide primers allowing the specific amplification of said target DNA regions can be used, preferably, pairs of oligonucleotide primers allowing said amplification in the smallest possible number of amplification reactions. Thus, using the suitable pairs of oligonucleotide primers and conditions, all the target DNA regions necessary for the genotyping of said gene variants to be analyzed can be amplified with the smallest possible number of reactions. In a particular embodiment, said oligonucleotide primers are selected from the oligonucleotide primers identified as SEQ ID NO: 277-278, SEQ ID NO: 285-319, SEQ ID NO: 321-326, SEQ ID NO: 333-340, SEQ ID NO: 343-356, SEQ ID NO: 359-362, SEQ ID NO: 364, SEQ ID NO: 367, SEQ ID NO: 369-371, SEQ ID NO: 374, SEQ ID NO: 377-381, SEQ ID NO: 383, SEQ ID NO: 385-402 and SEQ ID NO: 404-414.

Once the DNA sequences containing the gene variants to be identified have been amplified, the method of the invention comprises the step of simultaneously genotyping multiple human gene variants present in one or more genes of a subject associated with a pathology associated with aging. In a particular embodiment of the invention, said step of simultaneous genotyping is performed by means of an analysis with DNA-chips, for example, using a suitable DNA-chip, such as the DNA-chip provided by this invention (DNA-chip of the invention, the features of which are mentioned below), i.e., by hybridization with specific probes for said human gene variants. Additionally or alternatively, said genotyping can be performed by means of the gene sequencing of said amplification products.

Thus, if desired, during the amplification reaction, the amplification products can be labeled for the purpose of being able to subsequently detect the hybridization between the probes present in the DNA-chip of the invention, immobilized in the support, and the target DNA fragments containing the gene variants to be detected. The amplification products can be labeled by conventional methods, for example, incorporating a labeled nucleotide during the amplification reaction or using labeled primers. Said labeling can be direct, for which fluorophores, for example, Cy3, Cy5, fluorescein, Alexa, etc., enzymes, for example, alkaline phosphatase, peroxidase, etc., radioactive isotopes, for example, 33P, 125I, etc., or any other marker known by the person skilled in the art can be used. Alternatively, said labeling can be indirect by means of using chemical methods, enzymatic methods, etc.; by way of illustration, the amplification product can incorporate a member of a specific binding pair, for example, avidin or streptavidin conjugated with a fluorochrome (marker), and the probe binds to the other member of the specific binding pair, for example, biotin (indicator), the reading being performed by means of fluorometry, etc., or the amplification product can incorporate a member of a specific binding pair, for example, an anti-digoxigenin antibody conjugated with an enzyme (marker), and the probe binds to the other member of the specific binding pair, for example, digoxigenin (indicator), etc., the substrate of the enzyme being transformed into a luminescent or fluorescent product and the reading being performed by means of chemiluminescence, fluorometry, etc.

In a particular embodiment, the amplification product is labeled by means of using a nucleotide labeled directly or indirectly with one or more fluorophores. In another particular embodiment, the amplification product is labeled by means of using primers labeled directly or indirectly with one or more fluorophores.

In a particular case, said amplification products are subjected to a fragmentation reaction to obtain fragmentation products containing the gene variants to be identified, and, in the event that said amplification products were not previously labeled in the amplification step, said fragmentation products containing the gene variants to be identified can be labeled.

The optionally labeled amplification products are subsequently subjected to fragmentation reaction for the purpose of increasing the efficiency of the subsequent hybridization, fragmentation products containing the gene variants to be identified thus being obtained. The fragmentation of the amplification products can be carried out by any conventional method, for example, contacting the amplification products with a DNAse.

In the event that the amplification products were not previously labeled during the amplification reaction, and in the event that after the hybridization process, an amplification or ligation reaction is not carried out directly in the support, the products resulting from the fragmentation reaction (fragmentation products) are subjected to a labeling which is either direct, using, for example, fluorophores, enzymes, radioactive isotopes, etc. or indirect, using, for example, specific binding pairs incorporating fluorophores, enzymes, etc., by means of conventional methods. In a particular embodiment, the amplification products have not been previously labeled during the amplification reaction, and the fragmentation products are subjected to a direct or indirect labeling with one or several markers, for example, one or several fluorophores, although other markers known by persons skilled in the art can be used.

The fragmentation products are then contacted with probes capable of detecting the corresponding gene variants under conditions allowing the hybridization between said fragmentation products and said probes. Said probes are deposited on a solid support following a predetermined arrangement, forming a DNA-chip (DNA-chip of the invention), the design and development of which must comply with a series of requirements to be able to used in the method of the invention in relation to the design of the probes, the number of probes to be deposited per gene variant to be detected, the number of probe replicas to be deposited, the distribution of the probes on the support, etc. The typical features of said DNA-chip of the invention and of said probes are described in detail below.

The hybridization of the fragmentation products with the probes capable of detecting the corresponding gene variants deposited on a support (DNA-chip of the invention) is carried out by conventional methods using conventional devices. In a particular embodiment, the hybridization is carried out in an automatic hybridization station. To carry out the hybridization, the fragmentation products are contacted with said probes (DNA-chip of the invention) under conditions allowing the hybridization between said fragmentation products and said probes. Stable hybridization conditions allow establishing the strand and the suitable length of the probes for the purpose of maximizing the discrimination, as mentioned below.

Once the hybridization process has ended, the image is captured and quantified. To that end, the image of the hybridized and developed DNA-chip is collected with a suitable device, for example, a scanner, the absolute fluorescence values of each probe as well as the background noise then being quantified. Therefore, in a particular embodiment, after the hybridization, or after the post-hybridization ligation or amplification reactions, the hybridized and developed DNA-chip is introduced in a scanner where it is subjected to a scanning to quantify the intensity of the labeling at the points in which the hybridization has occurred. Although virtually any scanner can be used, in a particular embodiment, said scanner is a confocal fluorescence scanner. In this case, the DNA-chip is introduced in the scanner and the signal emitted by the labeling upon being excited by a laser is scanned, the intensity of the points in which the hybridization has occurred being quantified. In a particular embodiment, said scanner is a white light scanner. Illustrative non-limiting examples of scanners which can be used according to the present invention are Axon, Agilent, Perkin Elmer scanners, etc.

The data is then analyzed and interpreted, which can be carried out by means of using any suitable genotyping software, such as the genotyping software referred to in Example 1, which uses the functions described in section 1.3.5 of said Example 1, and by means of using functions developed by the inventors to calculate the corresponding particular genetic risks and, from them, the global genetic risk, as described in detail below.

The analysis of the data and its interpretation is generally carried out by means of using computer programs (software). The inventors have developed a sequential method for processing and interpreting the experimental data generated by the DNA-chip of the invention which allows detecting each of the gene variants with sensitivity, specificity and reproducibility, and calculating the values of the corresponding particular genetic risks and, from them, the global genetic risk, by means of algorithms according to the genotype of the processed sample. The algorithms and computer software developed by the inventors allow facilitating and automating the application of the method of the invention.

The execution of the algorithms and computer software developed by the inventors to sequentially process and interpret the experimental data generated by the DNA-chip of the invention comprises performing a series of steps for characterizing each of the gene variants of interest, specifically:

- firstly, the own background noise of the absolute intensity values of all the probes is subtracted therefrom;
- the replicas corresponding to each of the 4 probes used to characterize each gene variant are then grouped;
- the mean intensity value for each of the 4 probes is calculated using the bounded mean of the replicas to eliminate the aberrant points;
- once the mean intensity values for each of the probes are known, Ratio 1 and Ratio 2 are calculated, wherein:

Ratio 1 is the proportion of the bounded mean of the intensities of the 10, 8 or 6 replicas of the probe 1 detecting gene variant A divided by the bounded mean of the 10, 8 or 6 replicas of the probe 1 detecting gene variant A plus the bounded mean of the 10, 8 or 6 replicas of the probe 2 detecting gene variant B and can be calculated by means of the equation:

$$\text{Ratio 1} = \frac{\text{Mean intensity probe 1}}{\text{Mean intensity probe 1} + \text{Mean intensity probe 2}}$$

Ratio 2 is the proportion of the bounded mean of the intensities of the 10, 8 or 6 replicas of the probe 3 detecting gene variant A divided by the bounded mean of the 10, 8 or 6 replicas of the probe 3 detecting gene variant A plus the bounded mean of the 10, 8 or 6 replicas of the probe 4 detecting gene variant B and can be calculated by means of the equation:

$$\text{Ratio 2} = \frac{\text{Mean intensity probe 3}}{\text{Mean intensity probe 3 + Mean intensity probe 4}}$$

- said ratios (Ratio 1 and Ratio 2) are substituted in three linear functions, which characterize each of the three possible genotypes:

| | |
|---|---|
| AA | Function 1 |
| AB | Function 2 |
| BB | Function 3 |

wherein
AA represents the genotype of a homozygous subject for gene variant A;
AB represents the genotype of a heterozygous subject for gene variants A and B;
BB represents the genotype of a homozygous subject for gene variant B;

Function 1 is the Linear Function characterizing the patients with genotype AA and consists of a linear combination of the variables Ratio 1 and Ratio 2;
Function 2 is the Linear Function for genotype AB and consists of a linear combination of the variables Ratio 1 and Ratio 2;
Function 3 is the Linear Function for genotype BB and consists of a linear combination of the variables Ratio 1 and Ratio 2;
wherein the linear combinations are formed by constants and cofactors accompanying the variables Ratio 1 and Ratio 2; and the function having a greater absolute value determines the genotype presented by the patient for the gene variant analyzed.

These ratios serve as variables for classifying the three groups for generating the linear functions.
In another particular embodiment of the invention, the genotyping of the multiple human gene variants or polymorphisms present in one or more genes of a subject associated with a pathology associated with aging in said biological sample is performed by gene sequencing.
Once said gene variants have been genotyped, each particular genetic risk is determined. Depending on whether the particular genetic risk to be calculated is formed by a combination of partial particular risks, said particular genetic risk is calculated applying different functions, as described below.
In a particular embodiment, the determination (calculation) of the particular genetic risk (step ii) of the method of the invention) comprises:

i) grouping the results obtained relating to each particular genetic risk of developing a pathology associated with aging;
ii) standardizing the value of each genotype of each gene variant analyzed;
iii) calculating each particular genetic risk such that:

iiia) when said particular genetic risk is not formed by a combination of partial particular risks, said particular genetic risk is calculated by means of equation [1]:

$$\text{PGR} = \frac{\sum_{i=1}^{n} xi}{\sum_{i=1}^{n} Lsi} \qquad [1]$$

where
PGR represents the particular genetic risk to be calculated;
$x_i$ represents the standardized value of the genotype characterized for a gene variant in a sample, in relation to the particular genetic risk to be calculated;
$Ls_i$ represents the value of the upper limit of the range of standardized values assigned to each gene variant, in relation to the particular genetic risk to be calculated; and
n is the number of gene variants analyzed in relation to the particular genetic risk to be calculated; or, alternatively,
iiib) when said particular genetic risk is formed by a combination of partial particular risks, said particular genetic risk is calculated by means of equation [2]:

$$\text{PGR} = \frac{\sum_{i=1}^{n} PPGRi}{no.PPGR} \qquad [2]$$

where
PGR represents the particular genetic risk to be calculated;
PPGRi represents the value calculated for each partial particular genetic risk which, in combination with other partial particular genetic risks, forms the particular genetic risk to be calculated, wherein said PPGRi is calculated by means of equation [3]:

$$\text{PPGRi} = \frac{\sum_{i=1}^{n} xi}{\sum_{i=1}^{n} Lsi} \qquad [3]$$

where
PPGRi has the previously mentioned meaning;
$x_i$ represents the standardized value of the genotype characterized for a gene variant in a sample, in relation to the partial particular genetic risk to be calculated;
$Ls_i$ represents the value of the upper limit of the range of standardized values assigned to each gene variant, in relation to the partial particular genetic risk to be calculated; and
n is the number of gene variants analyzed in relation to the partial particular genetic risk to be calculated; and
no.PPGR is the number of partial particular genetic risks analyzed in relation to the partial particular genetic risk to be calculated.

Thus, in a first step, after the genotyping of the human gene variants, said variants are grouped by particular genetic risks and partial particular genetic risks, i.e., the results of the analysis of the gene variants [mutations, polymorphisms (e.g., SNPs), allelic variants, etc.] are grouped by particular genetic risks and, where appropriate, by partial particular genetic risks, for the purpose of calculating the particular genetic risk of each pathology associated with aging. In a particular embodiment of the invention, said particular genetic risk is selected from the group formed by particular genetic risk associated with suffering from vascular disease (vascular risk), particular genetic risk associated with osteoporosis, particular genetic risk associated with carcinogenesis and particular genetic risk associated with environmental stress and oxidative damage. Likewise, in a particular embodiment, said vascular risk is determined according to the partial particular genetic risks selected from the group formed by partial particular genetic risk associated with lipid metabolism, partial particular genetic risk associated with thrombosis, partial particular genetic risk associated with ictus, partial particular genetic risk associated with high blood pressure and partial particular genetic risk associated with endothelial vulnerability.

Subsequently, in a second step, the value of each genotype of each gene variant is standardized or scored. In this sense, said values will be comprised in a range of standardized values, in which the genotype or genotypes of the highest risk of suffering from a certain pathology will comprise the value of the upper limit of said range of values, and the genotype or genotypes of the lowest risk of suffering from a certain pathology will comprise the value of the lower limit of said range of values. Thus, according to the genotype present in the sample analyzed, a corresponding standardized value is assigned to said genotype. The particular genetic risks are then calculated according to equation [1] or [2] depending on whether the particular genetic risk to be calculated is formed by a combination of partial particular risks. In a particular embodiment, the particular genetic risk associated with osteoporosis, the particular genetic risk associated with carcinogenesis and the particular genetic risk associated with environmental stress and oxidative damage are calculated by means of equation [1], whereas in another particular embodiment, the vascular risk is determined using equation [2] according to the partial particular genetic risks selected from the group formed by partial particular genetic risk associated with lipid metabolism, partial particular genetic risk associated with thrombosis, partial particular genetic risk associated with ictus, partial particular genetic risk associated with high blood pressure and partial particular genetic risk associated with endothelial vulnerability, such that, in this case, the particular genetic risk is calculated according to the values of the different partial particular genetic risks analyzed as shown in Example 1 attached to the present description.

In any case, the person skilled in the art will understand that, depending on whether partial particular genetic risks are used to determine the particular genetic risks, he will use the suitable equation in each case.

Vascular risk

The particular genetic risk associated with suffering from vascular risk or suffering from a vascular disease (VD) (vascular risk) is altogether one of the main causes of mortality and morbidity virtually everywhere in the world, therefore the development of models for predicting the risk of suffering from this type of disease, both for attempting to know the possible mechanisms affecting the increase of the risk and for being able to intervene early on and prevent them, is of great interest.

In this sense, the research of the molecular bases of VD has indicated genes which are involved in each of the sections and which confer susceptibility to this disease.

On one hand, the genes regulating everything related to lipid metabolism have been considered. In addition, it is also known that another of the conditions predisposing to VD is in the tendency for thrombus formation, therefore the inventors have searched for polymorphisms of risk among the genes involved in the coagulation cascade and the fibrinolytic system. On the other hand, the method of the invention analyzes genetic risk factors among those genes with influence at the level of structural and functional preservation of the vascular endothelium and among the genes involved in the defense mechanisms against oxidative stress.

Partial particular genetic risk related to the integrity of the lipid metabolism (lipid metabolism)

The term dyslipidemia relates to various pathologic conditions the only common element of which is a lipid metabolism alteration, with its subsequent alteration of the concentrations of lipids and lipoproteins in the blood. The predisposition to dyslipidemia is very heterogeneous at molecular level and it is important to evaluate the entire set since among each of the alleles or variants of every genetic polymorphism which are inherited in an individual, synergies or antagonisms may be established which will determine highly variable and particular risks and therefore vulnerabilities which enable individualizing each case not only in its global assessment, but also in relation to the therapeutic strategy to be used.

Partial particular genetic risk of thrombosis

According to the classic Virchow's triad, three interrelated factors must be taken into account in the formation of a thrombus: alteration of the blood vessel wall, of the blood flow and of the blood coagulability. It is precisely the alteration of this latter factor which favors the coagulation of the blood, or hypercoagulability or prothrombotic state, which is defined as thrombophilia.

As a general rule, a hypercoagulability state must be suspected in individuals with recurrent episodes of deep vein thromboses, pulmonary embolism, family history of thrombotic events, unusual sites of arterial and venous thrombosis and in children, adolescents or young adults with thrombotic events in general.

This section includes several gene variations which can act in a synergic manner (enhancing the pathogenic effect) or antagonistic manner (providing a natural compensation).

Partial particular genetic risk of high blood pressure

In this case, the state at hemodynamic level is analyzed, specifically assessing the renin-angiotensin system and the adrenergic receptors which basically predispose to high blood pressure and cardiovascular disease in general. The assessment thereof would also allow objectively defining, on molecular bases, the most effective therapeutic strategy to achieve the control in each case.

Partial particular genetic risk of endothelial vulnerability

The most evident function of the vascular endothelium is that of maintaining a dilated vascular tone in the exact proportion to preserve the blood pressure at normal values and allow tissue perfusion. This vasodilating function is exerted by the endothelium by means of the synthesis and secretion of relaxation factors such as nitric oxide (NO). Furthermore, the endothelium is an important element for maintaining the balance with platelets and coagulation factors and thus maintaining the fluidity of the blood in what is referred to as homeostatic balance (hemostasis) since the imbalance in one direction or the other will cause hemorrhage or thrombosis.

Most of the factors capable of attacking and damaging the endothelium come from the external environment and one of the most harmful among them is smoking. Nevertheless, there are several gene variations which determine a greater vulnerability to this damage and therefore contribute considerably to the general increase of vascular risk. These gene variations even worsen the damage which would already be caused by classic non-genetic risk factors themselves such as smoking.

In addition, it is known that homocysteine (HCT), a demethylated amino acid derived from methionine and, therefore, an intermediate of the methionine cycle, is metabolized by remethylation to methionine or by sulfuration to cysteine. For the remethylation, the methionine synthase needs vitamin B12 as a cofactor and folic acid as a substrate. For the transsulfuration, a cystathionine beta-synthase (CBS) and vitamin B6 as a cofactor are required. A defect in the remethylation or the transsulfuration leads to a hyperhomocysteinemia. Various studies have demonstrated that hyperhomocysteinemia, even when it is mild to moderate (greater than 12 nmol/mL) is an independent factor for brain ischemia, myocardial infarction, peripheral artery disease and carotid stenosis and it is therefore important to take it into account in the assessment of vascular risk. Although the causes coming from the external environment (non-genetic) are important among the causes thereof, there are important genetic alterations to be considered because they determine both the prognosis and the degree of therapeutic response of each case.

Oxidative stress is another factor which can also affect our better or worse response at endothelial level and at vascular level in general. For this reason, this factor can be considered in the molecular etiopathogenesis of general vascular disease, and this is none other than the degree of defensive potential against oxidative stress.

Ischemic cardiopathy and acute myocardial infarction can be the expression of a process starting with an excess of free radicals, which start the atherosclerotic process by damage in vascular wall, causing the penetration into the subendothelial space of low density lipoproteins (LDL) and therefore into the atherosclerotic plaque. Various scientific publications analyze the mechanisms of the human organism to produce and at the same time limit the production of reactive oxygen species. An excess of free radicals usually starts the damage of the vascular wall and LDL-cholesterol is involved in this process. A decrease in the incidence of cardiovascular diseases with individual antioxidant supplements has been demonstrated.

Once each particular genetic risk has been determined, the global genetic risk is determined by applying suitable functions. In a particular embodiment, the determination (calculation) of the global genetic risk is carried out by means of equation [4]:

$$\mathrm{GGR} = \frac{\sum PGR}{n} \qquad [4]$$

where
GGR represents the global genetic risk to be calculated;
PGR represents the value calculated for each particular genetic risk analyzed in relation to the global genetic risk to be calculated, and is calculated by means of the previously described equations [1] or [2]; and
n is the number of particular genetic risks analyzed in relation to the global genetic risk to be calculated.

Merely by way of a non-limiting illustration, the method provided by this invention for determining the global genetic risk a subject has of developing a pathology associated with aging comprises calculating or determining the following particular genetic risks:

1. Particular genetic risk associated with suffering from vascular disease (vascular risk);
2. Particular genetic risk associated with osteoporosis (risk of osteoporosis);
3. Particular genetic risk associated with carcinogenesis (carcinogenic risk); and
4. Particular genetic risk associated with environmental stress and oxidative damage.

Likewise, in a particular embodiment, said vascular risk is determined according to the partial particular genetic risks selected from the group formed by partial particular genetic risk associated with lipid metabolism, partial particular genetic risk associated with thrombosis, partial particular genetic risk associated with ictus, partial particular genetic risk associated with high blood pressure and partial particular genetic risk associated with endothelial vulnerability.

More specifically, in a particular embodiment, said partial particular genetic risk associated with lipid metabolism is determined according to the gene variants selected from the group formed by -75 G>A of the APOA1 gene, Arg3480Trp of the APOB gene, Arg3500Gln of the APOB gene, Arg3531Cys of the APOB gene, Cys112Arg of the APOE gene, Arg158Cys of the APOE gene, Arg451Gln of the CETP gene, TaqIB B1>B2 of the CETP gene, Gln192Arg of the PON1 gene, Gly595Ala of the SREBF2 gene, Leu7Pro of the NPY gene and combinations thereof.

In another particular embodiment, said particular genetic risk associated with thrombosis is determined according to the gene variants selected from the group formed by 4G>5G of the PAI1 gene, Leu33Pro of the ITGB3 gene, 20210 G>A of the FII gene, Arg506Gln of the FV Leiden gene, Val34Leu of the F13A1 gene, Ala222Val of the MTHFR gene, 833 T>C of the CBS gene, 844ins68 of the CBS gene, -455 G>A of the FGB gene and combinations thereof.

In another particular embodiment, said particular genetic risk associated with ictus is determined according to the gene variants selected from the group formed by 4G>5G of the PAI1 gene, Leu33Pro of the ITGB3 gene, 20210 G>A of the FII gene, Arg506Gln of the FV Leiden gene, Val34Leu of the F13A1 gene and combinations thereof.

In another particular embodiment, said particular genetic risk associated with high blood pressure is determined according to the gene variants selected from the group formed by Gly389Arg of the ADRB1 gene; Gln27Glu of the ADRB2 gene, Gly16Arg of the ADRB2 gene, Met235Thr of the AGT gene, 1166 A>C of the AGTR1 gene, 393 T>C (Ile131Ile) of the GNAS gene, 825 C>T (Ser275Ser) of the GNB3 gene, intron 16 ins/del of the ACE gene, Trp64Arg of the ADRB3 gene and combinations thereof.

In another particular embodiment, said particular genetic risk associated with endothelial vulnerability is determined according to the gene variants selected from the group formed by 5A>6A of the MMP3 gene, -786 T>C of the NOS3 gene, Glu298Asp of the NOS3 gene, Ala222Val of the MTHFR gene, 833 T>C of the CBS gene, 844ins68 of the CBS gene, Pro319Ser of the GJA4 gene and combinations thereof.

In addition, in a particular embodiment of the invention, said particular genetic risk associated with osteoporosis is determined according to the gene variants selected from the group formed by 1546 G>T of the COL1A1 gene, IVS1-397 T>C p>P (PvuII) of the ESR1 gene, b>B of the VDR gene and combinations thereof.

In another particular embodiment, said particular genetic risk associated with carcinogenesis is determined according to the gene variants selected from the group formed by -34 A>G of the CYP17A1 gene, Ile462Val of the CYP1A1 gene, T3801C of the CYP1A1 gene, Leu432Val of the CYP1B1 gene, Allele*4 (Asn453Ser) of the CYP1B1 gene, 1558 C>T of the CYP19A1 gene, Val158Met (Allele*2) of the COMT gene, 331 G>A of the PGR gene, IVS1 -397 T>C p>P (PvuII) of the ESR1 gene, b>B of the VDR gene, Ala49Thr of the SRD5A2 gene, Val89Leu of the SRD5A2 gene, Ala541Thr of the ELAC2 gene and combinations thereof.

In a particular embodiment, said particular genetic risk associated with environmental stress and oxidative damage is determined according to the gene variants selected from the group formed by Cys326Ser of the OGG1 gene, Ala16Val of the SOD2 gene, Arg213His of the SULT1A1 gene, present>null GSTM1, present>null GSTT1, Ile105Val of the GSTP1 gene, Ala114Val of the GSTP1 gene, Val158Met (Allele*2) of the COMT gene, -174 C>G of the IL6 gene, -1082 G>A of the IL10 gene, R64Q of the NAT2 gene, 282 C>T (Y94Y) of the NAT2 gene, I114T of the NAT2 gene, 481C>T (L161L) of the NAT2 gene, R197Q of the NAT2 gene, K268R of the NAT2 gene, G286E of the NAT2 gene and combinations thereof.

If desired, the method of the invention further comprises evaluating or determining the particular genetic risk associated with the response to drugs, i.e., the particular genetic risk of suffering from adverse reactions to drugs.

In a particular embodiment, said particular genetic risk associated with the response to drugs is determined according to the gene variants selected from the group formed by R64Q, 282 C>T (Y94Y), I114T, 481C>T (L161L), R197Q, K268R and G286E of the NAT2 gene; Arg144Cys (allele*2) and Ile359Leu (allele*3) of the CYP2C9 gene; 681 G>A (Pro227Pro) (allele*2) of the CYP2C19 gene; 2549 A>del (allele*3), 1847 G>A (allele*4) and 1707 del>T (allele*6) of the CYP2D6 gene; and combinations thereof.

Therefore, in a particular embodiment, the method of the invention comprises simultaneously genotyping multiple human gene variants or polymorphisms present in one or more genes of a subject associated with a pathology associated with aging in a biological sample of said subject, wherein said gene variant [mutation, polymorphism (e.g., SNP) or allelic variation] to be genotyped is selected from the group formed by the intron 16 ins/del polymorphism of the ACE gene; the Gly389Arg polymorphism of the ADRB1 gene; the Gln27Glu and Gly16Arg polymorphisms of the ADRB2 gene; the Trp64Arg polymorphism of the ADRB3 gene; the Met235Thr polymorphism of the AGT gene; the 1166 A>C polymorphism of the AGTR1 gene; the -75 G>A polymorphism of the APOA1 gene; the Arg3480Trp, Arg3500Gln and Arg3531Cys polymorphisms of the APOB gene; the Cys112Arg and Arg158Cys polymorphisms of the APOE gene; the 833 T>C and 844ins68 polymorphisms of the CBS gene; the TaqIB B1>B2 and Arg451Gln polymorphisms of the CETP gene; the 1546 G>T polymorphism of the COL1A1 gene; the Val158Met (Allele*2) polymorphism of the COMT gene; the -34 A>G polymorphism of the CYP17A1 gene; the 1558 C>T polymorphism of the CYP19A1 gene; the Ile462Val and T3801C polymorphism of the CYP1A1 gene; the Leu432Val and Allele*4 (Asn453Ser) polymorphism of the CYP1B1 gene; the Arg144Cys (allele*2) and Ile359Leu (allele*3) polymorphism of the CYP2C9 gene; the 681 G>A (Pro227Pro) (allele*2) polymorphism of the CYP2C19 gene; the 2549 A>del (allele*3), 1847 G>A (allele*4) and 1707 del>T (allele*6) polymorphism of the CYP2D6 gene; the Ala541Thr polymorphism of the ELAC2 gene; the IVS1 -397 T>C p>P (PvuII) polymorphism of the ESR1 gene; the Val34Leu polymorphism of the F13A1 gene; the -455 G>A polymorphism of the FGB gene; the 20210 G>A polymorphism of the FII gene; the Arg506Gln polymorphism of the FV Leiden gene; the Pro319Ser polymorphism of the GJA4 gene; the 393 T>C (Ile131Ile) polymorphism of the GNAS gene; the 825 C>T (Ser275Ser) polymorphism of the GNB3 gene; the present>null GSTM1 polymorphism; the Ile105Val and Ala114Val polymorphisms of the GSTP1 gene; the present>null GSTT1 polymorphism; the - 174 C>G polymorphism of the IL6 gene; the -1082 G>A polymorphism of the IL10 gene; the Leu33Pro polymorphism of the ITGB3 gene; the 5A>6A polymorphism of the MMP3 gene; the Ala222Val polymorphism of the MTHFR gene; the R64Q, 282 C>T (Y94Y), I114T, 481C>T (L161L), R197Q, K268R and G286E polymorphisms of the NAT2 gene; the -786 T>C and Glu298Asp polymorphisms of the NOS3 gene; the Leu7Pro polymorphism of the NPY gene; the Cys326Ser polymorphism of the OGG1 gene; the 4G>5G polymorphism of the PAI1 gene; the 331 G>A polymorphism of the PGR gene; the Gln192Arg polymorphism of the PON1 gene; the Ala16Val polymorphism of the SOD2 gene; the Ala49Thr and Val89Leu polymorphisms of the SRD5A2 gene; the Gly595Ala polymorphism of the SREBF2 gene; the Arg213His polymorphism of the SULT1A1 gene; the b>B polymorphism of the VDR gene; and combinations thereof.

Likewise, if desired, the method of the invention further comprises genotyping one or more additional gene variants associated with pathologies associated with aging.

The method of the invention is therefore an extracorporeal *in vitro* method for the simultaneous, sensitive, specific and reproducible genotyping of multiple human gene variants present in different genes, associated with pathologies associated with aging. The method of the invention allows identifying changes of nucleotides, insertions, deletions, etc. and determining the genotype of a subject for the gene variants related to pathologies associated with aging analyzed.

To put the method of the invention into practice, a genotyping DNA-chip useful for detecting said gene variants has been developed.

Therefore, in another aspect, the invention relates to a DNA-chip, hereinafter DNA-chip of the invention, comprising a support on which there is deposited a plurality of probes useful for detecting human gene variants present in one or more genes associated with pathologies associated with aging. In a particular embodiment, said probes are selected from the group formed by the probes identified as SEQ ID NO: 1-13, SEQ ID NO: 15, SEQ ID NO: 17-44, SEQ ID NO: 53-128, SEQ ID NO: 130, SEQ ID NO: 132-172, SEQ ID NO: 181-200, SEQ ID NO: 202, SEQ ID NO: 204, SEQ ID NO: 206, SEQ ID NO: 208, SEQ ID NO: 210, SEQ ID NO: 212, SEQ ID NO: 222, and SEQ ID NO: 224-276 (see section 1.1 of Example 1 attached to the description).

The DNA-chip of the invention comprises a support on which there is deposited a plurality of probes useful for detecting human gene variants present in one or more genes associated with pathologies associated with aging. For every gene variant, the DNA chip of the invention comprises 4 probes, of which 2 probes detect a first gene variant and the other 2 detect a second gene variant, wherein the number of replicas of each of said probes is 10, 8 or 6 replicas and the two probes do not have to be identical. Said probes are deposited following a certain pattern and distributed homogeneously between the 2 areas forming the DNA-chip but not grouped by gene variant to be detected, i.e., they are distributed along the length and width of the chip and furthermore they are not grouped within one and the same gene variant.

The DNA-chip of the invention can also contain, if desired, oligonucleotides deposited on the support useful as positive and negative controls of the amplification and/or hybridization reactions.

For the present DNA-chip to allow the simultaneous, sensitive, specific and reproducible detection of gene variants, be completely effective and actually be a useful tool in anti-aging medicine, the clinical and practical translation of this analysis requires the corresponding algorithm integrating the real value of all these polymorphisms, taking into account the synergies and antagonisms occurring between them, presenting a risk in absolute values which is always different depending on the individual analyzed. The real value of this risk must be considered in the global context of each case taking into account all the classic (non-genetic) risk factors. An objective analysis and unitary vision of a complex and multifactorial disease such as for example vascular disease will only be assured in this way.

For the purpose of maximally decreasing the rate of false positives and negatives, the DNA-chip of the invention comprises two pairs of probes for detecting each genetic variation. Each pair of probes is formed by a specific probe for the detection of a genetic variation (e.g., allele A) and by another probe designed for the detection of another genetic variation (e.g., allele B). In the case of point mutations, the base differing between allele A and B (base to be interrogated) is placed in the central position of the probe, which assured the maximum specificity in the hybridization. In the case of insertions, duplications or deletions, there are several bases which can be interrogated. However, the design becomes completely equivalent considering as the central position the first nucleotide which is different in the normal sequence with respect to the mutated sequence.

In a particular embodiment, the DNA-chip of the invention comprises 10 replicas of each of the 4 probes used to detect each genetic variation; in another particular embodiment, the DNA-chip of the invention comprises 8 replicas of each of the 4 probes used to detect each genetic variation; and, in another particular embodiment, the DNA-chip of the invention comprises 6 replicas of each of the 4 probes used to detect each genetic variation.

The arrangement (placement) of the probes in the support is predetermined. In a particular embodiment, although the probes deposited on the support maintain a predetermined arrangement, they are not grouped by genetic variation but rather they have a random distribution, which, if desired, can always be the same.

The capacity of the specific probes of gene variants to discriminate between the gene variants (e.g., allele A and allele B) depend on the hybridization conditions, on the sequence flanking the mutation and on the secondary structure of the sequence in which the polymorphism is to be detected. Stable hybridization conditions allow establishing the strand and the suitable length of the probes for the purpose of maximizing the discrimination. Starting from probes of 25 nucleotides detecting a genetic variation (e.g., allele A) and another genetic variation (e.g., allele B) in both strands (sense strand and antisense strand), a mean of 8 experimentally assayed probes is required in order to be left with the two definitive pairs.

In a particular embodiment, for every genetic variation to be detected by means of the DNA-chip of the invention, the designed probes interrogate both strands, with lengths typically comprised between 19 and 27 nucleotides, and the hybridization temperature varies between 75°C and 85°C.

Table 1 (Example 1) includes a list of gene variants associated with pathologies associated with aging; nevertheless, probes allowing the identification of other gene variants associated with said diseases can be incorporated in the DNA-chips of the invention.

As has been mentioned previously, the DNA-chip of the invention can optionally contain oligonucleotides deposited on the support useful as positive and negative controls of the amplification and/or hybridization reactions. In a particular embodiment, the DNA-chip of the invention comprises oligonucleotides deposited on the support useful as positive and negative controls of the hybridization reactions. In general, each of the sub-arrays forming a DNA-chip is flanked by external hybridization controls which allow easily locating the points on the support. Although with the same sequence, the DNA-chip has two external hybridization controls labeled, for example, with a fluorophore (e.g., Cy3, Cy5, etc.), which serve to evaluate the hybridization quality in both channels. In a particular embodiment, the nucleotide sequence of the external control is the one identified in SEQ ID NO: 415 (CEH), and the sequences of the oligonucleotides for the detection thereof are those identified in SEQ ID NO: 416 and SEQ ID NO: 417.

The support on which the plurality of probes is deposited can be any solid surface on which the oligonucleotides can be bound. Virtually any support on which an oligonucleotide used in the production of DNA-chips can be bound or immobilized can be used to put this invention into practice. By way of illustration, said support can be a non-porous support, for example, a support made of glass, silicon, plastic, etc., or a porous support, for example, membranes (nylon, nitrocellulose, etc.), microparticles, etc. In a particular embodiment, said support is a glass slide.

The probes are immobilized (bound) on the support using conventional techniques for immobilizing oligonucleotides on the surface of the supports. Said techniques depend, among other factors, on the nature of the support used [porous (membranes, microparticles, etc.) or non-porous (glass, plastic, silicon, etc.)]. In general, the probes can be immobilized on the support by means of using non-covalent immobilization techniques or by means of using immobilization techniques based on the covalent binding of the probes to the surface of the support by means of chemical processes.

The preparation of non-porous supports (e.g., glass, silicon, plastic, etc.) generally requires a prior treatment with reactive groups (e.g., amino, aldehyde, etc.) or coating the surface of the support with a member of a specific binding pair (e.g., avidin, streptavidin, etc.). Likewise, it is generally convenient to previously activate the probes to be immobilized by means of thiol, amino groups, etc., or biotin, etc., for the purpose of achieving a specific immobilization of the probes on the support.

The immobilization of the probes on the support can be carried out by conventional methods, for example, by means of techniques based on the synthesis *in situ* of the probes on the support itself (e.g., photolithography, direct chemical synthesis, etc.), or by means of techniques based on the use of robotized arms depositing the corresponding pre-synthesized probe (printing without contact, printing by contact, etc.), etc.

The arrangement (placement) of the probes in the support is predetermined. In a particular embodiment, although the probes deposited on the solid support maintain a predetermined arrangement, they are not grouped by genetic variation but rather they have a random distribution, which, if desired, can always be the same.

In a particular embodiment, the support is a glass slide and, in this case, the probes, in the established number of replicas (6, 8 or 10), are printed in glass slides which are previously treated, for example, amino-silanized, using automatic DNA-chip production equipment by the deposition of the oligonucleotides in the glass slide ("microarrayer") under suitable conditions, for example, by means of crosslinking with ultraviolet radiation and baking (80°C), maintaining the humidity and temperature controlled during the deposition process, typically between 40-50% of relative humidity and 20°C of temperature.

The replicas (probes) are distributed in the printing plates, containing the oligonucleotides in solution, such that they are printed by a number of different tips equal to half the replicas. The replicas are distributed homogeneously between the areas or sectors (sub-arrays) forming the DNA-chip. The number of replicas as well as their homogeneous distribution along the length and width of the DNA-chip minimize the experimental variability coming from the printing and hybridization processes. Likewise, positive and negative hybridization controls are printed. In general, each of the sub-arrays forming the DNA-chip is flanked by external hybridization controls which allow easily locating the points on the support. Although with the same sequence, the DNA-chip has two external hybridization controls labeled, for example, with a fluorophore (e.g., Cy3, Cy5, etc.), which serve to evaluate the hybridization quality in both channels. In a particular embodiment, the nucleotide sequence of the external control is the one previously identified as "CEH" and the sequences of the oligonucleotides for the detection thereof are those previously identified as ON1 and ON2.

A commercial DNA can be used to control the quality of the process for manufacturing the DNA-chip in terms of hybridization signal, background noise, specificity, sensitivity, reproducibility of each replica (coefficient of variation) as well as of the size and shape of the printed points (probes). By way of illustration, as a quality control of the printing of the DNA-chips of the invention, hybridization is carried out with a DNA with known genotype of one of every certain number of supports loaded with the probes, for example, every 20 printed supports. The correct genotyping of this control DNA is verified.

The inventors have designed, produced and validated the clinical use of the method of the invention in the detection of gene variants associated with pathologies associated with aging. Therefore, in a particular embodiment, the DNA-chip of the invention is a DNA-chip allowing the simultaneous, sensitive, specific and reproducible detection of gene variants associated with pathologies associated with aging; illustrative non-limiting examples of gene variants associated with aging which can be identified are shown in Table 1; nevertheless, the list of gene variants contained in said table can be increased with other gene variants which are gradually identified subsequently and which are associated with pathologies associated with aging. The sequences of all the genes mentioned in Table 1 are known and are shown, among others, on the following websites: GeneBank (NCBI), and Snpper.chip.org (Innate Immunity PGA).

In another aspect, the invention relates to a kit for putting the method of the invention into practice, hereinafter kit of the invention, comprising a DNA-chip of the invention comprising a support on which there is deposited a plurality of probes allowing the detection of human gene variants present in one or more genes associated with pathologies associated with aging. In a particular embodiment, the kit of the invention contains a protocol for the detection of said gene variants, comprising the use of an algorithm for the interpretation of the data generated with the application of said method; and, optionally, a protocol for the calculation of the risk conferred by said gene variants, comprising the use of various algorithms generated with the application of said method; and, optionally, a computer software facilitating, automatizing and assuring the reproducibility of the application of said algorithm for the interpretation of the data generated with the application of the invention.

The following example serves to illustrate the invention and must not be considered as limiting the scope thereof.

EXAMPLE 1

Detection of human gene variants (polymorphisms) associated with pathologies associated with aging, using a DNA-chip

1.1 Design of the DNA-chip

A DNA-chip was designed and manufactured to detect human gene variants, particularly SNPs (Single Nucleotide Polymorphisms) associated with pathologies associated with aging which allow the simultaneous, specific and reproducible detection of gene variants associated with said pathologies.

A list of gene variants associated with pathologies associated with aging is included below; nevertheless, probes which allow the identification of other gene variants associated with said diseases can be incorporated in the DNA-chip of the invention.

Table 1

| Gene variants of pathologies associated with aging analyzed | |
|---|---|
| SNP01 | ACE intron 16 ins/del |
| SNP02 | ADRB1 Gly389Arg |
| SNP03 | ADRB2 Gln27Glu |
| SNP04 | ADRB2 Gly16Arg |
| SNP05 | ADRB3 Trp64Arg |
| SNP06 | AGT Met235Thr |
| SNP07 | AGTR1 1166 A>C |
| SNP08 | APOA1 -75 G>A |
| SNP09 | APOB Arg3480Trp |
| SNP10 | APOB Arg3500Gln |
| SNP11 | APOB Arg3531Cys |

(continued)

| Gene variants of pathologies associated with aging analyzed | |
|---|---|
| SNP12 | APOE Cys112Arg |
| SNP13 | APOE Arg158Cys |
| SNP14 | CBS 833 T>C |
| SNP15 | CBS 844ins68 |
| SNP16 | CETP TaqIB B1>B2 |
| SNP17 | CETP Arg451Gln |
| SNP18 | COL1A1 1546 G>T |
| SNP19 | COMT Val158Met (Allele*2) |
| SNP20 | CYP17A1 -34 A>G |
| SNP21 | CYP19A1 1558 C>T |
| SNP22 | CYP1A1 Ile462Val |
| SNP23 | CYP1A1 T3801C |
| SNP24 | CYP1B1 Leu432Val |
| SNP25 | CYP1B1 Allele*4 (Asn453Ser) |
| SNP26 | CYP2C9 Arg144Cys (allele*2) |
| SNP27 | CYP2C9 Ile359Leu (allele*3) |
| SNP28 | CYP2C19 681 G>A (Pro227Pro) (allele*2) |
| SNP29 | CYP2D6 2549 A>del (allele*3) |
| SNP30 | CYP2D6 1847 G>A (allele*4) |
| SNP31 | CYP2D6 1707 del>T (allele*6) |
| SNP32 | ELAC2 Ala541Thr |
| SNP33 | ESR1 IVS1 -397 T>C (PvuII) p>P |
| SNP34 | F13A1 Val34Leu |
| SNP35 | FGB -455 G>A |
| SNP36 | FII 20210 G>A |
| SNP37 | FV Leiden Arg506Gln |
| SNP38 | GJA4 Pro319Ser |
| SNP39 | GNAS 393 T>C (Ile131Ile) |
| SNP40 | GNB3 825 C>T (Ser275Ser) |
| SNP41 | GSTM1 present>null |
| SNP42 | GSTP1 Ile105Val |
| SNP43 | GSTP1 Ala114Val |
| SNP44 | GSTT1 present>null |
| SNP45 | IL6 -174 C>G |
| SNP46 | IL10 -1082 G>A |
| SNP47 | ITGB3 Leu33Pro |
| SNP48 | MMP3 5A>6A |
| SNP49 | MTHFR Ala222Val |
| SNP50 | NAT2 R64Q |
| SNP51 | NAT2 282 C>T (Y94Y) |
| SNP52 | NAT2 I114T |
| SNP53 | NAT2 481C>T (L161L) |
| SNP54 | NAT2 R197Q |
| SNP55 | NAT2 K268R |
| SNP56 | NAT2 G286E |
| SNP57 | NOS3 -786 T>C |
| SNP58 | NOS3 Glu298Asp |
| SNP59 | NPY Leu7Pro |
| SNP60 | OGG1 Cys326Ser |
| SNP61 | PAI1 4G>5G |

(continued)

| Gene variants of pathologies associated with aging analyzed | |
|---|---|
| SNP62 | PGR 331 G>A |
| SNP63 | PON1 Gln192Arg |
| SNP64 | SOD2 Ala16Val |
| SNP65 | SRD5A2 Ala49Thr |
| SNP66 | SRD5A2 Val89Leu |
| SNP67 | SREBF2 Gly595Ala |
| SNP68 | SULT1A1 Arg213His |
| SNP69 | VDR b>B |

In this specific case, the designed and manufactured DNA-chip consists of a support (glass slide) containing on its surface a plurality of probes which allow the detection of the aforementioned gene variants. These probes are capable of hybridizing with the amplified target sequences of genes associated with pathologies associated with aging the genetic variation of which is to be analyzed. The DNA sequences of each of the probes used are the following [generally, the name of the gene and the genetic variation (change of the amino acid, change of nucleotide, "ins": insertion, "del": deletion) are indicated]:

Probes used

| | | |
|---|---|---|
| **SNP01** | **ACE** | **Intron 16 ins/del** |
| SEQ ID NO: | 1 | GATTACAGGCGTGATACAGTCAC |
| SEQ ID NO: | 2 | GTGACTGTATCACGCCTGTAATC |
| SEQ ID NO: | 3 | AGACCTGCTGCCTATACAGTCAC |
| SEQ ID NO: | 4 | GTGACTGTATAGGCAGCAGGTCT |
| **SNP02** | **ADRB1** | **Gly389Arg** |
| SEQ ID NO: | 5 | AGGCCTTCCAGCGACTGCTCTGC |
| SEQ ID NO: | 6 | GCAGAGCAGTCGCTGGAAGGCCT |
| SEQ ID NO: | 7 | AGGCCTTCCAGGGACTGCTCTGC |
| SEQ ID NO: | 8 | GCAGAGCAGTCCCTGGAAGGCCT |
| **SNP03** | **ADRB2** | **Gln27Glu** |
| SEQ ID NO: | 9 | ACGTCACGCAGGAAAGGGACGAG |
| SEQ ID NO: | 10 | CGTCACGCAGGAAAGGGACGA |
| SEQ ID NO: | 11 | ACGTCACGCAGCAAAGGGACGAG |
| SEQ ID NO: | 12 | CGTCACGCAGCAAAGGGACGA |
| **SNP04** | **ADRB2** | **Gly16Arg** |
| SEQ ID NO: | 13 | TGGCACCCAATAGAAGCCATGCG |
| SEQ ID NO: | 14 | CTGGCACCCAATAGAAGCCATGCGC |
| SEQ ID NO: | 15 | TGGCACCCAATGGAAGCCATGCG |
| SEQ ID NO: | 16 | CTGGCACCCAATGGAAGCCATGCGC |
| **SNP05** | **ADRB3** | **Trp64Arg** |
| SEQ ID NO: | 17 | TGGCCATCGCCTGGACTCCGAGA |
| SEQ ID NO: | 18 | TCTCGGAGTCCAGGCGATGGCCA |
| SEQ ID NO: | 19 | TGGCCATCGCCCGGACTCCGAGA |
| SEQ ID NO: | 20 | TCTCGGAGTCCGGGCGATGGCCA |

(continued)

| | | |
|---|---|---|
| **SNP06** | **AGT** | **Met235Thr** |
| SEQ ID NO: | 21 | GGCTGCTCCCTGACGGGAGCCAGTGTG |
| SEQ ID NO: | 22 | CACACTGGCTCCCGTCAGGGAGCAGCC |
| SEQ ID NO: | 23 | GGCTGCTCCCTGATGGGAGCCAGTGTG |
| SEQ ID NO: | 24 | CACACTGGCTCCCATCAGGGAGCAGCC |
| **SNP07** | **AGTR1** | **1166 A>C** |
| SEQ ID NO: | 25 | ACCAAATGAGCATTAGCTACTTT |
| SEQ ID NO: | 26 | AAAGTAGCTAATGCTCATTTGGT |
| SEQ ID NO: | 27 | ACCAAATGAGCCTTAGCTACTTT |
| SEQ ID NO: | 28 | AAAGTAGCTAAGGCTCATTTGGT |
| **SNP08** | **APOA1** | **-75 G>A** |
| SEQ ID NO: | 29 | AGCCCAGCCCCGGCCCTGTTG |
| SEQ ID NO: | 30 | GCCCAGCCCCGGCCCTGTT |
| SEQ ID NO: | 31 | AGCCCAGCCCTGGCCCTGTTG |
| SEQ ID NO: | 32 | GCCCAGCCCTGGCCCTGTT |
| **SNP09** | **APOB** | **Arg3480Trp** |
| SEQ ID NO: | 33 | CGGTTCTTTCTCGGGAATATTCA |
| SEQ ID NO: | 34 | TGAATATTCCCGAGAAAGAACCG |
| SEQ ID NO: | 35 | CGGTTCTTTCTTGGGAATATTCA |
| SEQ ID NO: | 36 | TGAATATTCCCAAGAAAGAACCG |
| **SNP10** | **APOB** | **Arg3500Gln** |
| SEQ ID NO: | 37 | CAAGAGCACACGGTCTTCAGTGA |
| SEQ ID NO: | 38 | TCACTGAAGACCGTGTGCTCTTG |
| SEQ ID NO: | 39 | CAAGAGCACACAGTCTTCAGTGA |
| SEQ ID NO: | 40 | TCACTGAAGACTGTGTGCTCTTG |
| **SNP11** | **APOB** | **Arg3531Cys** |
| SEQ ID NO: | 41 | CCACACTCCAACGCATATATTCC |
| SEQ ID NO: | 42 | GGAATATATGCGTTGGAGTGTGG |
| SEQ ID NO: | 43 | CCACACTCCAATGCATATATTCC |
| SEQ ID NO: | 44 | GGAATATATGCATTGGAGTGTGG |
| **SNP12** | **APOE** | **Cys112Arg** |
| SEQ ID NO: | 45 | ATGGAGGACGTGTGCGGCCGCCTGG |
| SEQ ID NO: | 46 | CCAGGCGGCCGCACACGTCCTCCAT |
| SEQ ID NO: | 47 | ATGGAGGACGTGCGCGGCCGCCTGG |
| SEQ ID NO: | 48 | CCAGGCGGCCGCGCACGTCCTCCAT |
| **SNP13** | **APOE** | **Arg158Cys** |
| SEQ ID NO: | 49 | GACCTGCAGAAGCGCCTGGCAGTGT |
| SEQ ID NO: | 50 | ACACTGCCAGGCGCTTCTGCAGGTC |
| SEQ ID NO: | 51 | GACCTGCAGAAGTGCCTGGCAGTGT |
| SEQ ID NO: | 52 | ACACTGCCAGGCACTTCTGCAGGTC |
| **SNP14** | **CBS** | **833 T>C** |
| SEQ ID NO: | 53 | GATCCACCCCAGTGATCTGCAGA |
| SEQ ID NO: | 54 | ATCCACCCCAGTGATCTGCAG |
| SEQ ID NO: | 55 | GATCCACCCCAATGATCTGCAGA |
| SEQ ID NO: | 56 | ATCCACCCCAATGATCTGCAG |
| **SNP15** | **CBS** | **844ins68** |
| SEQ ID NO: | 57 | TGGGGTGGATCATCCAGGTGGGG |
| SEQ ID NO: | 58 | CCCCACCTGGATGATCCACCCCA |
| SEQ ID NO: | 59 | TGGGGTGGATCCCGAAGGGTCCA |
| SEQ ID NO: | 60 | TGGACCCTTCGGGATCCACCCCA |

(continued)

| | | |
|---|---|---|
| **SNP16** | **CETP** | **TaqIB B1>B2** |
| SEQ ID NO: | 61 | CACTGGGGTTCGAGTTAGGGTTC |
| SEQ ID NO: | 62 | GAACCCTAACTCGAACCCCAGTG |
| SEQ ID NO: | 63 | CACTGGGGTTCAAGTTAGGGTTC |
| SEQ ID NO: | 64 | GAACCCTAACTTGAACCCCAGTG |
| **SNP17** | **CETP** | **Arg451Gln** |
| SEQ ID NO: | 65 | GATTATCACTCGAGATGTGAGTA |
| SEQ ID NO: | 66 | ATTATCACTCGAGATGTGAGT |
| SEQ ID NO: | 67 | GATTATCACTCAAGATGTGAGTA |
| SEQ ID NO: | 68 | ATTATCACTCAAGATGTGAGT |
| **SNP18 COL1A1 1546 G>T** | | |
| SEQ ID NO: | 69 | TCATCCCGCCCCCATTCCCTGGG |
| SEQ ID NO: | 70 | CATCCCGCCCCCATTCCCTGG |
| SEQ ID NO: | 71 | TCATCCCGCCCACATTCCCTGGG |
| SEQ ID NO: | 72 | CATCCCGCCCACATTCCCTGG |
| **SNP19** | **COMT** | **Val158Met (Allele*2)** |
| SEQ ID NO: | 73 | ATTTCGCTGGCGTGAAGGACAAG |
| SEQ ID NO: | 74 | CTTGTCCTTCACGCCAGCGAAAT |
| SEQ ID NO: | 75 | ATTTCGCTGGCATGAAGGACAAG |
| SEQ ID NO: | 76 | CTTGTCCTTCATGCCAGCGAAAT |
| **SNP20** | **CYP17A1** | **-34 A>G** |
| SEQ ID NO: | 77 | TCTACTCCACTGCTGTCTATC |
| SEQ ID NO: | 78 | AGATAGACAGCAGTGGAGTAGAA |
| SEQ ID NO: | 79 | TCTACTCCACCGCTGTCTATC |
| SEQ ID NO: | 80 | AGATAGACAGCGGTGGAGTAGAA |
| **SNP21** | **CYP19A1** | **1558 C>T** |
| SEQ ID NO: | 81 | TGGTCAGTACCCACTCTGGAGCA |
| SEQ ID NO: | 82 | TGCTCCAGAGTGGGTACTGACCA |
| SEQ ID NO: | 83 | TGGTCAGTACCTACTCTGGAGCA |
| SEQ ID NO: | 84 | TGCTCCAGAGTAGGTACTGACCA |
| **SNP22** | **CYP1A1** | **Ile462Val** |
| SEQ ID NO: | 85 | TCGGTGAGACCATTGCCCGCTGG |
| SEQ ID NO: | 86 | CCAGCGGGCAATGGTCTCACCGA |
| SEQ ID NO: | 87 | TCGGTGAGACCGTTGCCCGCTGG |
| SEQ ID NO: | 88 | CCAGCGGGCAACGGTCTCACCGA |
| **SNP23** | **CYP1A1** | **T3801C** |
| SEQ ID NO: | 89 | TCCACCTCCTGGGCTCACA |
| SEQ ID NO: | 90 | TCCACCTCCCGGGCTCACA |
| SEQ ID NO: | 91 | TCCACCTCCTGGGCTCACA |
| SEQ ID NO: | 92 | TCCACCTCCCGGGCTCACA |
| **SNP24** | **CYP1B1** | **Leu432Val** |
| SEQ ID NO: | 93 | AATCATGACCCACTGAAGTGGCCTA |
| SEQ ID NO: | 94 | TAGGCCACTTCAGTGGGTCATGATT |
| SEQ ID NO: | 95 | AATCATGACCCAGTGAAGTGGCCTA |
| SEQ ID NO: | 96 | TAGGCCACTTCACTGGGTCATGATT |
| **SNP25** | **CYP1B1** | **Allele*4 (Asn453Ser)** |
| SEQ ID NO: | 97 | CGGCCTCATCAACAAGGACCTGA |
| SEQ ID NO: | 98 | TCAGGTCCTTGTTGATGAGGCCG |
| SEQ ID NO: | 99 | CGGCCTCATCAGCAAGGACCTGA |
| SEQ ID NO: | 100 | TCAGGTCCTTGCTGATGAGGCCG |

(continued)

| | | |
|---|---|---|
| **SNP26** | **CYP2C9** | **Arg144Cys (allele*2)** |
| SEQ ID NO: | 101 | GCATTGAGGACCGTGTTCAAGAG |
| SEQ ID NO: | 102 | CTCTTGAACACGGTCCTCAATGC |
| SEQ ID NO: | 103 | GCATTGAGGACTGTGTTCAAGAG |
| SEQ ID NO: | 104 | CTCTTGAACACAGTCCTCAATGC |
| **SNP27** | **CYP2C9** | **Ile359Leu (allele*3)** |
| SEQ ID NO: | 105 | TCCAGAGATACATTGACCTTCTC |
| SEQ ID NO: | 106 | GAGAAGGTCAATGTATCTCTGGA |
| SEQ ID NO: | 107 | TCCAGAGATACCTTGACCTTCTC |
| SEQ ID NO: | 108 | GAGAAGGTCAAGGTATCTCTGGA |
| **SNP28** | **CYP2C19** | **681 G>A (Pro227Pro) (allele*2)** |
| SEQ ID NO: | 109 | GATTATTTCCCGGGAACCCATAA |
| SEQ ID NO: | 110 | ATTATTTCCCGGGAACCCATA |
| SEQ ID NO: | 111 | GATTATTTCCCAGGAACCCATAA |
| SEQ ID NO: | 112 | ATTATTTCCCAGGAACCCATA |
| **SNP29** | **CYP2D6** | **2549 A>del (allele*3)** |
| SEQ ID NO: | 113 | CCAGGTCATCCTGTGCTCAGTTA |
| SEQ ID NO: | 114 | CAGGTCATCCTGTGCTCAGTT |
| SEQ ID NO: | 115 | CCAGGTCATCCGTGCTCAGTTAG |
| SEQ ID NO: | 116 | CAGGTCATCCGTGCTCAGTTA |
| **SNP30** | **CYP2D6** | **1847 G>A (allele*4)** |
| SEQ ID NO: | 117 | CCCACCCCCAGGACGCCCCTT |
| SEQ ID NO: | 118 | CCACCCCCAGGACGCCCCT |
| SEQ ID NO: | 119 | CCCACCCCCAAGACGCCCCTT |
| SEQ ID NO: | 120 | CCACCCCCAAGACGCCCCT |
| **SNP31** | **CYP2D6** | **1707 del>T (allele*6)** |
| SEQ ID NO: | 121 | GCTGGAGCAGTGGGTGACCGA |
| SEQ ID NO: | 122 | CTGGAGCAGTGGGTGACCG |
| SEQ ID NO: | 123 | CGCTGGAGCAGGGGTGACCGA |
| SEQ ID NO: | 124 | GCTGGAGCAGGGGTGACCG |
| **SNP32** | **ELAC2** | **Ala541Thr** |
| SEQ ID NO: | 125 | GCACCCTGGCTGCTGTGTTTGTG |
| SEQ ID NO: | 126 | CACAAACACAGCAGCCAGGGTGC |
| SEQ ID NO: | 127 | GCACCCTGGCTACTGTGTTTGTG |
| SEQ ID NO: | 128 | CACAAACACAGTAGCCAGGGTGC |
| **SNP33** | **ESR1** | **IVS1 -397 T>C (PvuII) p>P** |
| SEQ ID NO: | 129 | AATGTCCCAGCTGTTTTATGCTT |
| SEQ ID NO: | 130 | ATGTCCCAGCTGTTTTATGCT |
| SEQ ID NO: | 131 | AATGTCCCAGCCGTTTTATGCTT |
| SEQ ID NO: | 132 | ATGTCCCAGCCGTTTTATGCT |
| **SNP34** | **F13A1** | **Val34Leu** |
| SEQ ID NO: | 133 | AGCTTCAGGGCGTGGTGCCCCGG |
| SEQ ID NO: | 134 | GCTTCAGGGCGTGGTGCCCCG |
| SEQ ID NO: | 135 | AGCTTCAGGGCTTGGTGCCCCGG |
| SEQ ID NO: | 136 | GCTTCAGGGCTTGGTGCCCCG |
| **SNP35** | **FGB** | **-455 G>A** |
| SEQ ID NO: | 137 | TTGATTTTAATGGCCCCTTTTGA |
| SEQ ID NO: | 138 | TCAAAAGGGGCCATTAAAATCAA |
| SEQ ID NO: | 139 | TTGATTTTAATAGCCCCTTTTGA |
| SEQ ID NO: | 140 | TCAAAAGGGGCTATTAAAATCAA |

(continued)

| | | |
|---|---|---|
| **SNP36** | **FII** | **20210 G>A** |
| SEQ ID NO: | 141 | TGACTCTCAGCGAGCCTCAATGC |
| SEQ ID NO: | 142 | GCATTGAGGCTCGCTGAGAGTCA |
| SEQ ID NO: | 143 | TGACTCTCAGCAAGCCTCAATGC |
| SEQ ID NO: | 144 | GCATTGAGGCTTGCTGAGAGTCA |
| **SNP37 FV Leiden Arg506Gln** | | |
| SEQ ID NO: | 145 | CCTGGACAGGCGAGGAATACAGG |
| SEQ ID NO: | 146 | CCTGTATTCCTCGCCTGTCCAGG |
| SEQ ID NO: | 147 | CCTGGACAGGCAAGGAATACAGG |
| SEQ ID NO: | 148 | CCTGTATTCCTTGCCTGTCCAGG |
| **SNP38** | **GJA4** | **Pro319Ser** |
| SEQ ID NO: | 149 | ATGGCCAAAAACCCCCAAGTCGT |
| SEQ ID NO: | 150 | ACGACTTGGGGGTTTTTGGCCAT |
| SEQ ID NO: | 151 | ATGGCCAAAAATCCCCAAGTCGT |
| SEQ ID NO: | 152 | ACGACTTGGGGATTTTTGGCCAT |
| **SNP39** | **GNAS** | **393 T>C (Ile131Ile)** |
| SEQ ID NO: | 153 | GTGGACTACATTCTGAGTGTGAT |
| SEQ ID NO: | 154 | ATCACACTCAGAATGTAGTCCAC |
| SEQ ID NO: | 155 | GTGGACTACATCCTGAGTGTGAT |
| SEQ ID NO: | 156 | ATCACACTCAGGATGTAGTCCAC |
| **SNP40** | **GNB3** | **825 C>T (Ser275Ser)** |
| SEQ ID NO: | 157 | GGCATCACGTCCGTGGCCTTCTC |
| SEQ ID NO: | 158 | GAGAAGGCCACGGACGTGATGCC |
| SEQ ID NO: | 159 | GGCATCACGTCTGTGGCCTTCTC |
| SEQ ID NO: | 160 | GAGAAGGCCACAGACGTGATGCC |
| **SNP41** | **GSTM1** | **present>null** |
| SEQ ID NO: | 161 | CACATATTCTTGGCCTTCTGCAGAT |
| SEQ ID NO: | 162 | ATCTGCAGAAGGCCAAGAATATGTG |
| SEQ ID NO: | 163 | CACATATTCTTGACCTTCTGCAGAT |
| SEQ ID NO: | 164 | ATCTGCAGAAGGTCAAGAATATGTG |
| **SNP42** | **GSTP1** | **Ile105Val** |
| SEQ ID NO: | 165 | GCTGCAAATACATCTCCCTCATC |
| SEQ ID NO: | 166 | GATGAGGGAGATGTATTTGCAGC |
| SEQ ID NO: | 167 | GCTGCAAATACGTCTCCCTCATC |
| SEQ ID NO: | 168 | GATGAGGGAGACGTATTTGCAGC |
| **SNP43** | **GSTP1** | **Ala114Val** |
| SEQ ID NO: | 169 | CTGGCAGGAGGCGGGCAAGGATG |
| SEQ ID NO: | 170 | ATCCTTGCCCGCCTCCTGCCA |
| SEQ ID NO: | 171 | CTGGCAGGAGGTGGGCAAGGATG |
| SEQ ID NO: | 172 | ATCCTTGCCCACCTCCTGCCA |
| **SNP44** | **GSTT1** | **present>null** |
| SEQ ID NO: | 173 | CTGCCTAGTGGGTTCACCTGCCCAC |
| SEQ ID NO: | 174 | GTGGGCAGGTGAACCCACTAGGCAG |
| SEQ ID NO: | 175 | CTGCCTAGTGGGGTCACCTGCCCAC |
| SEQ ID NO: | 176 | GTGGGCAGGTGACCCCACTAGGCAG |
| **SNP45** | **IL6** | **-174 C>G** |
| SEQ ID NO: | 177 | TTGTGTCTTGCGATGCTAAAGGA |
| SEQ ID NO: | 178 | TCCTTTAGCATCGCAAGACACAA |
| SEQ ID NO: | 179 | TTGTGTCTTGCCATGCTAAAGGA |
| SEQ ID NO: | 180 | TCCTTTAGCATGGCAAGACACAA |

(continued)

| | | |
|---|---|---|
| **SNP46** | **IL10** | **-1082 G>A** |
| SEQ ID NO: | 181 | CTTCTTTGGGAAGGGGAAGTAGG |
| SEQ ID NO: | 182 | CCTACTTCCCCTTCCCAAAGAAG |
| SEQ ID NO: | 183 | CTTCTTTGGGAGGGGGAAGTAGG |
| SEQ ID NO: | 184 | CCTACTTCCCCCTCCCAAAGAAG |
| **SNP47** | **ITGB3** | **Leu33Pro** |
| SEQ ID NO: | 185 | GCCCTGCCTCTGGGCTCACCT |
| SEQ ID NO: | 186 | GAGGTGAGCCCAGAGGCAGGGCC |
| SEQ ID NO: | 187 | GCCCTGCCTCCGGGCTCACCT |
| SEQ ID NO: | 188 | GAGGTGAGCCCGGAGGCAGGGCC |
| **SNP48** | **MMP3** | **5A>6A** |
| SEQ ID NO: | 189 | ATGGGGGGAAAAAACCATGTCTT |
| SEQ ID NO: | 190 | GGGGAAAAAACCATGTCTTGTC |
| SEQ ID NO: | 191 | ATGGGGGGAAAAACCATGTCTTG |
| SEQ ID NO: | 192 | GGGGAAAAACCATGTCTTGTCC |
| **SNP49** | **MTHFR** | **Ala222Val** |
| SEQ ID NO: | 193 | TCTGCGGGAGCCGATTTCATC |
| SEQ ID NO: | 194 | TGATGAAATCGGCTCCCGCAGAC |
| SEQ ID NO: | 195 | TCTGCGGGAGTCGATTTCATC |
| SEQ ID NO: | 196 | TGATGAAATCGACTCCCGCAGAC |
| **SNP50** | **NAT2** | **R64Q** |
| SEQ ID NO: | 197 | ACCACCCACCCCGGTTTCTTCTT |
| SEQ ID NO: | 198 | CCACCCACCCCGGTTTCTTCT |
| SEQ ID NO: | 199 | ACCACCCACCCTGGTTTCTTCTT |
| SEQ ID NO: | 200 | CCACCCACCCTGGTTTCTTCT |
| **SNP51** | **NAT2** | **282 C>T (Y94Y)** |
| SEQ ID NO: | 201 | AGGGTATTTTTACATCCCTCCAGTT |
| SEQ ID NO: | 202 | GGGTATTTTTACATCCCTCCAGT |
| SEQ ID NO: | 203 | AGGGTATTTTTATATCCCTCCAGTT |
| SEQ ID NO: | 204 | GGGTATTTTTATATCCCTCCAGT |
| **SNP52** | **NAT2** | **I114T** |
| SEQ ID NO: | 205 | GCAGGTGACCATTGACGGCAGGA |
| SEQ ID NO: | 206 | CAGGTGACCATTGACGGCAGG |
| SEQ ID NO: | 207 | GCAGGTGACCACTGACGGCAGGA |
| SEQ ID NO: | 208 | CAGGTGACCACTGACGGCAGG |
| **SNP53** | **NAT2** | **481C>T (L161L)** |
| SEQ ID NO: | 209 | GGAATCTGGTACCTGGACCAAATCA |
| SEQ ID NO: | 210 | AGGAATCTGGTACCTGGACCAAATCAG |
| SEQ ID NO: | 211 | GGAATCTGGTACTTGGACCAAATCA |
| SEQ ID NO: | 212 | AGGAATCTGGTACTTGGACCAAATCAG |
| **SNP54** | **NAT2** | **R197Q** |
| SEQ ID NO: | 213 | CGCTTGAACCTCGAACAATTGAAGA |
| SEQ ID NO: | 214 | GCTTGAACCTCGAACAATTGAAG |
| SEQ ID NO: | 215 | CGCTTGAACCTCAAACAATTGAAGA |
| SEQ ID NO: | 216 | GCTTGAACCTCAAACAATTGAAG |
| **SNP55** | **NAT2** | **K268R** |
| SEQ ID NO: | 217 | AAGAAGTGCTGAAAAATATATTTAA |
| SEQ ID NO: | 218 | TTAAATATATTTTTCAGCACTTCTT |
| SEQ ID NO: | 219 | AAGAAGTGCTGAGAAATATATTTAA |
| SEQ ID NO: | 220 | TTAAATATATTTCTCAGCACTTCTT |

(continued)

| | | |
|---|---|---|
| **SNP56** | **NAT2** | **G286E** |
| SEQ ID NO: | 221 | AACCTGGTGATGGATCCCTTACTAT |
| SEQ ID NO: | 222 | ACCTGGTGATGGATCCCTTACTA |
| SEQ ID NO: | 223 | AACCTGGTGATGAATCCCTTACTAT |
| SEQ ID NO: | 224 | ACCTGGTGATGAATCCCTTACTA |
| **SNP57** | **NOS3** | **-786 T>C** |
| SEQ ID NO: | 225 | TCTTCCCTGGCTGGCTGACCCTG |
| SEQ ID NO: | 226 | CAGGGTCAGCCAGCCAGGGAAGA |
| SEQ ID NO: | 227 | TCTTCCCTGGCCGGCTGACCCTG |
| SEQ ID NO: | 228 | CAGGGTCAGCCGGCCAGGGAAGA |
| **SNP58** | **NOS3** | **Glu298Asp** |
| SEQ ID NO: | 229 | GCCCCAGATGAGCCCCCAGAACT |
| SEQ ID NO: | 230 | AGTTCTGGGGGCTCATCTGGGGC |
| SEQ ID NO: | 231 | GCCCCAGATGATCCCCCAGAACT |
| SEQ ID NO: | 232 | AGTTCTGGGGGATCATCTGGGGC |
| **SNP59** | **NPY** | **Leu7Pro** |
| SEQ ID NO: | 233 | CGGACAGCCCCAGTCGCTTGTTA |
| SEQ ID NO: | 234 | TAACAAGCGACTGGGGCTGTCCG |
| SEQ ID NO: | 235 | CGGACAGCCCCGGTCGCTTGTTA |
| SEQ ID NO: | 236 | TAACAAGCGACCGGGGCTGTCCG |
| **SNP60** | **OGG1** | **Cys326Ser** |
| SEQ ID NO: | 237 | CCTGCGCCAATCCCGCCATGCTC |
| SEQ ID NO: | 238 | CTGCGCCAATCCCGCCATGCT |
| SEQ ID NO: | 239 | CCTGCGCCAATGCCGCCATGCTC |
| SEQ ID NO: | 240 | CTGCGCCAATGCCGCCATGCT |
| **SNP61** | **PAI1** | **4G>5G** |
| SEQ ID NO: | 241 | CTGACTCCCCCACGTGT |
| SEQ ID NO: | 242 | CTGACTCCCCACGTGTC |
| SEQ ID NO: | 243 | CTGACTCCCCCACGTGT |
| SEQ ID NO: | 244 | CTGACTCCCCACGTGTC |
| **SNP62** | **PGR** | **331 G>A** |
| SEQ ID NO: | 245 | CGGGAGATAAAAGAGCCGCGTGT |
| SEQ ID NO: | 246 | ACACGCGGCTCTTTTATCTCCCG |
| SEQ ID NO: | 247 | CGGGAGATAAAGGAGCCGCGTGT |
| SEQ ID NO: | 248 | ACACGCGGCTCCTTTATCTCCCG |
| **SNP63** | **PON1** | **Gln192Arg** |
| SEQ ID NO: | 249 | CCCCTACTTACAATCCTGGGAGA |
| SEQ ID NO: | 250 | TCTCCCAGGATTGTAAGTAGGGG |
| SEQ ID NO: | 251 | CCCCTACTTACGATCCTGGGAGA |
| SEQ ID NO: | 252 | TCTCCCAGGATCGTAAGTAGGGG |
| **SNP64** | **SOD2** | **Ala16Val** |
| SEQ ID NO: | 253 | GATACCCCAAAGCCGGAGCCAGC |
| SEQ ID NO: | 254 | ATACCCCAAAGCCGGAGCCAG |
| SEQ ID NO: | 255 | GATACCCCAAAACCGGAGCCAGC |
| SEQ ID NO: | 256 | ATACCCCAAAACCGGAGCCAG |
| **SNP65** | **SRD5A2** | **Ala49Thr** |
| SEQ ID NO: | 257 | CCCGCCTGCCAGCCCGCGCCGCC |
| SEQ ID NO: | 258 | CCGCCTGCCAGCCCGCGCCGC |
| SEQ ID NO: | 259 | CCCGCCTGCCAACCCGCGCCGCC |
| SEQ ID NO: | 260 | CCGCCTGCCAACCCGCGCCGC |

(continued)

| | | |
|---|---|---|
| **SNP66** | **SRD5A2** | **Val89Leu** |
| SEQ ID NO: | 261 | CCTCTTCTGCGTACATTACTT |
| SEQ ID NO: | 262 | CTCTTCTGCGTACATTACT |
| SEQ ID NO: | 263 | CCTCTTCTGCCTACATTACTT |
| SEQ ID NO: | 264 | CTCTTCTGCCTACATTACT |
| **SNP67** | **SREBF2** | **Gly595Ala** |
| SEQ ID NO: | 265 | GCTGCTGCCGGCAACCTACAA |
| SEQ ID NO: | 266 | TTGTAGGTTGCCGGCAGCAGC |
| SEQ ID NO: | 267 | GCTGCTGCCGCCAACCTACAA |
| SEQ ID NO: | 268 | TTGTAGGTTGGCGGCAGCAGC |
| **SNP68** | **SULT1A1** | **Arg213His** |
| SEQ ID NO: | 269 | TTTGTGGGGCGCTCCCTGCCA |
| SEQ ID NO: | 270 | TTGTGGGGCGCTCCCTGCC |
| SEQ ID NO: | 271 | TTTGTGGGGCACTCCCTGCCA |
| SEQ ID NO: | 272 | TTGTGGGGCACTCCCTGCC |
| **SNP69** | **VDR** | **b>B** |
| SEQ ID NO: | 273 | GACAGGCCTGCGCATTCCCAATA |
| SEQ ID NO: | 274 | TATTGGGAATGCGCAGGCCTGTC |
| SEQ ID NO: | 275 | GACAGGCCTGCACATTCCCAATA |
| SEQ ID NO: | 276 | TATTGGGAATGTGCAGGCCTGTC |

1.2 Production of the DNA-chip for the genotyping of gene variants associated with pathologies associated with aging: Printing and processing of the glass slides

The probes capable of detecting the different previously identified gene variants are printed in the amino-silanized support (glass slide) using DMSO as a printing buffer. The printing is carried out with a spotter or oligonucleotide (probes) printer controlling the temperature and the relative humidity.

The binding of the probes to the support (glass slide) is carried out by means of crosslinking with ultraviolet radiation and baking as described in the documentation provided by the manufacturer (for example, Corning Lifesciences http://www.corning.com). The relative humidity during the deposition process is maintained between 40-50% and the temperature around 20°C.

1.3 Validation of the clinical usefulness of the DNA-chip for the identification of gene variants associated with pathologies associated with aging: Simultaneous, sensitive, specific and reproducible detection of human gene variants associated with pathologies associated with aging

1.3.1 Preparation of the sample to be hybridized

DNA of the individual is extracted from a biological sample (for example, peripheral blood, saliva, etc) by means of a filtration protocol (for example, commercial kits by Macherey Nagel, Qiagen, etc).

All the exons and introns of interest are amplified by means of multiplex amplification using the suitable oligonucleotide primer pairs. Virtually any oligonucleotide primer pair can be used which allows the specific amplification of gene fragments in which the genetic variation to be detected exists, advantageously, those pairs which allow said amplification in the least possible number of amplification reactions; particularly oligonucleotide primers were selected which allow amplifying in only 5 multiplex amplification reactions the fragments necessary for the genotyping of the aforementioned 69 gene variants analyzed using the DNA-chip of the invention for the detection of gene variants associated with pathologies associated with aging.

The oligonucleotide primers used to carry out multiplex amplification for the detection of gene variants associated with pathologies associated with aging can be designed using the sequences of the corresponding genes as described in GenBank using, for example, the softwares:

**Primer** 3 (http://frodo.wi.mit.edu/cgi-bin/primer3/primer3 www.cgi) or
Web Primer (http://seq.yeastgenome.org/cgi-bin/web-primer)

The oligonucleotide primers used to amplify the corresponding gene variants associated with pathologies associated with aging by means of multiplex amplification are mentioned below.

**Oligonucleotide primers used**

| | | |
|---|---|---|
| **SNP01** | **ACE** | **Intron 16 ins/del** |
| SEQ ID NO: | 277 | GGGACTCTGTAAGCCACTGC |
| SEQ ID NO: | 278 | CCATGCCCATAACAGGTCTT |
| **SNP02** | **ADRB1** | **Gly389Arg** |
| SEQ ID NO: | 279 | GGCCTTCAACCCCATCATCTA |
| SEQ ID NO: | 280 | CCGGTCTCCGTGGGTCGCGT |
| **SNP03** | **ADRB2** | **Gln27Glu** |
| SEQ ID NO: | 281 | GCTCACCTGCCAGACTGC |
| SEQ ID NO: | 282 | GCCAGGACGATGAGAGACAT |
| **SNP04** | **ADRB2** | **Gly16Arg** |
| SEQ ID NO: | 283 | GCTCACCTGCCAGACTGC |
| SEQ ID NO: | 284 | GCCAGGACGATGAGAGACAT |
| **SNP05** | **ADRB3** | **Trp64Arg** |
| SEQ ID NO: | 285 | CAATACCGCCAACACCAGT |
| SEQ ID NO: | 286 | CGAAGTCACGAACACGTTG |
| **SNP06** | **AGT** | **Met235Thr** |
| SEQ ID NO: | 287 | GAACTGGATGTTGCTGCTGA |
| SEQ ID NO: | 288 | TTGCCTTACCTTGGAAGTGG |
| **SNP07** | **AGTR1** | **1166 A>C** |
| SEQ ID NO: | 289 | CCGCCCCTCAGATAATGTAA |
| SEQ ID NO: | 290 | GCAAAATGTGGCTTTGCTTT |
| **SNP08** | **APOA1** | **-75 G>A** |
| SEQ ID NO: | 291 | CACCTCCTTCTCGCAGTCTC |
| SEQ ID NO: | 292 | GGGACAGAGCTGATCCTTGA |
| **SNP09** | **APOB** | **Arg3480Trp** |
| SEQ ID NO: | 293 | AGCCTCACCTCTTACTTTTCCATTGAGTC |
| SEQ ID NO: | 294 | CGTTGGTGAAAAAGAGGCCCTCTA |
| **SNP10** | **APOB** | **Arg3500Gln** |
| SEQ ID NO: | 295 | AGCCTCACCTCTTACTTTTCCATTGAGTC |
| SEQ ID NO: | 296 | CGTTGGTGAAAAAGAGGCCCTCTA |
| **SNP11** | **APOB** | **Arg3531Cys** |
| SEQ ID NO: | 297 | AGCCTCACCTCTTACTTTTCCATTGAGTC |
| SEQ ID NO: | 298 | CGTTGGTGAAAAAGAGGCCCTCTA |
| **SNP12** | **APOE** | **Cys112Arg** |
| SEQ ID NO: | 299 | CTGTCCAAGGAGCTGCAG |
| SEQ ID NO: | 300 | CTGTTCCACCAGGGGCCC |
| **SNP13** | **APOE** | **Arg158Cys** |
| SEQ ID NO: | 301 | CTGTCCAAGGAGCTGCAG |
| SEQ ID NO: | 302 | CTGTTCCACCAGGGGCCC |
| **SNP14** | **CBS** | **833 T>C** |
| SEQ ID NO: | 303 | GCTTTTGCTGGCCTTGAG |
| SEQ ID NO: | 304 | GGGTGAGTTACAGGCTGCAC |
| **SNP15** | **CBS** | **844ins68** |
| SEQ ID NO: | 305 | GCTTTTGCTGGCCTTGAG |
| SEQ ID NO: | 306 | GGGTGAGTTACAGGCTGCAC |

(continued)

| | | |
|---|---|---|
| **SNP16** | **CETP** | **TaqIB B1>B2** |
| SEQ ID NO: | 307 | GCAAACAGCCAGGTATAGGG |
| SEQ ID NO: | 307 | AAGAGACTGAGGCCCAGAGA |
| **SNP17** | **CETP** | **Arg451Gln** |
| SEQ ID NO: | 309 | AGCCCTCATGAACAGCAAAG |
| SEQ ID NO: | 310 | AATCCTGTCTGGGCCTCTCT |
| **SNP18** | **COL1A1** | **1546 G>T** |
| SEQ ID NO: | 311 | AGCCGCTCCCATTCTCTTAG |
| SEQ ID NO: | 312 | GCGTGGTAGAGACAGGAGGA |
| **SNP19** | **COMT** | **Va1158Met (Allele*2)** |
| SEQ ID NO: | 313 | GGGCCTACTGTGGCTACTCA |
| SEQ ID NO: | 314 | CCCTTTTTCCAGGTCTGACA |
| **SNP20** | **CYP17A1 -34 A>G** | |
| SEQ ID NO: | 315 | GGGCTCCAGGAGAATCTTTC |
| SEQ ID NO: | 316 | AGGGTAAGCAGCAAGAGAGC |
| **SNP21** | **CYP19A1** | **1558 C>T** |
| SEQ ID NO: | 317 | CCTTGCACCCAGATGAGACT |
| SEQ ID NO: | 318 | GGCAAGGATGGATGATTTGT |
| **SNP22** | **CYP1A1** | **Ile462Val** |
| SEQ ID NO: | 319 | TGATGGTGCTATCGACAAGG |
| SEQ ID NO: | 320 | TTTGGAAGTGCTCACAGCAG |
| **SNP23** | **CYP1A1** | **T3801C** |
| SEQ ID NO: | 321 | CCGCTGCACTTAAGCAGTCT |
| SEQ ID NO: | 322 | GGCCCCAACTACTCAGAGG |
| **SNP24** | **CYP1B1** | **Leu432Val** |
| SEQ ID NO: | 323 | ACCTCTGTCTTGGGCTACCA |
| SEQ ID NO: | 324 | GCCAGGATGGAGATGAAGAG |
| **SNP25** | **CYP1B1** | **Allele*4 (Asn453Ser)** |
| SEQ ID NO: | 325 | ACCTCTGTCTTGGGCTACCA |
| SEQ ID NO: | 326 | GCCAGGATGGAGATGAAGAG |
| **SNP26** | **CYP2C9** | **Arg144Cys (allele*2)** |
| SEQ ID NO: | 327 | CCTGGGATCTCCCTCCTAGT |
| SEQ ID NO: | 328 | CCACCCTTGGTTTTTCTCAA |
| **SNP27** | **CYP2C9** | **Ile359Leu (allele*3)** |
| SEQ ID NO: | 329 | CCACATGCCCTACACAGATG |
| SEQ ID NO: | 330 | TCGAAAACATGGAGTTGCAG |
| **SNP28** | **CYP2C19** | **681 G>A (Pro227Pro) (allele*2)** |
| SEQ ID NO: | 331 | CAACCAGAGCTTGGCATATTG |
| SEQ ID NO: | 332 | TAAAGTCCCGAGGGTTGTTG |
| **SNP29** | **CYP2D6** | **2549 A>del (allele*3)** |
| SEQ ID NO: | 333 | GGGCCTGAGACTTGTCCAGG |
| SEQ ID NO: | 334 | GCCGAGAGCATACTCGGGAC |
| **SNP30** | **CYP2D6** | **1847 G>A (allele*4)** |
| SEQ ID NO: | 335 | CCACGCGCACGTGCCCGTCCCA |
| SEQ ID NO: | 336 | CCTGCAGAGACTCCTCGGTCTCTC |
| **SNP31** | **CYP2D6** | **1707 del>T (allele*6)** |
| SEQ ID NO: | 337 | CCACGCGCACGTGCCCGTCCCA |
| SEQ ID NO: | 338 | CCTGCAGAGACTCCTCGGTCTCTC |
| **SNP32** | **ELAC2** | **Ala541Thr** |
| SEQ ID NO: | 339 | CCGACACGTCTCTGCTACTG |
| SEQ ID NO: | 340 | AACAAAAGCTCTGGGCAAGT |

(continued)

| | | |
|---|---|---|
| **SNP33** | **ESR1** | **IVS1 -397 T>C (PvuII) p>P** |
| SEQ ID NO: | 341 | AGGGTTATGTGGCAATGACG |
| SEQ ID NO: | 342 | ACCAATGCTCATCCCAACTC |
| **SNP34** | **F13A1** | **Val34Leu** |
| SEQ ID NO: | 343 | CATGCCTTTTCTGTTGTCTTCTT |
| SEQ ID NO: | 344 | CCCAGTGGAGACAGAGGATG |
| **SNP35** | **FGB -455 G>A** | |
| SEQ ID NO: | 345 | GGGTCTTTCTGATGTGTATTTTTCA |
| SEQ ID NO: | 346 | GACCTACTCACAAGGCAACCA |
| **SNP36 FII 20210 G>A** | | |
| SEQ ID NO: | 347 | GAGAGTAGGGGGCCACTCAT |
| SEQ ID NO: | 348 | CCTGAGCCCAGAGAGCTG |
| **SNP37** | **FV Leiden Arg506Gln** | |
| SEQ ID NO: | 349 | GCCCAGTGCTTAACAAGACC |
| SEQ ID NO: | 350 | CCCATTATTTAGCCAGGAGACC |
| **SNP38** | **GJA4** | **Pro319Ser** |
| SEQ ID NO: | 351 | CCTCCTCAGACCCTTACACG |
| SEQ ID NO: | 352 | GCAGCCAGACTTCTCAGGAC |
| **SNP39** | **GNAS** | **393 T>C (Ile131Ile)** |
| SEQ ID NO: | 353 | AGTACGTGCTGGCTCCTTGT |
| SEQ ID NO: | 354 | CACAAGTCGGGGTGTAGCTT |
| **SNP40** | **GNB3** | **825 C>T (Ser275Ser)** |
| SEQ ID NO: | 355 | CTGCCGCTTGTTTGACCT |
| SEQ ID NO: | 356 | CACACGCTCAGACTTCATGG |
| **SNP41** | **GSTM1** | **present>null** |
| SEQ ID NO: | 357 | TGCTTCACGTGTTATGGAGGT |
| SEQ ID NO: | 358 | GGGCTCAAATATACGGTGGA |
| **SNP42** | **GSTP1** | **Ile105Val** |
| SEQ ID NO: | 359 | CTCTATGGGAAGGACCAGCA |
| SEQ ID NO: | 360 | GAAGCCCCTTTCTTTGTTCA |
| **SNP43** | **GSTP1** | **Ala114Val** |
| SEQ ID NO: | 361 | GCAAGCAGAGGAGAATCTGG |
| SEQ ID NO: | 362 | CTCACCTGGTCTCCCACAAT |
| **SNP44** | **GSTT1** | **present>null** |
| SEQ ID NO: | 363 | GGCAGCATAAGCAGGACTTC |
| SEQ ID NO: | 364 | CTGCAGTTGCTCGAGGACAA |
| **SNP45** | **IL6** | **-174 C>G** |
| SEQ ID NO: | 365 | GCCTCAATGACGACCTAAGC |
| SEQ ID NO: | 366 | TCATGGGAAAATCCCACATT |
| **SNP46** | **IL10** | **-1082 G>A** |
| SEQ ID NO: | 367 | TCCCCAGGTAGAGCAACACT |
| SEQ ID NO: | 368 | ATGGAGGCTGGATAGGAGGT |
| **SNP47** | **ITGB3** | **Leu33Pro** |
| SEQ ID NO: | 369 | GCTCCAATGTACGGGGTAAA |
| SEQ ID NO: | 370 | ACTCACTGGGAACTCGATGG |
| **SNP48** | **MMP3** | **5A>6A** |
| SEQ ID NO: | 371 | TCACTGCCACCACTCTGTTC |
| SEQ ID NO: | 372 | GCCTCAACCTCTCAAAGTGC |
| **SNP49** | **MTHFR** | **Ala222Val** |
| SEQ ID NO: | 373 | GCCTCTCCTGACTGTCATCC |
| SEQ ID NO: | 374 | CAAAGCGGAAGAATGTGTCA |

(continued)

| | | |
|---|---|---|
| **SNP50** | **NAT2** | **R64Q** |
| SEQ ID NO: | 375 | CCATGGAGTTGGGCTTAGAG |
| SEQ ID NO: | 376 | GGCTGATCCTTCCCAGAAAT |
| **SNP51** | **NAT2** | **282 C>T (Y94Y)** |
| SEQ ID NO: | 377 | CCATGGAGTTGGGCTTAGAG |
| SEQ ID NO: | 378 | CCATGCCAGTGCTGTATTTG |
| **SNP52** | **NAT2** | **I114T** |
| SEQ ID NO: | 379 | CCATGGAGTTGGGCTTAGAG |
| SEQ ID NO: | 380 | CCATGCCAGTGCTGTATTTG |
| **SNP53** | **NAT2** | **481C>T (L161L)** |
| SEQ ID NO: | 381 | CAGGTGCCTTGCATTTTCT |
| SEQ ID NO: | 382 | GATGAAGCCCACCAAACAGT |
| **SNP54** | **NAT2** | **R197Q** |
| SEQ ID NO: | 383 | CAGGTGCCTTGCATTTTCT |
| SEQ ID NO: | 384 | GATGAAGCCCACCAAACAGT |
| **SNP55** | **NAT2** | **K268R** |
| SEQ ID NO: | 385 | AAAGACAATACAGATCTGGTCGAG |
| SEQ ID NO: | 386 | TCTTCAAAATAACGTGAGGGTAGA |
| **SNP56** | **NAT2** | **G286E** |
| SEQ ID NO: | 387 | AAAGACAATACAGATCTGGTCGAG |
| SEQ ID NO: | 388 | TCTTCAAAATAACGTGAGGGTAGA |
| **SNP57** | **NOS3** | **-786 T>C** |
| SEQ ID NO: | 389 | GTGTACCCCACCTGCATTCT |
| SEQ ID NO: | 390 | CCCACCCTGTCATTCAGTG |
| **SNP58** | **NOS3** | **Glu298Asp** |
| SEQ ID NO: | 391 | GAAGGCAGGAGACAGTGGAT |
| SEQ ID NO: | 392 | CAGTCAATCCCTTTGGTGCT |
| **SNP59** | **NPY** | **Leu7Pro** |
| SEQ ID NO: | 393 | CTCTGCCTGGTGATGAGGTT |
| SEQ ID NO: | 394 | GCAGAGGAGGGAGGTGCT |
| **SNP60** | **OGG1** | **Cys326Ser** |
| SEQ ID NO: | 395 | TAGTCTCACCAGCCCTGACC |
| SEQ ID NO: | 396 | TGGGGAATTTCTTTGTCCAG |
| **SNP61** | **PAI1** | **4G>5G** |
| SEQ ID NO: | 397 | CAACCTCAGCCAGACAAGGT |
| SEQ ID NO: | 398 | CAGCCACGTGATTGTCTAGG |
| **SNP62** | **PGR** | **331 G>A** |
| SEQ ID NO: | 399 | GCTTCACAGCATGCACGAGT |
| SEQ ID NO: | 400 | GAGGACTGGAGACGCAGAGT |
| **SNP63** | **PON1** | **Gln192Arg** |
| SEQ ID NO: | 401 | TATTGTTGCTGTGGGACCTG |
| SEQ ID NO: | 402 | CAAATCCTTCTGCCACCACT |
| **SNP64** | **SOD2** | **Ala16Val** |
| SEQ ID NO: | 403 | GGCTGTGCTTTCTCGTCTTC |
| SEQ ID NO: | 404 | CCGTAGTCGTAGGGCAGGT |
| **SNP65** | **SRD5A2 Ala49Thr** | |
| SEQ ID NO: | 405 | AGCACACGGAGAGCCTGA |
| SEQ ID NO: | 406 | AGGGGAAAAACGCTACCTGT |
| **SNP66** | **SRD5A2** | **Val89Leu** |
| SEQ ID NO: | 407 | AGCACACGGAGAGCCTGA |
| SEQ ID NO: | 408 | AGGGGAAAAACGCTACCTGT |

(continued)

| | | |
|---|---|---|
| **SNP67** | **SREBF2** | **Gly595Ala** |
| SEQ ID NO: | 409 | GGCCAGTGACCATTAACACC |
| SEQ ID NO: | 410 | TCTTCAAAGCCTGCCTCAGT |
| **SNP68** | **SULT1A1** | **Arg213His** |
| SEQ ID NO: | 411 | GTAATCCGAGCCTCCACTGA |
| SEQ ID NO: | 412 | GCTGTGGTCCATGAACTCCT |
| **SNP69** | **VDR** | **b>B** |
| SEQ ID NO: | 413 | CCTCACTGCCCTTAGCTCTG |
| SEQ ID NO: | 414 | CCCGCAAGAAACCTCAAATA |

The multiplex amplifications are carried out simultaneously under the same time and temperature conditions which allow the specific amplification of the gene fragments in which the gene variant to be detected may exist. Once the multiplex amplification has ended, it is verified in agarose gel that an amplification reaction has taken place.

Then, the sample to be hybridized (amplification product) is subjected to fragmentation with a DNAse and the products resulting from the fragmentation process are subjected to an indirect labeling reaction. A terminal transferase incorporates a nucleotide bound to a specific binding molecule, for example, biotin, at the end of these small fragments.

Before applying the sample on the DNA-chip, the sample is denatured by means of heating at 95°C for 5 minutes and the hybridization buffer, "ChipMap Kit Hybridization Buffer" (Ventana Medical System), is added.

1.3.2 Hybridization

Hybridization is carried out automatically in the Ventana Discovery hybridization station (Ventana Medical Systems).

Prehybridization or blocking of the slide with BSA is carried out. Then, the sample together with the hybridization solution [ChipMap Kit Hybridization Buffer (Ventana Medical System)] is applied and is maintained for 1 hour at 45°C following the Ventana 9.0 Europe protocol (Ventana Medical System). Finally, the slide is subjected to the action of different washing solutions [ChipMap hybridization Kit Buffers (Ventana Medical System)]. Once the hybridization process has ended, the final washing and drying of the slide is performed.

After hybridization has ended, development with streptavidin-Cy3 marks the points (probes) in which hybridization has taken place.

1.3.3. Scanning of the slide

The slide is introduced in the confocal fluorescence scanner, for example Axon 4100A scanner, and the signal emitted by the standard labeling upon being excited by a laser is scanned.

1.3.4 Quantification of the image

The software of the scanner itself allows quantification in the image obtained of the signal of the points in which hybridization has occurred.

1.3.5 Interpretation of the results

Determination of the genotype of the individual, with respect to the human gene variants associated with pathologies associated with aging.

The genotype of the individual is established from the signal which is obtained with the probes detecting the different gene variants. To that end, briefly, first the background noise of all the probes are subtracted from their absolute intensity values; then, the replicas corresponding to each of the 4 probes which are used to characterize each gene variant are grouped. The mean intensity value for each of the 4 probes is calculated using the bounded mean of the replicas to eliminate aberrant points. Once the mean intensity values for each of the probes are known, two ratios (ratio 1 and ratio 2) are calculated:

$$\text{Ratio 1} = \frac{\text{Mean intensity probe 1}}{\text{Mean intensity probe 1} + \text{Mean intensity probe 2}}$$

$$\text{Ratio 2} = \frac{\text{Mean intensity probe 3}}{\text{Mean intensity probe 3} + \text{Mean intensity probe 4}}$$

These ratios are substituted in three linear functions characterizing each of the three possible genotypes:

| | |
|---|---|
| AA | Function 1 |
| AB | Function 2 |
| BB | Function 3 |

The function having a higher absolute value determines the genotype that the patient has.

In this case, said linear functions are obtained by means of the analysis of 10 subjects for each of the three possible genotypes of the gene variant (AA, AB, BB). With the results, ratios 1 and 2 are calculated for the 30 subjects. These ratios serve as classification variables of the three groups to generate the linear functions. The classification capacity of the two probe pairs designed is evaluated with these three linear functions. In the event that the classification capacity is not 100%, the probes would be re-designed. New subjects characterized for each of the three genotypes form new ratios 1 and 2 in order to improve the linear combinations thereof which form the linear functions and, in summary, in order to improve the classification capacity of the algorithm based on these three functions.

Provided that ratios 1 and 2 are within the range of the ratios used to construct the groups, the mean fluorescence intensity of the 40 replicas with respect to the background noise is greater than 5 and the coefficient of variation of all the replicas of the DNA-chip is under 0.25 (using a confocal fluorescence scanner), the result of the linear functions is considered correct.

In summary, each mutation has in the slide 4 probes (repeated 10 times) for detection thereof. Two of said probes detect one gene variant and the other two detect the other gene variant.

In the case of a homozygous subject for gene variant A, said subject will not have gene variant B; accordingly, in the image obtained of the glass support the probes detecting gene variant B have a considerably inferior hybridization signal than that of gene variant A and vice versa; in this case, ratios 1 and 2 will tend to 1 and the subjects will be assigned as AA homozygotes.

In addition, a heterozygous subject for a certain gene variant has both gene variants; therefore, the probes that detect them have an equivalent hybridization signal. Ratios 1 and 2 will tend to 0.5 and the subjects will be assigned as AB heterozygotes.

1.3.6. Analysis of the results:

The slide was introduced in the scanner and the signal emitted by the standard labeling upon being excited by a laser was scanned (section 1.3.3) and the image obtained from the signal of the points in which hybridization has occurred quantified (section 1.3.4).

The analysis of the results was conducted using the functions described in section 1.3.5. After genotyping the 69 human gene variants described in the Table 1, said variants are grouped by particular genetic risks.

Therefore, for the determination of a particular genetic risk, first the results obtained corresponding to each particular genetic risk are grouped together. Thus in this step, the gene variants corresponding to each particular genetic risk studied are grouped together. Tables 2, 4, 6, 8, 10, 13, 15, 17 and 25 show (see column 1) the gene variants associated with each of the particular pathologies associated with aging that are studied.

Subsequently, each genotype associated with each gene variant is standardized or scored. In this sense, said values will be comprised in a range of standardized values, in which the genotype or genotypes of the highest risk of suffering from a certain pathology will comprise the value of the upper limit of said range of values, and the genotype or genotypes of the lowest risk of suffering from a certain pathology will comprise the value of the lower limit of said range of values. Thus, according to the genotype present in the sample analyzed, a corresponding standardized value is assigned to said genotype.

The particular genetic risk is then calculated according to equation [1] or [2] depending on whether said particular genetic risk is formed (or not) by a combination of partial particular risks.

When the particular genetic risk is not formed by a combination of partial particular risks, said particular genetic risk is calculated by means of the equation [1]:

$$\text{PGR} = \frac{\sum_{i=1}^{n} xi}{\sum_{i=1}^{n} Lsi} \qquad [1]$$

where
PGR represents the particular genetic risk to be calculated;
$x_i$ represents the standardized value of the genotype characterized for a gene variant in a sample, in relation to the particular genetic risk to be calculated;
$Ls_i$ represents the value of the upper limit of the range of standardized values assigned to each gene variant, in relation to the particular genetic risk to be calculated; and
n is the number of gene variants analyzed in relation to the particular genetic risk to be calculated.

When the particular genetic risk is formed by a combination of partial particular risks, said particular genetic risk is calculated by means of equation [2]:

$$\text{PGR} = \frac{\sum_{i=1}^{n} PPGRi}{no.PPGR} \qquad [2]$$

where
PGR represents the particular genetic risk to be calculated;
PPGRi represents the value calculated for each partial particular genetic risk which, in combination with other partial particular genetic risks, forms the particular genetic risk to be calculated, wherein said PPGRi is calculated by means of equation [3]:

$$\text{PPGRi} = \frac{\sum_{i=1}^{n} xi}{\sum_{i=1}^{n} Lsi} \qquad [3]$$

where
PPGRi has the previously mentioned meaning;
$x_i$ represents the standardized value of the genotype characterized for a gene variant in a sample, in relation to the partial particular genetic risk to be calculated;
$Ls_i$ represents the value of the upper limit of the range of standardized values assigned to each gene variant, in relation to the partial particular genetic risk to be calculated; and
n is the number of gene variants analyzed in relation to the partial particular genetic risk to be calculated; and
no.PPGR is the number of partial particular genetic risks analyzed in relation to the partial particular genetic risk to be calculated.

Once the particular genetic risks are calculated, the global genetic risk is determined by means of equation [4]:

$$\text{GGR} = \frac{\sum PGR}{n} \qquad [4]$$

where
GGR represents the global genetic risk to be calculated;
PGR represents the value calculated for each particular genetic risk analyzed in relation to the global genetic risk to be calculated, and is calculated by means of the previously described equations [1] or [2]; and
n is the number of particular genetic risks analyzed in relation to the global genetic risk to be calculated.

Merely by way of a non-limiting illustration in this example, the global genetic risk that the analyzed subject has of a pathology associated with aging comprises the determination of the following particular genetic risks:

1. Particular genetic risk associated with suffering from vascular disease (vascular risk) [Tables 2-12];
2. Particular genetic risk associated with osteoporosis (risk of osteoporosis) [Tables 13-14];
3. Particular genetic risk associated with carcinogenesis (carcinogenic risk) [Tables 15-16]; and
4. Particular genetic risk associated with environmental stress and oxidative damage [Tables 17-18].

Furthermore, the following partial particular genetic risks have been determined to determine the vascular risk:

- partial particular genetic risk associated with lipid metabolism [Tables 2-3];
- partial particular genetic risk associated with thrombosis [Tables 4-5];
- partial particular genetic risk associated with ictus [Tables 6-7];
- partial particular genetic risk associated with high blood pressure [Tables 8-9]; and
- partial particular genetic risk associated with endothelial vulnerability [Tables 10-11].

Table 2 shows an example of how the value of a partial particular genetic risk, lipid metabolism, has been determined in a sample of a subject. In this case, the partial particular genetic risk associated with lipid metabolism has been calculated according to 11 SNPs (SNP08, SNP09, SNP10, SNP11, SNP12, SNP13, SNP17, SNP16, SNP63, SNP67 and SNP59).

Table 12 shows the result of the calculation of the vascular genetic risk, which has been calculated according to partial particular genetic risks: lipid metabolism, thrombosis, ictus, high blood pressure and endothelial vulnerability.

Table 24 shows the result of the calculation of the global genetic risk of suffering from a pathology associated with aging as explained above from an exemplary sample of a subject according to the particular genetic risks: vascular risk, osteoporosis risk, carcinogenic risk and environmental stress risk.

The particular genetic risk of the subject under study associated with response to drugs, i.e., the particular genetic risk of suffering from adverse reactions to drugs, has additionally been determined in this example. Table 25 shows the result of the general response to drugs in relation to those drugs metabolized by the following pathways: NAT2, CYP2D6, CYP2C19 and CYP2C9.

**VASCULAR RISK**

| | LIPID METABOLISM | SCORE ACCORDING TO GENOTYPE | | | | | |
|---|---|---|---|---|---|---|---|
| SNP08 | APOA1 -75 G>**A** | G/G=0 | G/A=1 | A/A=2 | | | |
| SNP09 | APOB Arg3480**Trp** | Arg/Arg=0 | Arg/Trp=1 | Trp/Trp=2 | | | |
| SNP10 | APOB Arg3500**Gln** | Arg/Arg=0 | Arg/Gln=1 | Gln/Gln=2 | | | |
| SNP11 | APOB Arg3531**Cys** | Arg/Arg=0 | Arg/Cys=1 | Cys/Cys=2 | | | |
| SNP12-13* | APOE Alleles *2, *3, *4 Cys112Arg, Arg158Cys | E3/E3=0 | E3/E2=0 | E3/E4=1 | E2/E4=1 | E2/E2=2 | **E4/E4=13.5** |
| SNP17 | CETP Arg451**Gln** | Arg/Arg=0 | Arg/Gln=1 | Gln/Gln=2 | | | |
| SNP16 | CETP TaqIB **B1**/B2 | B2/B2=0 | B1/B2=1 | B1/B1=2 | | | |
| SNP63 | PON1 Gln192**Arg** | Gln/Gln=0 | Gln/Arg=0.5 | Arg/Arg=1 | | | |
| SNP67 | SREBF2 Gly595**Ala** | Gly/Gly=0 | Gly/Ala=1 | Ala/Ala=2 | | | |
| SNP59 | NPY Leu>**Pro** | Leu/Leu=0 | Leu/Pro=1 | Pro/Pro=2 | | | |

* See Table 20

**Table 2**

| Genotype | Score of the Genotype |
|---|---|
| **G/G** | 0 |
| **Arg/Arg** | 0 |
| **Arg/Arg** | 0 |
| **Arg/Arg** | 0 |
| **E3/E3** | 0 |
| **Arg/Arg** | 0 |
| **B1/B2** | 1 |
| **Gln/Gln** | 0 |
| **Gly/Ala** | 1 |
| **Leu/Leu** | 0 |
| **Sum** | 2 |
| **PPGR** | **2/19=0.11** |

The summation of the maximum score of the upper limits of the ranges of values is 19 points ($\sum_{i=1}^{n} Lsi = 19$).

The risk is calculated on a scale of 0-1 considering the maximum value of 19 as risk = 1.

**Table 3**

| | THROMBOSIS RISK | | SCORE ACCORDING TO GENOTYPE | |
|---|---|---|---|---|
| SNP61 | PAI1 **4G**>5G | 5G/5G=0 | 4G/5G=1 | 4G/4G=2 |
| SNP47 | ITGB3 Leu33**Pro** | Leu/Leu=0 | Leu/Pro=1 | Pro/Pro=2 |
| SNP36 | FII 20210 G>**A** | G/G=0 | G/A=1 | A/A=2 |
| SNP37 | FV Leiden Arg506**Gln** | Arg/Arg=0 | Arg/Gln=1 | Gln/Gln=2 |
| SNP34 | F13A1 **Val**34Leu | Leu/Leu=0 | Val/Leu=1 | Val/Val=2 |
| SNP49 | MTHFR Ala222**Val** | Ala/Ala=0 | Ala/Val=1 | Val/Val=2 |
| SNP14 | CBS 833 T>**C** | T/T=0 | T/C=1 | C/C=2 |
| SNP15 | CBS 844**ins68** | Del/Del=0 | Ins/Del=1 | Ins/Ins=2 |
| SNP35 | FGB –455 G>**A** | G/G=0 men, =0 women | G/A=1 men, =2 women | A/A=2 men, =4 women |

**Table 4**

The summation of the maximum score of the upper limits of the ranges of values is 20 points ($\sum_{i=1}^{n} Lsi = 20$).

The risk is calculated on a scale of 0-1 considering the maximum value of 20 as risk=1.

| Genotype | Score of the Genotype |
|---|---|
| **5G/4G** | 1 |
| **Leu/Pro** | 1 |
| **G/G** | 0 |
| **Arg/Arg** | 0 |
| **Val/Leu** | 1 |
| **Ala/Val** | 1 |
| **T/T** | 0 |
| **Del/Del** | 0 |
| **G/A** | 1 |
| **Sum** | 5 |
| **PPGR** | **5/20=0.25** |

**Table 5**

| | ICTUS RISK | | SCORE ACCORDING TO GENOTYPE | |
|---|---|---|---|---|
| SNP61 | PAI1 4G>**5G** | 4G/4G=0 | 4G/5G=1 | 5G/5G=2 |
| SNP47 | ITGB3 Leu33**Pro** | Leu/Leu=0 | Leu/Pro=1 | Pro/Pro=2 |
| SNP36 | FII 20210 G>**A** | G/G=0 | G/A=1 | A/A=2 |
| SNP37 | FV Leiden Arg506**Gln** | Arg/Arg=0 | Arg/Gln=1 | Gln/Gln=2 |
| SNP34 | F13A1 Val34**Leu** | Val/Val=0 | Val/Leu=1 | Leu/Leu=2 |

**Table 6**

The summation of the maximum score of the upper limits of the ranges of values is 30 points ($\sum_{i=1}^{n} Lsi = 30$).

The risk is calculated on a scale of 0-1 considering the maximum value of 30 as risk=1

| Genotype | Score of the Genotype |
|---|---|
| **5G/4G** | 1 |
| **Leu/Pro** | 1 |
| **G/G** | 0 |
| **Arg/Arg** | 0 |
| **Val/Leu** | 1 |
| **Sum** | 3 |
| **PPGR** | **3/10=0.30** |

**Table 7**

| | HIGH BLOOD PRESSURE RISK | SCORE ACCORDING TO GENOTYPE | | |
|---|---|---|---|---|
| SNP02 | ADRB1 Gly389**Arg** | Gly/Gly=0 | Gly/Arg=1 | Arg/Arg=2 |
| SNP04 | ADRB2 Gly16**Arg** | Gly/Gly=0 | Gly/Arg=1 | Arg/Arg=2 |
| SNP03 | ADRB2 **Gln**27Glu | Glu/Glu=0 (1 if genotype ADRB2 Gly16Arg= Gly/Arg or Arg/Arg) | Gln/Glu=1 (2 if genotype ADRB2 Gly16Arg= Gly/Arg or Arg/Arg) | Gln/Gln=2 |
| SNP06 | AGT Met235**Thr** | Met/Met=0 | Met/Thr=1 | Thr/Thr=2 |
| SNP07 | AGTR1 1166 A>**C** | A/A=0 | A/C=1 | C/C=2 |
| SNP39 | GNAS 393 T>**C** (Ile131Ile) | **T/T=2** | **T/C=1** | **C/C=0** |
| SNP40 | GNB3 825 C>**T** (Ser275Ser) | C/C=0 | C/T=1 | T/T=2 |
| SNP01 | ACE Intron 16 ins/**del** | ins/ins=0 | ins/del=1 | del/del=2 |
| SNP05 | ADRB3 Trp64**Arg** | Trp/Trp=0 | Trp/Arg=1 | Arg/Arg=2 |

**Table 8**

| Genotype | Score of the Genotype |
|---|---|
| **Arg/Arg** | 2 |
| **Gly/Arg** | 1 |
| **Gln/Gln** | 2 |
| **Thr/Thr** | 2 |
| **A/A** | 0 |
| **T/C** | 1 |
| **C/C** | 0 |
| **Del/Del** | 2 |
| **Trp/Arg** | 1 |
| **Sum** | 11 |
| **PPGR** | **11/18=0.61** |

**Table 9**

The summation of the maximum score of the upper limits of the ranges of values is 18 points ($\sum_{i=1}^{n} Lsi = 18$).

The risk is calculated on a scale of 0-1 considering the maximum value of 18 as risk=1

| | ENDOTHELIAL VULNERABILITY RISK | SCORE ACCORDING TO GENOTYPE | | |
|---|---|---|---|---|
| SNP48 | MMP3 **5A>6A** | 5A/5A=1 | 5A/6A=0 | 6A/6A=1 |
| SNP57 | NOS3 -786 T>**C** | T/T=0 | T/C=1 (2 if genotype NOS3 Glu298Asp: Glu/Asp or Asp/Asp) | C/C=2 (4 if genotype NOS3 Glu298Asp: Glu/Asp or Asp/Asp) |
| SNP58 | NOS3 Glu298**Asp** | Glu/Glu=0 | Glu/Asp=1 | Asp/Asp=2 |
| SNP49 | MTHFR Ala222**Val** | Ala/Ala=0 | Ala/Val=1 | Val/Val=2 |
| SNP14 | CBS 833 T>**C** | T/T=0 | T/C=1 | C/C=2 |
| SNP15 | CBS 844**ins68** | del/del=0 | ins/del=1 | ins/ins=2 |
| SNP38 | GJA4 Pro319**Ser** | Pro/Pro=0 | Pro/Ser=1 | Ser/Ser=2 |

**Table 10**

| Genotype | Score of the Genotype |
|---|---|
| 6A/6A | 1 |
| T/T | 0 |
| Glu/Glu | 0 |
| Ala/Val | 1 |
| T/T | 0 |
| Del/Del | 0 |
| Pro/Ser | 1 |
| Sum | 3 |
| **PPGR** | **3/15=0.20** |

**Table 11**

The summation of the maximum score of the upper limits of the ranges of values is 15 points ($\sum_{i=1}^{n} Lsi = 15$).

The risk is calculated on a scale of 0-1 considering the maximum value of 15 as risk=1

## VASCULAR RISK

| | | |
|---|---|---|
| **LIPID METABOLISM** | | 0.11 |
| **THROMBOSIS RISK** | | 0.25 |
| **ICTUS RISK** | | 0.30 |
| **HIGH BLOOD PRESSURE RISK** | | 0.61 |
| **ENDOTHELIAL VULNERABILITY RISK** | | 0.20 |
| | **PGR: VASCULAR RISK** | **0.29** |

**Table 12**

| | OSTEOPOROSIS RISK | SCORE ACCORDING TO GENOTYPE | | |
|---|---|---|---|---|
| SNP18* | COL1A1 1546 G>**T** | G/G=0 | G/T=1 | T/T=2 |
| SNP33 | ESR1 IVS1 -397 T>**C** p>**P** (PvuII) | p/p=0 | p/P=1 | P/P=2 |
| SNP69 | VDR b>**B** | b/b=0 | b/B=1 | B/B=2 |

* only this polymorphism is considered in men

**Table 13**

| Genotype | Score of the Genotype |
|---|---|
| **G/G** | 0 |
| **p/P** | 1 |
| **b/B** | 1 |
| **Sum** | 2 |
| **PGR** | **2/6=0.33** |

The summation of the maximum score of the upper limits of the ranges of values is 6 points in women and 2 in men($\sum_{i=1}^{n} Lsi$ =6 or 2).
The risk is calculated on a scale of 0-1 considering the maximum value of 6 or 2 as risk=1

**Table 14**

| | CARCINOGENIC RISK | | SCORE ACCORDING TO GENOTYPE | |
|---|---|---|---|---|
| SNP20* | CYP17A1 –34 A>**G** | A/A=0 | A/G=1 | G/G=2 |
| SNP23* | CYP1A1 3801 T>**C** | T/T=0 | T/C=1 | C/C=2 |
| SNP22* | CYP1A1 Ile462**Val** | Ile/Ile=0 | Ile/Val=1 | Val/Val=2 |
| SNP24* | CYP1B1 **Leu**432Val | Val/Val=2 | Val/Leu=1 | Leu/Leu=0 |
| SNP25* | CYP1B1 Allele*4 (Asn453**Ser**) | Asn/Asn=*1/*1=0 | Asn/Ser=*1/*4=1 | Ser/Ser=*4/*4=2 |
| SNP21* | CYP19A1 1558 C>**T** | C/C=0 | C/T=1 | T/T=2 |
| SNP19** | COMT Val158**Met** (Allele*2) | Val/Val=0 | Val/Met=1 | Met/Met=2 |
| SNP62** | PGR 331 G>**A** | G/G=0 | G/A=1 | A/A=2 |
| SNP33** | ESR1 IVS1 -397 T>**C** p>**P** (PvuII) | p/p=0 | p/P=1 | P/P=2 |
| SNP69** | VDR b>**B** | b/b=0 | b/B=1 | B/B=2 |
| SNP65*** | SRD5A2 Ala49**Thr** | Ala/Ala=0 | Ala/Thr=1 | Thr/Thr=2 |
| SNP66*** | SRD5A2 Val89**Leu** | Val/Val=0 | Val/Leu=1 | Leu/Leu=2 |
| SNP32*** | ELAC2 Ala541**Thr** | Ala/Ala=0 | Ala/Thr=12.8 | Thr/Thr=12.8 |

**Table 15**

*SNPs included in men and in women.

**SNPs included in women.

***SNPs included in men.

| Genotype | Score of the Genotype |
|---|---|
| **A/G** | 1 |
| **T/T** | 0 |
| **Ile/Ile** | 0 |
| **Leu/Val** | 1 |
| ***1/*1** | 0 |
| **C/T** | 1 |
| **Val/Met** | 1 |
| **G/G** | 0 |
| **p/P** | 1 |
| **b/B** | 1 |
| **Ala/Ala** | 0 |
| **Val/Leu** | 1 |
| **Ala/Ala** | 0 |
| **Sum** | 7 |
| **PGR** | **7/26=0.27** |

The summation of the maximum score of the upper limits of the ranges of values is 18 points in men and 20 in women ($\sum_{i=1}^{n} Lsi = 18$ or 20).

The risk is calculated on a scale of 0-1 considering the maximum value of 18 or 20 as risk=1

**Table 16**

## ENVIRONMENTAL STRESS RISK

| | ENVIRONMENTAL STRESS RISK | SCORE ACCORDING TO GENOTYPE | | |
|---|---|---|---|---|
| SNP60 | OGG1 Cys326**Ser** | Cys/Cys=2 | Cys/Ser=1 | Ser/Ser=0 |
| SNP64 | SOD2 Ala16**Val** | Ala/Ala=0 | Ala/Val=1 | Val/Val=2 |
| SNP68 | SULT1A1 Arg213**His** | Arg/Arg=0 | Arg/His=1 | His/His=2 |
| SNP41 | GSTM1 Present/**Null** | Present=0 | Null=1 | |
| SNP44 | GSTT1 Present/**Null** | Present=0 | Null=1 | |
| SNP42 | GSTP1 Ile105**Val** | Ile/Ile=0 | Ile/Val=1 (2 if genotype GSTM1= Null) | Val/Val=2 (4 if genotype GSTM1= Null) |
| SNP43 | GSTP1 Ala114**Val** | Ala/Ala=0 | Ala/Val=1 | Val/Val=2 |
| SNP19 | COMT Val158**Met** (Allele*2) | Val/Val=0 | Val/Met=1 | Met/Met=2 |
| SNP45 | IL6 -174 C>**G** | C/C=0 | C/G=1 | G/G=2 |
| SNP46 | IL10 -1082 G>**A** | G/G=0 | G/A=1 | A/A=2 |
| SNP50-51-52-53-54-55-56 | NAT2 Allele*4(wt) | See Table 19 | | |

**Table 17**

| Genotype | Score of the Genotype |
|---|---|
| **Cys/Ser** | 1 |
| **Ala/Val** | 1 |
| **Arg/Arg** | 0 |
| **Present** | 0 |
| **Present** | 0 |
| **Ile/Ile** | 0 |
| **Ala/Ala** | 0 |
| **Val/Met** | 1 |
| **C/G** | 1 |
| **G/G** | 0 |
| ***4/*5B or *5A/*12A** | 1 |
| **Sum** | 5 |
| **PGR** | **5/22=0.23** |

The summation of the maximum score of the upper limits of the ranges of values is 22 points ($\sum_{i=1}^{n} Lsi = 22$).

The risk is calculated on a scale of 0-1 considering the maximum value of 22 as risk=1

**Table 18**

**Table 19**

| | | SNP50 | SNP51 | SNP52 | SNP53 | SNP54 | SNP55 | SNP56 | |
|---|---|---|---|---|---|---|---|---|---|
| Genotype | SCORE OF THE GENOTYPE | NAT2 R64Q | NAT2 282 C>T (Y94Y) | NAT2 I114T | NAT2 481C>T (L161L) | NAT2 R197Q | NAT2 K268R | NAT2 G286E | Metabolizer |
| *4/*4 | 0 | R/R | C/C | I/I | C/C | R/R | K/K | G/G | Fast |
| *4/*5A | 1 | R/R | C/C | I/T | C/T | R/R | K/K | G/G | Intermediate |
| *4/*5B or *5A/ *12A | 1 | R/R | C/C | I/T | C/T | R/R | K/R | G/G | Intermediate |
| *4/*5C | 1 | R/R | C/C | I/T | C/C | R/R | K/R | G/G | Intermediate |
| *4/*6A | 1 | R/R | C/T | I/I | C/C | R/Q | K/K | G/G | Intermediate |
| *4/*6B | 1 | R/R | C/C | I/I | C/C | R/Q | K/K | G/G | Intermediate |
| *4/*7A | 1 | R/R | C/C | I/I | C/C | R/R | K/K | G/E | Intermediate |
| *4/*7B | 1 | R/R | C/T | I/I | C/C | R/R | K/K | G/E | Intermediate |
| *4/*12A | 0 | R/R | C/C | I/I | C/C | R/R | K/R | G/G | Fast |
| *4/*14A | 1 | R/Q | C/C | I/I | C/C | R/R | K/K | G/G | Intermediate |
| *4/*14B | 1 | R/Q | C/T | I/I | C/C | R/R | K/K | G/G | Intermediate |
| *5A/*5A | 2 | R/R | C/C | T/T | T/T | R/R | K/K | G/G | Slow |
| *5A/*5B | 2 | R/R | C/C | T/T | T/T | R/R | K/R | G/G | Slow |
| *5A/*5C | 2 | R/R | C/C | T/T | C/T | R/R | K/R | G/G | slow |
| *5A/*6A | 2 | R/R | C/T | I/T | C/T | R/Q | K/K | G/G | slow |
| *5A/*6B | 2 | R/R | C/C | I/T | C/T | R/Q | K/K | G/G | slow |
| *5A/*7A | 2 | R/R | C/C | I/T | C/T | R/R | K/K | G/E | slow |
| *5A/*7B | 2 | R/R | C/T | I/T | C/T | R/R | K/K | G/E | slow |
| *4/*5B or *5A/ *12A | 1 | R/R | C/C | I/T | C/T | R/R | K/R | G/G | intermediate |
| *5A/*14A | 2 | R/Q | C/C | I/T | C/T | R/R | K/K | G/G | slow |
| *5A/*14B | 2 | R/Q | C/T | I/T | C/T | R/R | K/K | G/G | slow |

(continued)

| | | SNP50 | SNP51 | SNP52 | SNP53 | SNP54 | SNP55 | SNP56 | |
|---|---|---|---|---|---|---|---|---|---|
| Genotype | SCORE OF THE GENOTYPE | NAT2 R64Q | NAT2 282 C>T (Y94Y) | NAT2 I114T | NAT2 481C>T (L161L) | NAT2 R197Q | NAT2 K268R | NAT2 G286E | Metabolizer |
| *5B/*5B | 2 | R/R | C/C | T/T | T/T | R/R | R/R | G/G | slow |
| *5B/*5C | 2 | R/R | C/C | T/T | C/T | R/R | R/R | G/G | slow |
| *5B/*6A | 2 | R/R | C/T | I/T | C/T | R/Q | K/R | G/G | slow |
| *5B/*7A | 2 | R/R | C/C | I/T | C/T | R/R | K/R | G/E | slow |
| *5B/*7B | 2 | R/R | C/T | I/T | C/T | R/R | K/R | G/E | slow |
| *5B/*12A | 1 | R/R | C/C | I/T | C/T | R/R | R/R | G/G | intermediate |
| *5B/*14A | 2 | R/Q | C/C | I/T | C/T | R/R | K/R | G/G | slow |
| *5B/*14B | 2 | R/Q | C/T | I/T | C/T | R/R | K/R | G/G | slow |
| *5C/*5C | 2 | R/R | C/C | T/T | C/C | R/R | R/R | G/G | slow |
| *5C/*6A | 2 | R/R | C/T | I/T | C/C | R/Q | K/R | G/G | slow |
| *5C/*6B | 2 | R/R | C/C | I/T | C/C | R/Q | K/R | G/G | slow |
| *5C/*7A | 2 | R/R | C/C | I/T | C/C | R/R | K/R | G/E | slow |
| *5C/*7B | 2 | R/R | C/T | I/T | C/C | R/R | K/R | G/E | slow |
| *5C/*12A | 1 | R/R | C/C | I/T | C/C | R/R | R/R | G/G | intermediate |
| *5C/*14A | 2 | R/Q | C/C | I/T | C/C | R/R | K/R | G/G | slow |
| *5C/*14B | 2 | R/Q | C/T | I/T | C/C | R/R | K/R | G/G | slow |
| *6A/*6A | 2 | R/R | T/T | I/I | C/C | Q/Q | K/K | G/G | slow |
| *6A/*6B | 2 | R/R | C/T | I/I | C/C | Q/Q | K/K | G/G | slow |
| *6A/*7A or *6B/*7B | 2 | R/R | C/T | I/I | C/C | R/Q | K/K | G/E | slow |
| *6A/*7B | 2 | R/R | T/T | I/I | C/C | R/Q | K/K | G/E | slow |
| *6A/*12A | 1 | R/R | C/T | I/I | C/C | R/Q | K/R | G/G | intermediate |

(continued)

| | | SNP50 | SNP51 | SNP52 | SNP53 | SNP54 | SNP55 | SNP56 | |
|---|---|---|---|---|---|---|---|---|---|
| Genotype | SCORE OF THE GENOTYPE | NAT2 R64Q | NAT2 282 C>T (Y94Y) | NAT2 I114T | NAT2 481C>T (L161L) | NAT2 R197Q | NAT2 K268R | NAT2 G286E | Metabolizer |
| *6A/*14A or *6B/*14B | 2 | R/Q | C/T | I/I | C/C | R/Q | K/K | G/G | slow |
| *6A/*14B | 2 | R/Q | T/T | I/I | C/C | R/Q | K/K | G/G | slow |
| *6B/*6B | 2 | R/R | C/C | I/I | C/C | Q/Q | K/K | G/G | slow |
| *6B/*7A | 2 | R/R | C/C | I/I | C/C | R/Q | K/K | G/E | slow |
| *6A/*7A or *6B/*7B | 2 | R/R | C/T | I/I | C/C | R/Q | K/K | G/E | slow |
| *6B/*12A | 1 | R/R | C/C | I/I | C/C | R/Q | K/R | G/G | intermediate |
| *6B/*14A | 2 | R/Q | C/C | I/I | C/C | R/Q | K/K | G/G | slow |
| *6A/*14A or *6B/*14B | 2 | R/Q | C/T | I/I | C/C | R/Q | K/K | G/G | slow |
| *7A/*7A | 2 | R/R | C/C | I/I | C/C | R/R | K/K | E/E | slow |
| *7A/*7B | 2 | R/R | C/T | I/I | C/C | R/R | K/K | E/E | slow |
| *7A/*12A | 1 | R/R | C/C | I/I | C/C | R/R | K/R | G/E | intermediate |
| *7A/*14A | 2 | R/Q | C/C | I/I | C/C | R/R | K/K | G/E | slow |
| *7A/*14B or *7B/*14A | 2 | R/Q | C/T | I/I | C/C | R/R | K/K | G/E | slow |
| *7B/*7B | 2 | R/R | T/T | I/I | C/C | R/R | K/K | E/E | slow |
| *7B/*12A | 1 | R/R | C/T | I/I | C/C | R/R | K/R | G/E | intermediate |
| *7A/*14B or *7B/*14A | 2 | R/Q | C/T | I/I | C/C | R/R | K/K | G/E | slow |
| *7B/*14B | 2 | R/Q | T/T | I/I | C/C | R/R | K/K | G/E | slow |
| *12A/*12A | 0 | R/R | C/C | I/I | C/C | R/R | R/R | G/G | fast |
| *12A/*14A | 1 | R/Q | C/C | I/I | C/C | R/R | K/R | G/G | intermediate |

(continued)

| | | SNP50 | SNP51 | SNP52 | SNP53 | SNP54 | SNP55 | SNP56 | |
|---|---|---|---|---|---|---|---|---|---|
| Genotype | SCORE OF THE GENOTYPE | NAT2 R64Q | NAT2 282 C>T (Y94Y) | NAT2 I114T | NAT2 481C>T (L161L) | NAT2 R197Q | NAT2 K268R | NAT2 G286E | Metabolizer |
| *12A/*14B | 1 | R/Q | C/T | I/I | C/C | R/R | K/R | G/G | intermediate |
| *14A/*14A | 2 | Q/Q | C/C | I/I | C/C | R/R | K/K | G/G | slow |
| *14A/*14B | 2 | Q/Q | C/T | I/I | C/C | R/R | K/K | G/G | slow |
| *14B/*14B | 2 | Q/Q | T/T | I/I | C/C | R/R | K/K | G/G | slow |

**Table 20**

| | SNP12 | SNP13 | |
|---|---|---|---|
| | APOE Cys112Arg | APOE Arg158Cys | SCORE OF THE GENOTYPE |
| E3/E3 | Cys/Cys | Arg/Arg | 0 |
| E3/E2 | Cys/Cys | Arg/Cys | 0 |
| E3/E4 | Cys/Arg | Arg/Arg | 1 |
| E2/E4 | Cys/Arg | Arg/Cys | 1 |
| E2/E2 | Cys/Cys | Cys/Cys | 2 |
| E4/E4 | Arg/Arg | Arg/Arg | 13.5 |

Table 21

| | | CYP2D6 SNP29 | SNP30 | SNP31 |
|---|---|---|---|---|
| Genotype | Metabolizer | CYP2D6 2549A>del | CYP2D6 1847G>A | CYP2D6 1707T>del |
| Not determined | Not determined | A/A | G/G | T/T |
| Not determined | Not determined | A/del | G/G | T/T |
| Not determined | Not determined | A/A | G/A | T/T |
| Not determined | Not determined | A/A | G/G | T/of the |
| *3/*3 | Slow | del/del | G/G | T/T |
| *3/*4 | Slow | A/del | G/A | T/T |
| *3/*6 | Slow | A/del | G/G | T/del |
| *4/*4 | Slow | A/A | A/A | T/T |
| *4/*6 | Slow | A/A | G/A | T/del |
| *6/*6 | Slow | A/A | G/G | del/del |

**Table 22**

| | CYP2C19 | SNP28 |
|---|---|---|
| Genotype | Metabolizer | CYP2C19 681G>A |
| *1/*1 | fast | G/G |
| *1/*2 | intermediate | G/A |
| *2/*2 | slow | A/A |

**Table 23**

| | CYP2C9 | SNP26 | SNP27 |
|---|---|---|---|
| Genotype | Metabolizer | CYP2C9 3608 C>T | CYP2C9 42614 A>C |
| *1/*1 | fast | C/C | A/A |
| *1/*2 | intermediate | C/T | A/A |

(continued)

| CYP2C9 | | | |
|---|---|---|---|
| | | SNP26 | SNP27 |
| Genotype | Metabolizer | CYP2C9 3608 C>T | CYP2C9 42614 A>C |
| *1/*3 | slow | C/C | A/C |
| *2/*2 | slow | T/T | A/A |
| *2/*3 | slow | C/T | A/C |
| *3/*3 | very slow | C/C | C/C |

Table 24

## GLOBAL GENETIC RISK SUMMARY

| | |
|---|---|
| **VASCULAR RISK** | 0.29 |
| **OSTEOPOROSIS RISK** | 0.33 |
| **CARCINOGENIC RISK** | 0.27 |
| **ENVIRONMENTAL STRESS** | 0.23 |
| **GGR** | **0.28** |

Table 25

## RESPONSE TO DRUGS

**NAT2** (Allele *4 (wt))

| GENOTYPE | METABOLIZER | | |
|---|---|---|---|
| ***5B/*5B** | **Slow** | See Table 19 | SNP50-51-52-53-54-55-56 |

**CYP2D6** (Alleles *3, *4 and *6)

| GENOTYPE | METABOLIZER | | |
|---|---|---|---|
| ***4/*4** | **Slow** | See Table 21 | SNP29-30-31 |

**CYP2C19** (Alleles *1 (wt) and *2)

| GENOTYPE | METABOLIZER | | |
|---|---|---|---|
| ***1/*1** | **Fast** | See Table 22 | SNP28 |

**CYP2C9** (Alleles *1 (wt), *2 and *3)

| GENOTYPE | METABOLIZER | | |
|---|---|---|---|
| ***1/*3** | **Fast** | See Table 23 | SNP26-27 |

## Claims

1. An *in vitro* method for determining the global genetic risk of a subject to develope a pathology associated with aging from a combination of particular genetic risks comprising:

   i) simultaneously genotyping multiple human gene variants present in one or more genes of a subject associated with a pathology associated with aging in a biological sample of said subject;
   ii) determining each particular genetic risk; and
   iii) determining said global genetic risk according to the value of each particular genetic risk obtained in step ii).

2. Method according to claim 1, wherein said step i) is performed by means of DNA-chip analysis and/or gene sequencing.

3. Method according to claim 1, wherein said step ii) comprises:

   i) grouping the results obtained relating to each particular genetic risk of developing a pathology associated with aging;
   ii) standardizing the value of each genotype of each gene variant analyzed;
   iii) calculating each particular genetic risk such that:

      iiia) when said particular genetic risk is not formed by a combination of partial particular risks, said particular genetic risk is calculated by means of equation [1]:

$$\text{PGR} = \frac{\sum_{i=1}^{n} xi}{\sum_{i=1}^{n} Lsi} \qquad [1]$$

where
PGR represents the particular genetic risk to be calculated;
$x_i$ represents the standardized value of the genotype **characterized** for a gene variant in a sample, in relation to the particular genetic risk to be calculated;
$Ls_i$ represents the value of the upper limit of the range of standardized values assigned to each gene variant, in relation to the particular genetic risk to be calculated; and
n is the number of gene variants analyzed in relation to the particular genetic risk to be calculated; or, alternatively,
iiib) when said particular genetic risk is formed by a combination of partial particular risks, said particular genetic risk is calculated by means of equation [2]:

$$\text{PGR} = \frac{\sum_{i=1}^{n} PPGRi}{no.PPGR} \qquad [2]$$

where
PGR represents the particular genetic risk to be calculated;
PPGRi represents the value calculated for each partial particular genetic risk which, in combination with other partial particular genetic risks, forms the particular genetic risk to be calculated, wherein said PPGRi is calculated by means of equation [3]:

$$\text{PPGRi} = \frac{\sum_{i=1}^{n} xi}{\sum_{i=1}^{n} Lsi} \qquad [3]$$

where
PPGRi has the previously mentioned meaning;
$x_i$ represents the standardized value of the genotype **characterized** for a gene variant in a sample, in relation to the partial particular genetic risk to be calculated;
$Ls_i$ represents the value of the upper limit of the range of standardized values assigned to each gene variant, in relation to the partial particular genetic risk to be calculated; and
n is the number of gene variants analyzed in relation to the partial particular genetic risk to be calculated; and
no.PPGR is the number of partial particular genetic risks analyzed in relation to the partial particular genetic risk to be calculated.

**4.** Method according to claim 1, wherein the global genetic risk is calculated by means of equation [4]:

$$\text{GGR} = \frac{\sum PGR}{n} \qquad [4]$$

where
GGR represents the global genetic risk to be calculated;
PGR represents the value calculated for each particular genetic risk analyzed in relation to the global genetic risk to be calculated, and is calculated by means of the previously described equations [1] or [2]; and
n is the number of particular genetic risks analyzed in relation to the global genetic risk to be calculated.

**5.** Method according to any of claims 1 to 4, wherein said particular genetic risk is selected from the group formed by particular genetic risk associated with suffering from vascular disease (vascular risk), particular genetic risk associated with osteoporosis, particular genetic risk associated with carcinogenesis and particular genetic risk associated with environmental stress and oxidative damage.

**6.** Method according to claim 5, wherein said vascular risk is determined according to the partial particular genetic risks selected from the group formed by partial particular genetic risk associated with lipid metabolism, partial particular genetic risk associated with thrombosis, partial particular genetic risk associated with ictus, partial particular genetic risk associated with high blood pressure and partial particular genetic risk associated with endothelial vulnerability.

**7.** Method according to claim 6, wherein said partial particular genetic risk associated with lipid metabolism is determined according to the gene variants selected from the group formed by -75 G>A of the APOA1 gene, Arg3480Trp of the APOB gene, Arg3500Gln of the APOB gene, Arg3531Cys of the APOB gene, Cys112Arg of the APOE gene, Arg158Cys of the APOE gene, Arg451Gln of the CETP gene, TaqIB B1>B2 of the CETP gene, Gln192Arg of the PON1 gene, Gly595Ala of the SREBF2 gene, Leu7Pro of the NPY gene and combinations thereof.

**8.** Method according to claim 6, wherein said particular genetic risk associated with thrombosis is determined according to the gene variants selected from the group formed by 4G>5G of the PAI1 gene, Leu33Pro of the ITGB3 gene, 20210 G>A of the FII gene, Arg506Gln of the FV Leiden gene, Val34Leu of the F13A1 gene, Ala222Val of the MTHFR gene, 833 T>C of the CBS gene, 844ins68 of the CBS gene, -455 G>A of the FGB gene and combinations thereof.

**9.** Method according to claim 6, wherein said partial particular genetic risk associated with ictus is determined according to the gene variants selected from the group formed by 4G>5G of the PAI1 gene, Leu33Pro of the ITGB3 gene, 20210 G>A of the FII gene, Arg506Gln of the FV Leiden gene, Val34Leu of the F13A1 gene and combinations thereof.

**10.** Method according to claim 6, wherein said partial particular genetic risk associated with high blood pressure is determined according to the gene variants selected from the group formed by Gly389Arg of the ADRB1 gene; Gln27Glu of the ADRB2 gene, Gly16Arg of the ADRB2 gene, Met235Thr of the AGT gene, 1166 A>C of the AGTR1 gene, 393 T>C (Ile131Ile) of the GNAS gene, 825 C>T (Ser275Ser) of the GNB3 gene, intron 16 ins/del of the ACE gene, Trp64Arg of the ADRB3 gene and combinations thereof.

**11.** Method according to claim 6, wherein said partial particular genetic risk associated with endothelial vulnerability is determined according to the gene variants selected from the group formed by 5A>6A of the MMP3 gene, -786 T>C of the NOS3 gene, Glu298Asp of the NOS3 gene, Ala222Val of the MTHFR gene, 833 T>C of the CBS gene, 844ins68 of the CBS gene, Pro319Ser of the GJA4 gene and combinations thereof.

**12.** Method according to claim 5, wherein said particular genetic risk associated with osteoporosis is determined according to the gene variants selected from the group formed by 1546 G>T of the COL1A1 gene, IVS1-397 T>C p>P (PvuII) of the ESR1 gene, b>B of the VDR gene and combinations thereof.

**13.** Method according to claim 5, wherein said particular genetic risk associated with carcinogenesis is determined according to the gene variants selected from the group formed by -34 A>G of the CYP17A1 gene, Ile462Val of the CYP1A1 gene, T3801C of the CYP1A1 gene, Leu432Val of the CYP1B1 gene, Allele*4 (Asn453Ser) of the CYP1B1 gene, 1558 C>T of the CYP19A1 gene, Val158Met (Allele*2) of the COMT gene, 331 G>A of the PGR gene, IVS1 -397 T>C p>P (PvuII) of the ESR1 gene, b>B of the VDR gene, Ala49Thr of the SRD5A2 gene, Val89Leu of the SRD5A2 gene, Ala541Thr of the ELAC2 gene and combinations thereof.

**14.** Method according to claim 5, wherein said particular genetic risk associated with environmental stress and oxidative damage is determined according to the gene variants selected from the group formed by Cys326Ser of the OGG1 gene, Ala16Val of the SOD2 gene, Arg213His of the SULT1A1 gene, present>null GSTM1, present>null GSTT1, Ile105Val of the GSTP1 gene, Ala114Val of the GSTP1 gene, Val158Met (Allele*2) of the COMT gene, -174 C>G of the IL6 gene, -1082 G>A of the IL10 gene, R64Q of the NAT2 gene, 282 C>T (Y94Y) of the NAT2 gene, I114T of the NAT2 gene, 481C>T (L161L) of the NAT2 gene, R197Q of the NAT2 gene, K268R of the NAT2 gene, G286E of the NAT2 gene and combinations thereof.

**15.** Method according to claim 1, further comprising determining the particular genetic risk associated with the response to drugs.

**16.** Method according to claim 15, wherein said particular genetic risk associated with the response to drugs is determined according to the gene variants selected from the group formed by R64Q, 282 C>T (Y94Y), I114T, 481C>T (L161L), R197Q, K268R and G286E of the NAT2 gene; Arg144Cys (allele*2) and Ile359Leu (allele*3) of the CYP2C9 gene; 681 G>A (Pro227Pro) (allele*2) of the CYP2C19 gene; 2549 A>del (allele*3), 1847 G>A (allele*4) and 1707 del>T (allele*6) of the CYP2D6 gene; and combinations thereof.

**17.** Method according to claim 1, wherein said gene variant to be genotyped is selected from the group formed by the intron 16 ins/del polymorphism of the ACE gene; the Gly389Arg polymorphism of the ADRB1 gene; the Gln27Glu and Gly16Arg polymorphisms of the ADRB2 gene; the Trp64Arg polymorphism of the ADRB3 gene; the Met235Thr polymorphism of the AGT gene; the 1166 A>C polymorphism of the AGTR1 gene; the -75 G>A polymorphism of the APOA1 gene; the Arg3480Trp, Arg3500Gln, and Arg3531Cys polymorphisms of the APOB gene; the Cys112Arg and Arg158Cys polymorphisms of the APOE gene; the 833 T>C and 844ins68 polymorphisms of the CBS gene; the TaqIB B1>B2 and Arg451Gln polymorphisms of the CETP gene; the 1546 G>T polymorphism of the COL1A1 gene; the Va1158Met (Allele*2) polymorphism of the COMT gene; the -34 A>G polymorphism of the CYP17A1 gene; the 1558 C>T polymorphism of the CYP19A1 gene; the Ile462Val and T3801C polymorphism of the CYP1A1 gene; the Leu432Val and Allele*4 (Asn453Ser) polymorphism of the CYP1B1 gene; the Arg144Cys (allele*2) and Ile359Leu (allele*3) polymorphism of the CYP2C9 gene; the 681 G>A (Pro227Pro) (allele*2) polymorphism of the CYP2C19 gene; the 2549 A>del (allele*3), 1847 G>A (allele*4) and 1707 del>T (allele*6) polymorphism of the CYP2D6 gene; the Ala541Thr polymorphism of the ELAC2 gene; the IVS1 -397 T>C p>P (PvuII) polymorphism of the ESR1 gene; the Val34Leu polymorphism of the F13A1 gene; the -455 G>A polymorphism of the FGB gene; the 20210 G>A polymorphism of the FII gene; the Arg506Gln polymorphism of the FV Leiden gene; the Pro319Ser polymorphism of the GJA4 gene; the 393 T>C (Ile131Ile) polymorphism of the GNAS gene; the 825 C>T (Ser275Ser) polymorphism of the GNB3 gene; the present>null GSTM1 polymorphism; the Ile105Val and Ala114Val polymorphisms of the GSTP1 gene; the present>null GSTT1 polymorphism; the - 174 C>G polymorphism of the IL6 gene; the -1082 G>A polymorphism of the IL10 gene; the Leu33Pro polymorphism of the ITGB3 gene; the 5A>6A polymorphism of the MMP3 gene; the Ala222Val polymorphism of the MTHFR gene; the R64Q, 282 C>T (Y94Y), I114T, 481C>T (L161L), R197Q, K268R and G286E polymorphisms of the NAT2 gene; the -786 T>C and Glu298Asp polymorphisms of the NOS3 gene; the Leu7Pro polymorphism of the NPY gene; the Cys326Ser polymorphism of the OGG1 gene; the 4G>5G polymorphism of the PAI1 gene; the 331 G>A polymorphism of the PGR gene; the Gln192Arg polymorphism of the PON1 gene; the Ala16Val polymorphism of the SOD2 gene; the Ala49Thr and Val89Leu polymorphisms of the SRD5A2 gene; the Gly595Ala polymorphism of the SREBF2 gene; the Arg213His polymorphism of the SULT1A1 gene; the b>B polymorphism of the VDR gene; and combinations thereof.

**18.** Method according to claim 17, further comprising genotyping one or more additional gene variants associated with pathologies associated with aging.

**19.** A DNA-chip comprising a support on which there is deposited a plurality of probes useful for detecting human gene variants present in one or more genes, wherein said probes are selected from the group formed by the probes identified as SEQ ID NO: 1-13, SEQ ID NO: 15, SEQ ID NO: 17-44, SEQ ID NO: 53-128, SEQ ID NO: 130, SEQ ID NO: 132-172, SEQ ID NO: 181-200, SEQ ID NO: 202, SEQ ID NO: 204, SEQ ID NO: 206, SEQ ID NO: 208, SEQ ID NO: 210, SEQ ID NO: 212, SEQ ID NO: 222 and SEQ ID NO: 224-276.

**20.** A kit comprising a DNA-chip according to claim 19.

**21.** An oligonucleotide primer selected from the oligonucleotide primers identified as SEQ ID NO: 277-278, SEQ ID NO: 285-319, SEQ ID NO: 321-326, SEQ ID NO: 333-340, SEQ ID NO: 343-356, SEQ ID NO: 359-362, SEQ ID NO: 364, SEQ ID NO: 367, SEQ ID NO: 369-371, SEQ ID NO: 374, SEQ ID NO: 377-381, SEQ ID NO: 383, SEQ ID NO: 385-402 and SEQ ID NO: 404-414.